# EUROPÄISCHE PATENTSCHRIFT

(11) **EP 0 759 058 B1**
(45) Veröffentlichungstag und Bekanntmachung des Hinweises auf die Patenterteilung: **02.04.2003**
(21) Anmeldenummer: 95919427.5
(22) Anmeldetag: 08.05.1995
(51) Int. Cl.: C09K 19/30, C09K 19/02, C09K 19/44, C09K 19/46, C07C 13/28, C07C 25/24, C07C 43/225, C07C 255/50

(54) **SUPERTWIST-FLÜSSIGKRISTALLANZEIGE**
SUPERTWIST LIQUID CRYSTAL DISPLAY
DISPOSITIF D'AFFICHAGE A CRISTAUX LIQUIDES DU TYPE A ROTATION COMPLETE

(30) Priorität: 10.05.1994 DE 4416454; 10.05.1994 DE 4416455; 29.09.1994 DE 4434851; 25.11.1994 DE 4441963; 01.12.1994 DE 4442842; 30.03.1995 DE 19511632
(43) Veröffentlichungstag der Anmeldung: 26.02.1997
(73) Patentinhaber: MERCK PATENT GmbH, 64293 Darmstadt (DE)
(72) Erfinder: REIFFENRATH, Volker, D-64380 Rossdorf (DE); RIEGER, Bernhard, D-64839 Münster (DE); JUNGE, Michael, D-64319 Pfungstadt (DE); SCHMIDT, Martina, D-64839 Münster (DE); PFISTER, Christine, D-24969 Grossenwiehe (DE)
(86) Internationale Anmeldenummer: EP9501740
(87) Internationale Veröffentlichungsnummer: WO95030723

(56) Entgegenhaltungen:
- EP-A- 0 261 614
- EP-A- 0 470 590
- EP-A- 0 571 916
- WO-A-90/12073
- WO-A-91/00898
- DE-A- 3 509 170
- SCHEFFER T.J. AND NEHRING J.: 'A new, highly multiplexable liquid crystal display.' APPLIED PHYSICAL LETTERS Bd. 45, 1984, Seiten 1021 - 1023
- "Liquid Crystal Mixtures for STN-Displays", Broschüre von der Fa. Merck KGaA, Tokyo und Osaka, Japan, Juli 1989
- COGNARD J.: 'Alignment of nematic liquid crystals and their mixtures.' MOLECULAR CRYSTALS LIQUID CRYSTALS (SUPPLEMENT 1) Bd. 78, 1981, Seiten 1 - 77
- CUSTALLANO J.A.: 'Surface anchoring of liquid crystal molecules on various substrates.' MOLECULAR CRYSTALS AND LIQUID CRYSTALS Bd. 94, 1983, Seiten 33 - 41
- SCHEFFER T.J. UND NEHRING J.: 'Accurate determination of liquid crystal tilt bins angels.' JOURNAL OF APPLIED PHYSICS Bd. 48, 1977, Seiten 1783 - 1792
- "Techniques for measuring tilt angels"; T. Uchida und H. Seki; Liquid Crysatla, Application and Uses, Vol. 3; B. Bahadur, Herausgeber; 1992; 36-63
- BAUR G. ET AL: 'Determination of the tilt angels at surfaces of substrates in liquid crystal cells.' PHYSICS LETTERS Bd. 56, 1976, Seiten 142 - 144
- MOSLEY A. ET AL: 'Surface alignment of liquid crystals by rubbed polymer layers.' DISPLAYS Januar 1987, Seiten 17 - 21
- "Calculation of the pitch of nematic mixtures using chiral additions such as RO-CM-5715A, -5815C, -9207, -9209 and CN", Broschüre der Fa. Hoffmann LaRoche, 1987
- "How to dope liquid crystal mixtures in order to ensure optimim pitch and to compensate the temperature dependence", F. Moia, Broschüre der Fa. Hoffmann LaRoche, 1990
- "Influence of material parameters on the temperature dependence of the electrooptic characteristic of STN Displays", H.J. Plach et al., präsentiert auf der Japandisplay, Kyoto, Japan, 1989.

## Beschreibung

Die Erfindung betrifft Supertwist-Flüssigkristallanzeigen (SFA) mit sehr kurzen Schattzeiten und guten Steilheiten und Winkelabhängigkeiten sowie die darin verwendeten neuen nematischen Flüssigkristallmischungen.

SFA gemäß des Oberbegriffs sind bekannt, z.B. aus EP 0 131 216 B1; DE 34 23 993 A1; EP 0 098 070 A2; M. Schadt und F. Leenhouts, 17. Freiburger Arbeitstagung Flüssigkristalle (8.-10.04.87); K. Kawasaki et al., SID 87 Digest 391 (20.6); M. Schadt und F. Leenhouts, SID 87 Digest 372 (20.1); K. Katoh et al., Japanese Journal of Applied Physics, Vol. 26, No. 11, L 1784-L 1786 (1987); F. Leenhouts et al., Appl. Phys. Lett. 50 (21), 1468 (1987); H.A. van Sprang und H.G. Koopman, J. Appl. Phys. 62 (5), 1734 (1987); T.J. Scheffer und J. Nehring, Appl. Phys. Lett. 45 (10), 1021 (1984), M. Schadt und F. Leenhouts, Appl. Phys. Lett. 50 (5), 236 (1987) und E.P. Raynes, Mol. Cryst. Liq. Cryst. Letters Vol. 4 (1), pp. 1-8 (1986). Der Begriff SFA umfaßt hier jedes höher verdrillte Anzeigeelement mit einem Verdrillungswinkel dem Betrage nach zwischen 160° und 360°, wie beispielsweise die Anzeigeelemente nach Waters et al. (C.M. Waters et al., Proc. Soc. Inf. Disp. (New York) (1985) (3rd Intern. Display Conference, Kobe, Japan), die STN-LCD's (DE OS 35 03 259), SBE-LCD's (T.J. Scheffer und J. Nahring, Appl. Phys. Lett. 45 (1984) 1021), OM1-LCD's (M. Schadt und F. Leenhouts, Appl. Phys. Lett. 50 (1987), 236, DST-LCD's (EP OS 0 246 842) oder BW-STN-LCD's (K. Kawasaki et al., SID 87 Digest 391 (20.6)).

Derartige SFA zeichnen sich im Vergleich zu Standard-TN-Anzeigen durch wesentlich bessere Steilheiten der elektrooptischen Kennlinie und damit verbundenen besseren Kontrastwerten sowie durch eine wesentlich geringere Winkelabhängigkeit des Kontrastes aus. Von besonderem Interesse sind SFA mit sehr kurzen Schaltzeiten insbesondere auch bei tieferen Temperaturen. Zur Erzielung von kurzen Schaltzeiten wurden bisher insbesondere die Viskositäten der Flüssigkristallmischungen optimiert unter Verwendung von meist monotropen Zusätzen mit relativ hohem Dampfdruck. Die erzielten Schaltzeiten waren jedoch nicht für jede Anwendung ausreichend.

Zur Erzielung einer steilen elektrooptischen Kennlinie sollen die Flüssigkristallmischungen relativ große Werte für K₃/K₁ und relativ kleine Werte für Δε/ε_{⊥} aufweisen.

Über die Optimierung des Kontrastes und der Schaltzeiten hinaus werden an derartige Mischungen weitere wichtige Anforderungen gestellt:
1. Breites d/p-Fenster
2. Hohe chemische Dauerstabilität
3. Hoher elektrischer Widerstand
4. Geringe Frequenzabhängigkeit der Schwellenspannung.

Die erzielten Parameterkombinationen sind bei weitem noch nicht ausreichend, insbesondere für Hochmultiplex-STN (1/400). Zum Teil ist dies darauf zurückzuführen, daß die verschiedenen Anforderungen durch Materialparameter gegenläufig beeinflußt werden.

Es besteht somit immer noch ein großer Bedarf nach SFA mit sehr kurzen Schaltzeiten bei gleichzeitig großem Arbeitstemperaturbereich, hoher Kennliniensteilheit, guter Winketabhängigkeit des Kontrastes und niedriger Schwellenspannung, die den oben angegebenen Anforderungen gerecht werden.

In der DE 35 09 170 werden Verbindungen der Formel 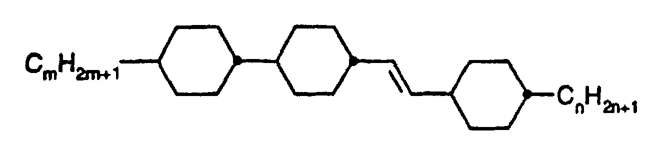 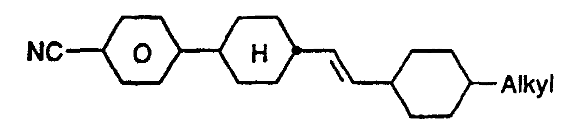 genannt.

In der Chemiker Zeitung 104 (1980), 269-271 werden Verbindungen der Formel I mit langkettigen Alkylresten (m + n > 9) beschrieben. Letztere Vertreter weisen jedoch ausschließlich die für eine Verwendung in nematischen Flüssigkristallmischungen unvorteilhafte, smektische Phasen auf. Überraschenderweise konnte durch eine Verkürzung der Kettenlänge ein nematischer Phasenbereich bei gleichzeitiger Unterdrückung der smektischen Phase erhalten werden.

Der Erfindung liegt die Aufgabe zugrunde, SFA bereitzustellen, die die oben angegebenen Nachteile nicht oder nur in geringerem Maße und gleichzeitig sehr gute Steilheiten aufweisen.

Es wurde nun gefunden, daß diese Aufgabe gelöst werden kann, wenn man nematische Flüssigkristallmischungen verwendet, die 1,2-Dicyclohexylethylenderivate der Formel I 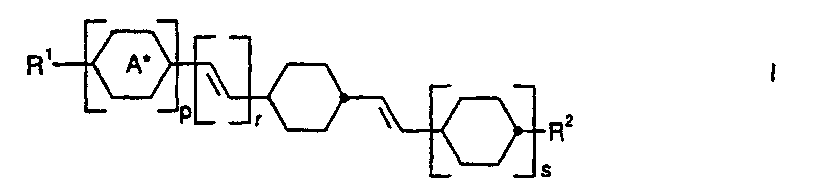 enthalten,
worin 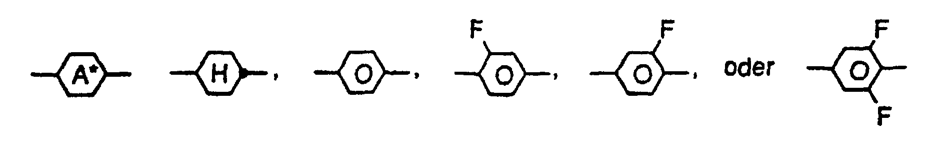
- R¹: F, Cl, einen unsubstituierten oder einfach durch CN oder CF₂ oder mindestens einfach durch Halogen substituierten Alkyl-, Alkoxy- oder Alkenylrest mit 1 bis 10 C-Atomen, wobei in diesem Rest auch ein oder mehrere CH₂-Gruppen durch -O- ersetzt sein können,
- R²: (CH₂)ₙ-CH=CH-R^{a} oder (CH₂)ₙ-CH=CH₂ und im Fall r = 1 auch R^{a}
- R^{a}: geradkettiges Alkyl mit 1-6 C-Atomen
- n: 0-6
- p: 0 oder 1,
- r: 0, 1 oder 2, und
- s: 1 oder 2
bedeuten.

Die Verbindungen der Formel I erhöhen deutlich die Steilheit von STN-Mischungen ohne die Schaltzeiten zu erhöhen.

Gegenstand der Erfindung ist somit ein SFA mit
- zwei planparallelen Trägerplatten, die mit einer Umrandung eine Zelle bilden,
- einer in der Zelle befindlichen nematischen Flüssigkristallmischung mit positiver dielektrischer Anisotropie,
- Elektrodenschichten mit darüberliegenden Orientierungsschichten auf den Innenseiten der Trägerplatten,
- einem Anstellwinkel zwischen der Längsachse der Moleküle an der Oberfläche der Trägerplatten und den Trägerplatten von 1 Grad bis 30 Grad, und
- einem Verdrillungswinkel der Flüssigkristallmischung in der Zelle von Orientierungsschicht zu Orientierungsschicht dem Betrag nach zwischen 100 und 600°,
- einer nematischen Flüssigkristallmischung mit einem Δε ≥ 1 bestehend aus
   a) 20 - 90 Gew.% einer flüssigkristallinen Komponente A, bestehend aus einer oder mehreren Verbindungen mit einer dielektrischen Anisotropie von über +1,5;
   b) 10 - 65 Gew.% einer flüssigkristallinen Komponente B, bestehend aus einer oder mehreren Verbindungen mit einer dielektrischen Anisotropie zwischen -1,5 und +1,5;
   c) 0 - 20 Gew.% einer flüssigkristallinen Komponente D, bestehend aus einer oder mehreren Verbindungen mit einer dielektrischen Anisotropie von unter -1,5 und
   d) einer optisch aktiven Komponente C in einer Menge, daß das Verhältnis zwischen Schichtdicke (Abstand der planparallelen Trägerplatten) und natürlicher Ganghöhe der chiralen nematischen Flüssigkristallmischung 0,2 bis 1,3 beträgt,
dadurch gekennzeichnet, daß Komponente B mindestens eine Verbindung der Formel I 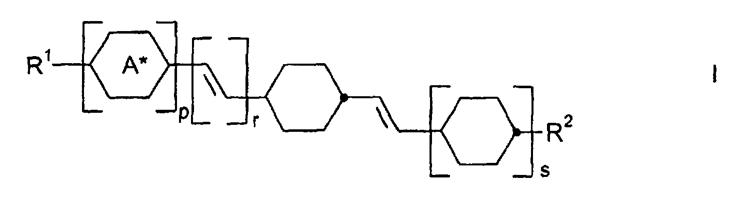 enthält,
worin 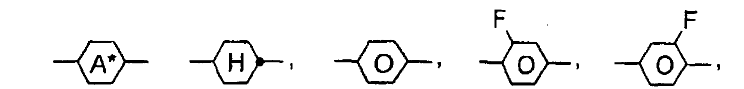 oder 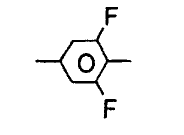
- R¹: F, Cl, einen unsubstituierten oder einfach durch CN oder CF₂ oder mindestens einfach durch Halogen substituierten Alkyl-, Alkoxy- oder Alkenylrest mit 1 bis 10 C-Atomen, wobei in diesem Rest auch ein oder mehrere CH₂-Gruppen durch -O- ersetzt sein können,
- R²: (CH₂)ₙ-CH=CH-R^{a} oder (CH₂)ₙ-CH=CH₂ und im Fall r = 1 auch R^{a}
- R^{a}: geradkettiges Alkyl mit 1-6 C-Atomen
- n: 0-6
- p: 0 oder 1,
- r: 0, 1 oder 2, und
- s: 1 oder 2
bedeuten.

Gegenstand der Erfindung sind auch entsprechende Flüssigkristallmischungen zur Verwendung in SFA.

Bevorzugte Verbindungen der Formel I sind insbesondere Verbindungen der Teilformeln IA bis IK (L¹, L² = H oder F; n = 0-6; o = 0-6; Alkyl = C₁₋₆)

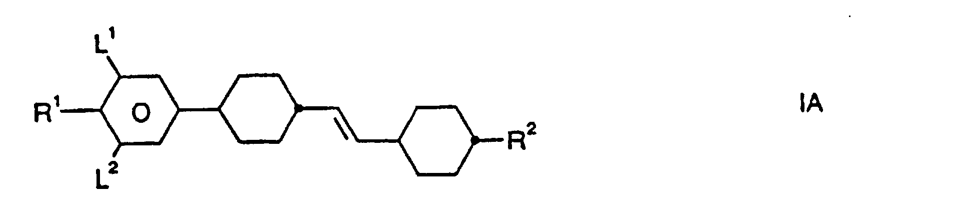

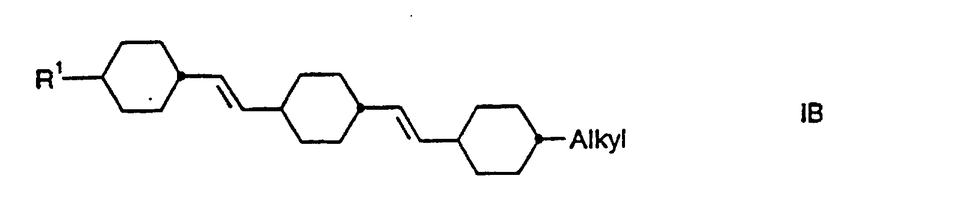

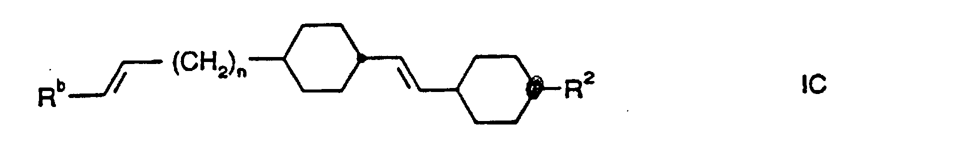

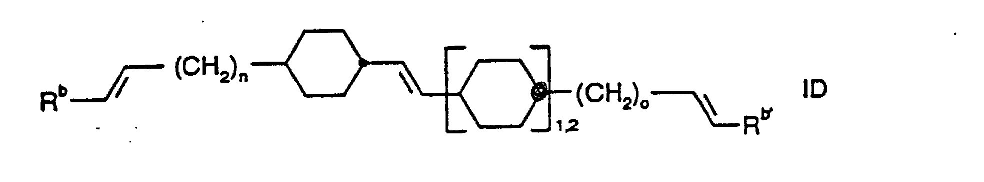

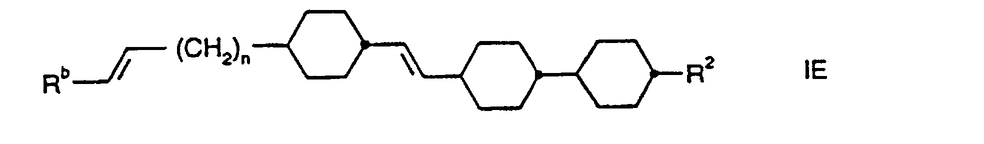

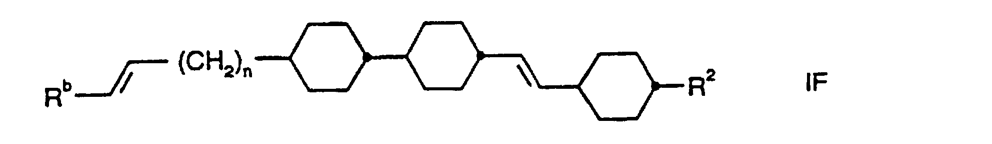

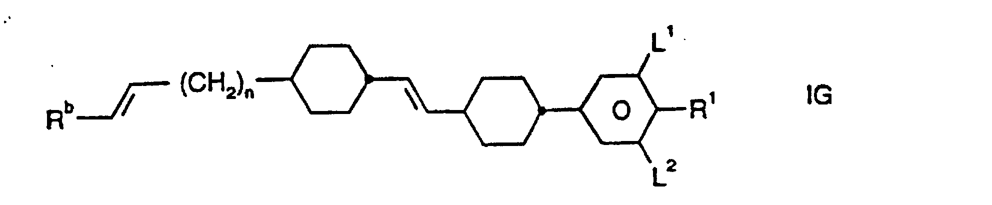

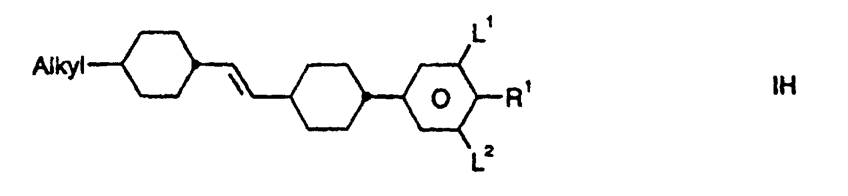

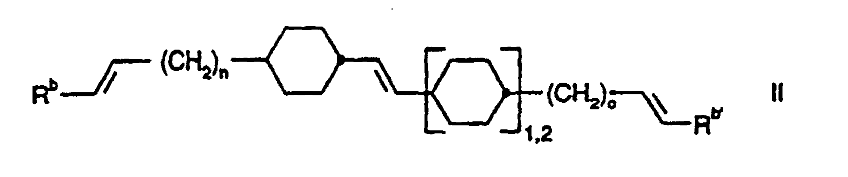

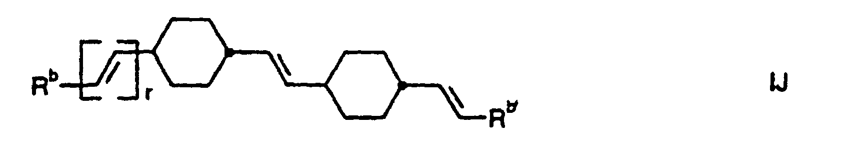

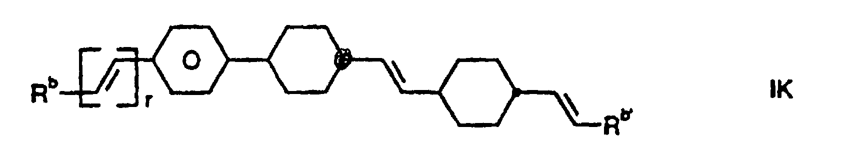

Bevorzugte erfindungsgemäße Mischungen, enthaltend insbesondere Verbindungen der Teilformeln I1-I8:

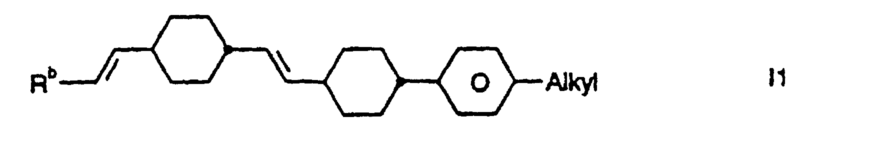

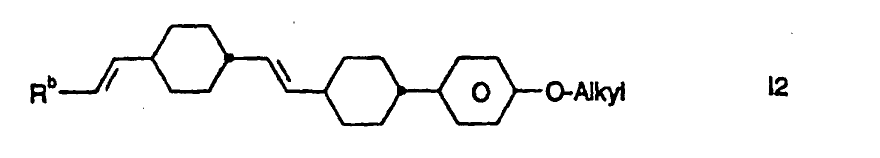

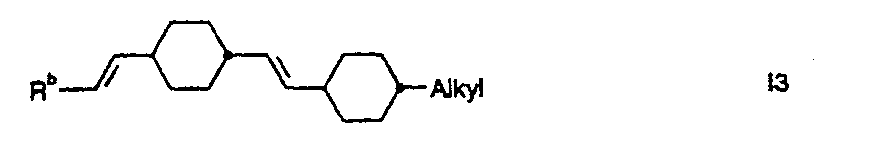

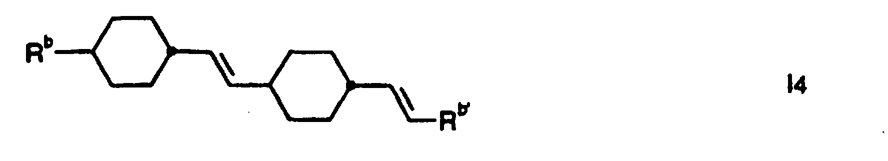

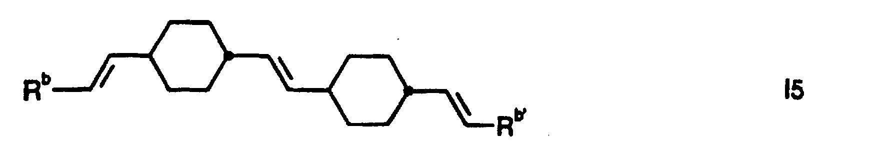

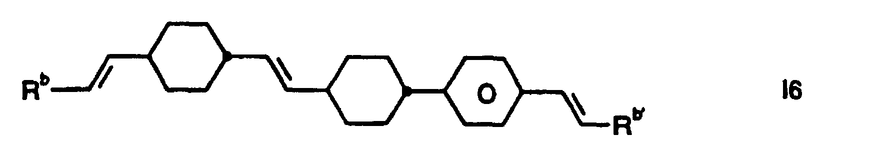

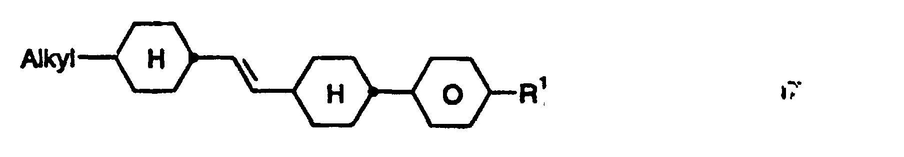

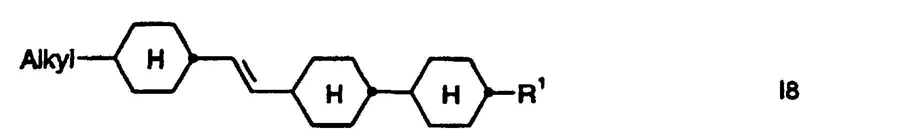

R^{a} ist vorzugsweise geradkettiges Alkyl und n ist 0-6. R^{b} und R^{b'} bedeuten jeweils unabhängig voneinander in den Teilformeln IC-IJ vorzugsweise H oder Methyl, femer Ethyl, Propyl, Butyl, Pentyl oder Hexyl. o ist 0 bis 6.

In den Verbindungen der Formel I bedeutet R¹ vorzugsweise Alkyl, Alkoxy, CN oder ein halogenierter Alkyl- oder Alkenylrest. Halogen bedeutet vorzugsweise F. R¹ bedeutet insbesondere F, Cl, OCF₃, OCF₂H, CF₃, OCH₂CF₃, OCHFCF₃, OC₂F₅, C₂F₅, OC₂F₄H, OCHF₂, wenn p = 1 ist und Ring A* 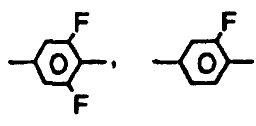 oder 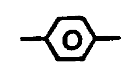 ist.

Insbesondere bevorzugt sind Verbindungen, worin R¹ und/oder R² ein Alkenylrest ist, wobei die Doppelbindung in 1- oder 3-Position ist und trans-Konfiguration besitzt.

Falls R¹ einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, femer Methyl, Octyl, Nonyl, Decyl, Methoxy, Octoxy, Nonoxy oder Decoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3-oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ einen Alkylrest bedeutet, in dem eine CH₂-Gruppe durch -CH=CHersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acrytoyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R¹ einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigten Flügelgruppen R¹ können gelegentlich wegen einer besseren Löslichkeit in den üblichen flüssigkristallinen Basismaterialien von Bedeutung sein, insbesondere aber als chirale Dotierstoffe, wenn sie optisch aktiv sind.

Verzweigte Gruppen dieser Art enthalten in der Regel nicht mehr als eine Kettenverzweigung. Bevorzugte verzweigte Reste sind Isopropyl, 2-Butyl (= 1-Methylpropyl), Isobutyl (= 2-Methylpropyl), 2-Methylbutyl, Isopentyl (= 3-Methyl- butyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxypentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl 4,4-Bis-(ethoxycarbonyl)-butyl, 5,5-Bis-(ethoxycarbonyl)-hexyl.

Verbindungen der Formel I, die über für Polykondensationen geeignete Flügelgruppen R¹ verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyclohexanringe trans-1,4-disubstituiert sind.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind.

Die Herstellung der erfindungsgemäßen Verbindungen erfolgt z.B. wie in der DE 35 09 170 A1 beschrieben:

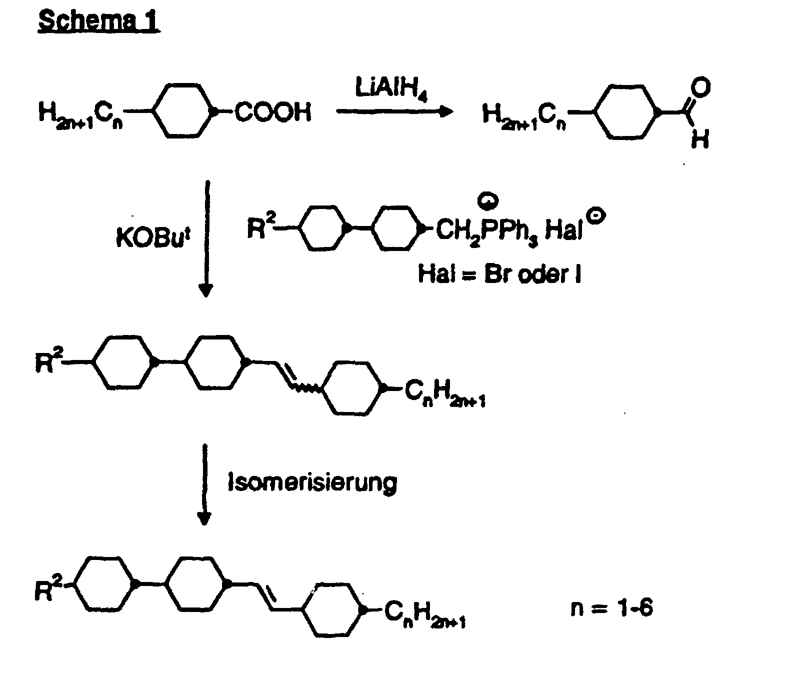

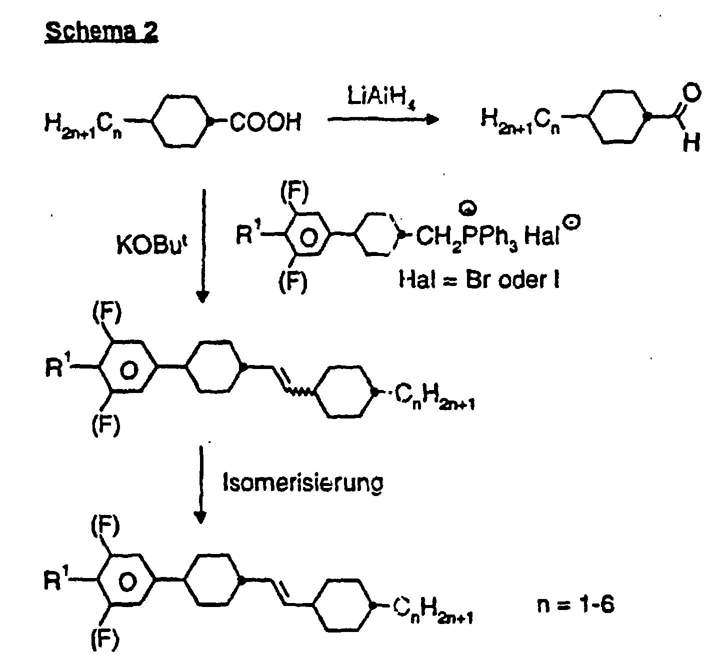

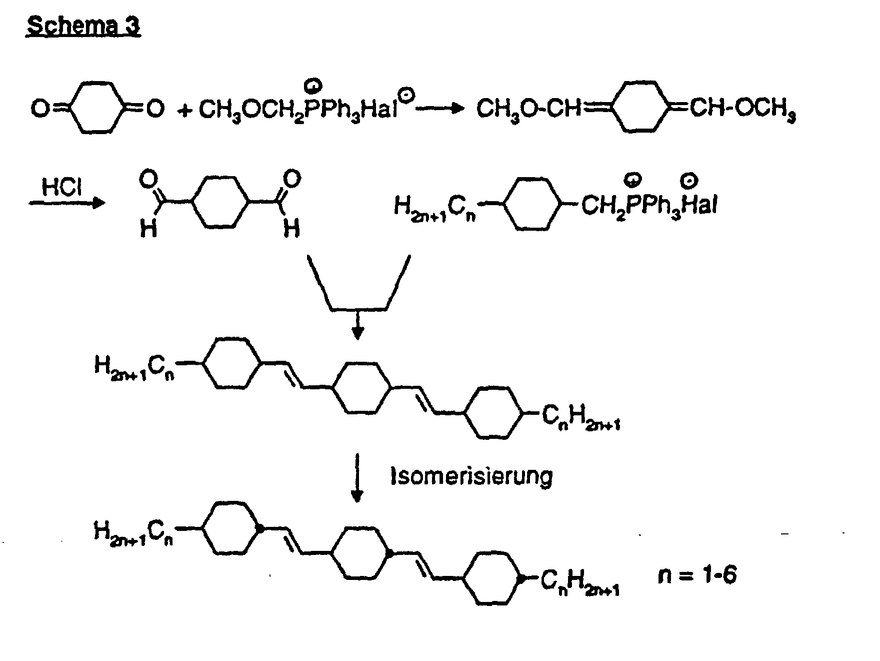

Die erfindungsgemäßen Verbindungen können weiterhin wie folgt hergestellt werden; dabei kann man auch von an sich bekannten, hier nicht näher erwähnten Varianten Gebrauch machen.

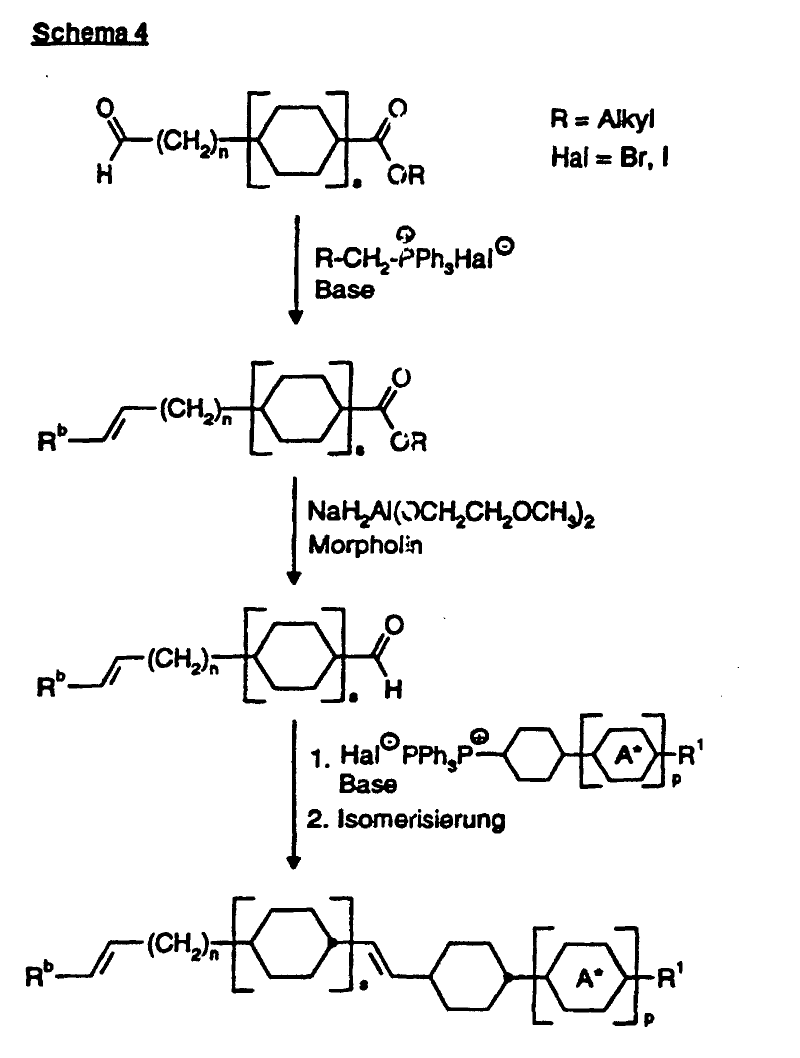

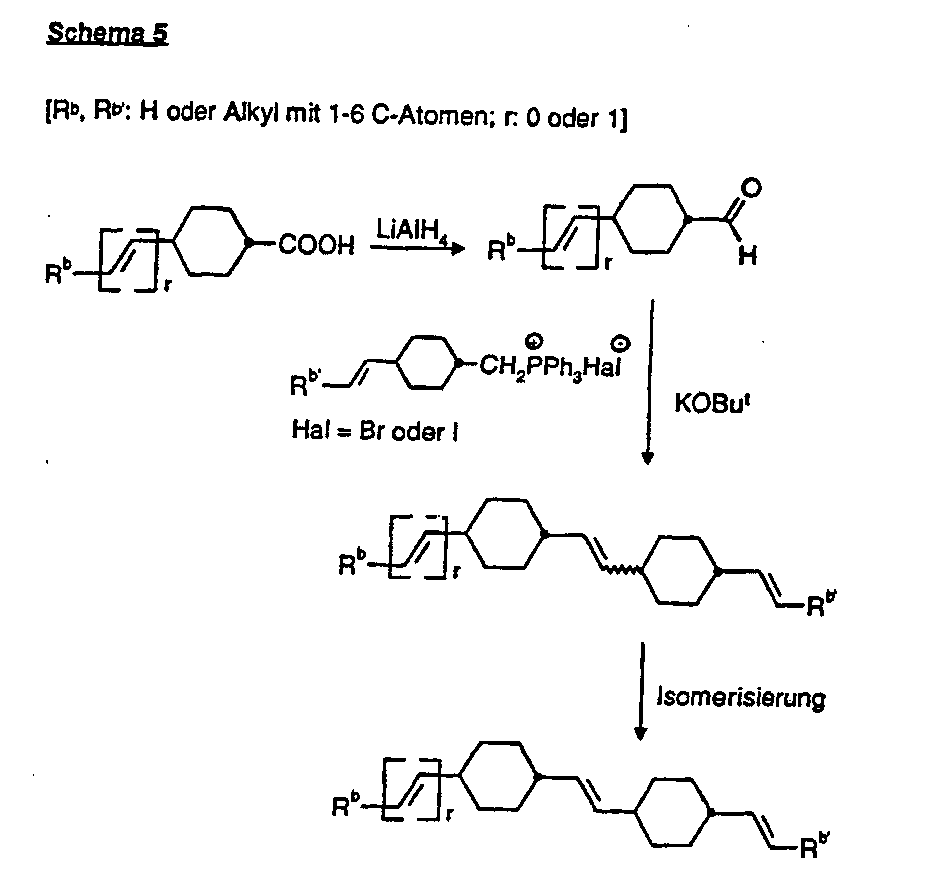

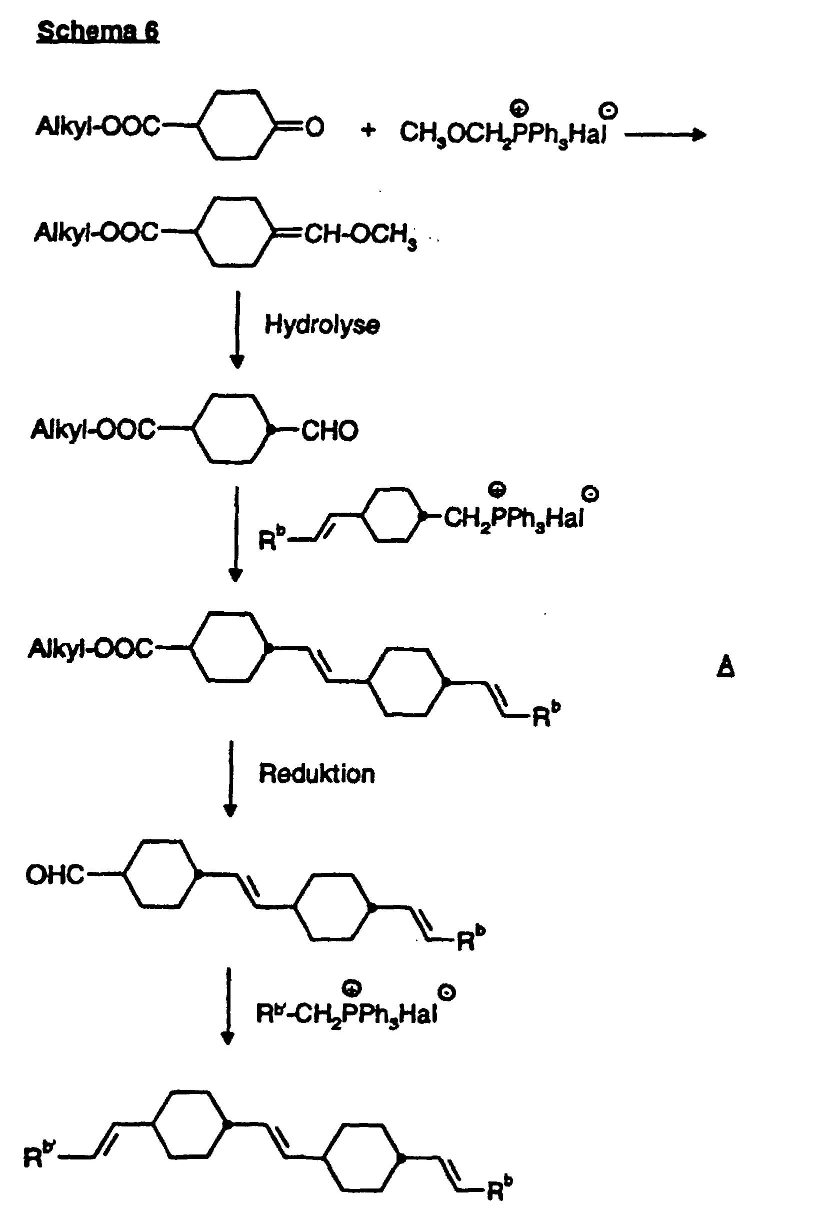

Gegenstand der Erfindung ist ebenfalls ein Verfahren zur Herstellung der Verbindungen der Formeln I4, I5 sowie das neue Zwischenprodukt der Formel A 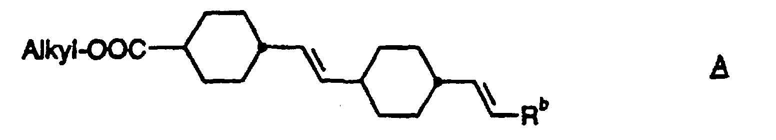 worin
R^{b} H oder Alkyl mit 1-6 C-Atomen und Alkyl C₁-C₆-Alkyl bedeuten.

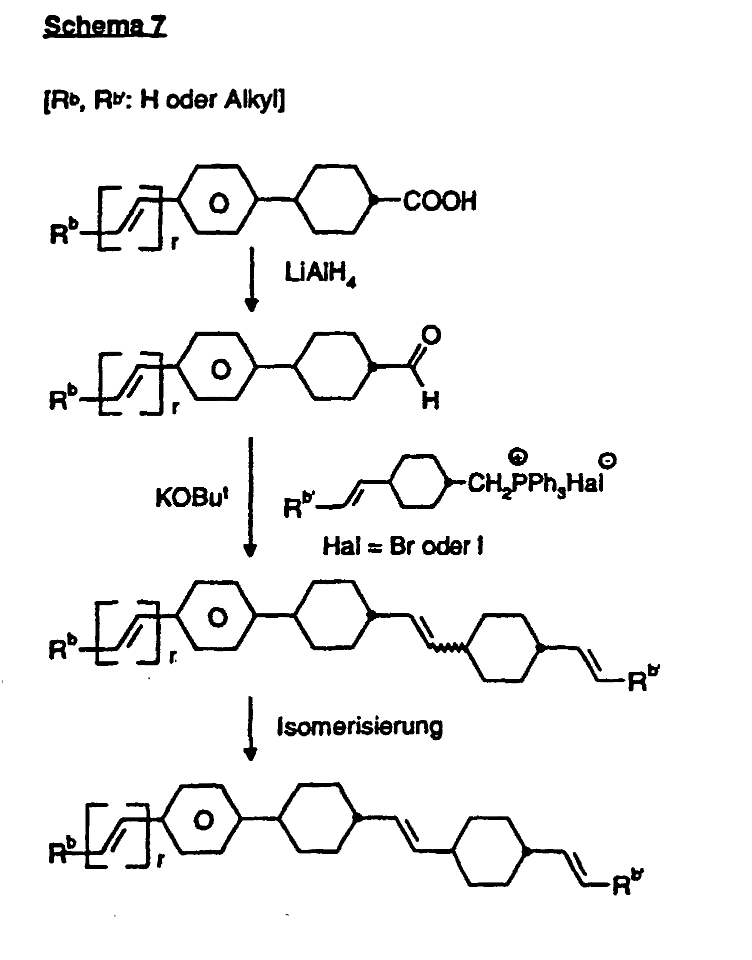

Die erfindungsgemäßen Flüssigkristallmischungen setzen sich vorzugsweise wie folgt zusammen:
- Komponente A enthält vorzugsweise Verbindungen der Formeln II und III, 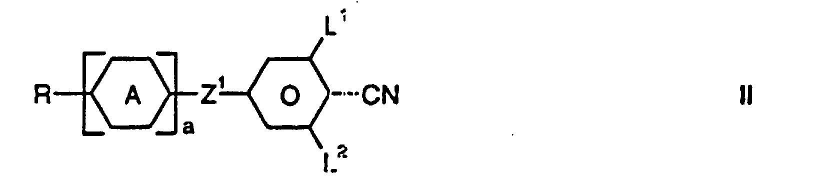 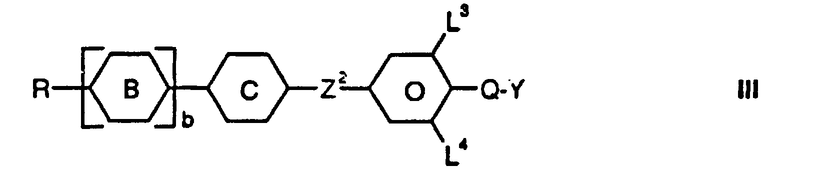
worin
- R: eine Alkylgruppe mit 1 bis 12 C-Atomen ist, wobei auch ein oder zwei nicht benachbarte CH₂-Gruppen durch -O-, -CH=CH-, -CO-, -OCO- oder -COO- ersetzt sein können, 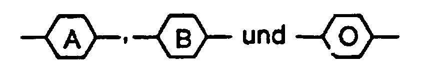 jeweils unabhängig voneinander 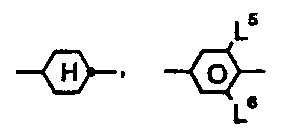 oder 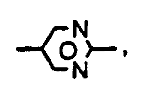
- L¹⁻⁶: jeweils unabhängig voneinander H oder F,
- Z¹: -COO-, -CH₂CH₂- oder eine Einfachbindung,
- Z²: -CH₂CH₂-, -COO-, -C≡C- oder eine Einfachbindung,
- Q: -CF₂-, -CHF-, -OCF₂-, -OCHF- oder eine Einfachbindung,
- Y: F oder Cl,
- a: 1 oder 2, und
- b: 0 oder 1
bedeuten.

Bevorzugte Verbindungen der Formel II entsprechen den Unterformeln IIa bis IId: 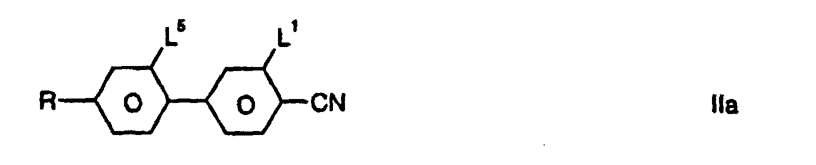 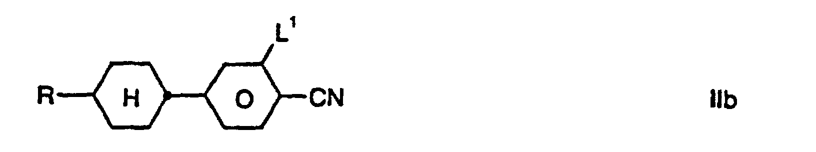 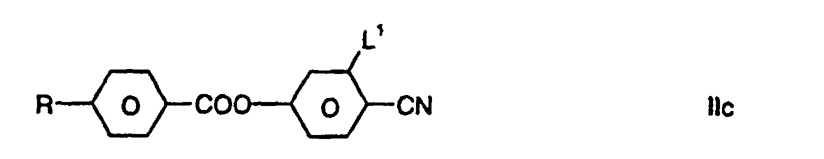 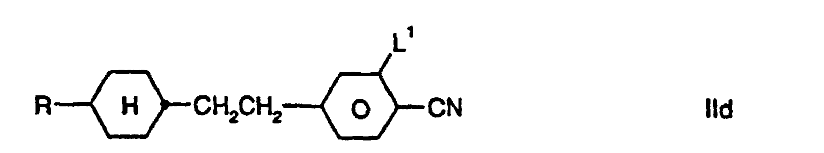 wobei R, L¹ und L⁵ die oben angegebene Bedeutung haben.

Bevorzugte Verbindungen der Formel III entsprechen den Unterformeln IIIa-IIIt:

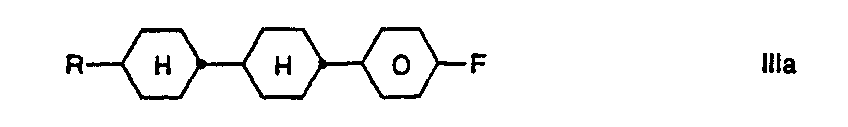

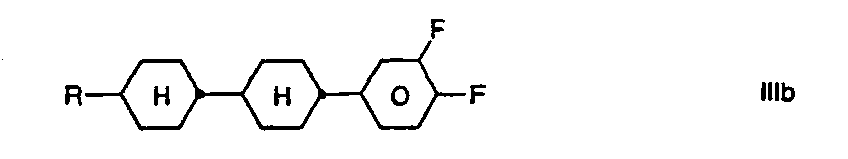

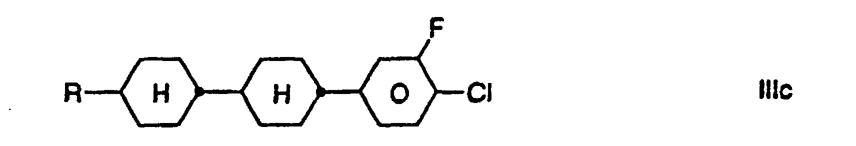

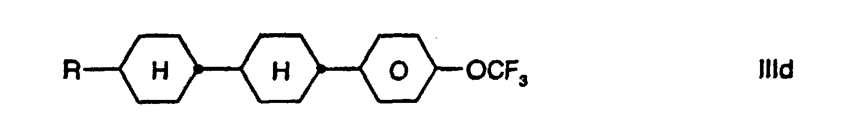

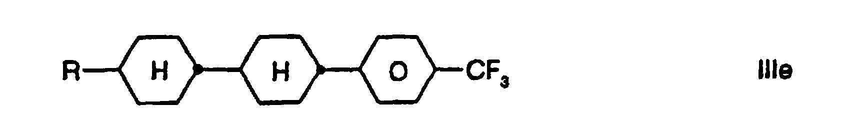

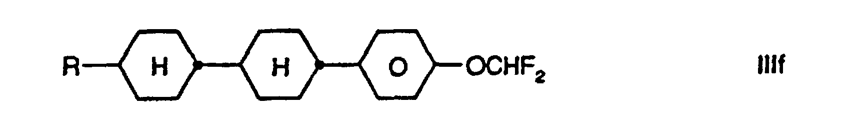

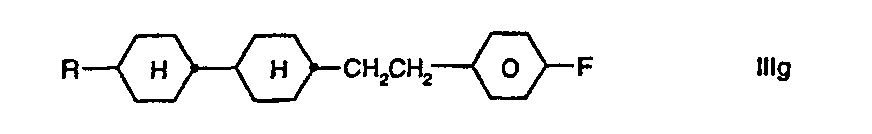

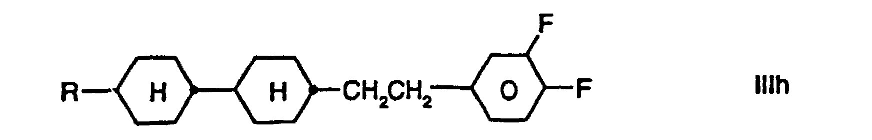

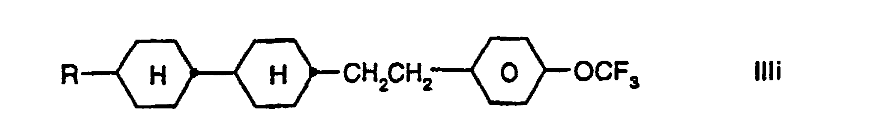

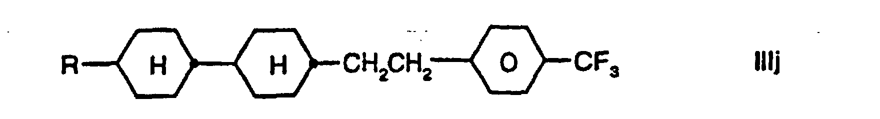

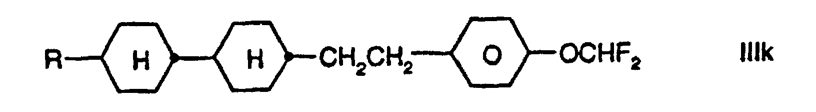

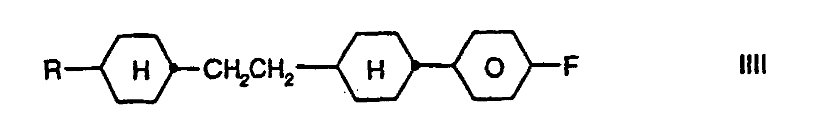

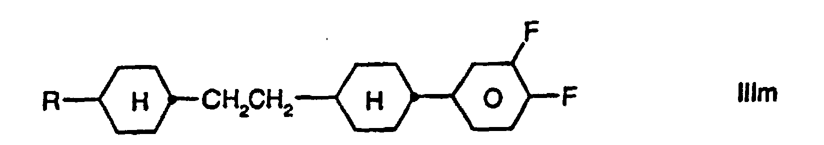

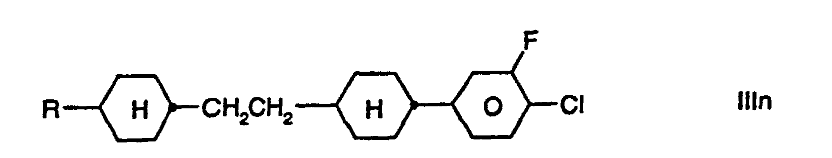

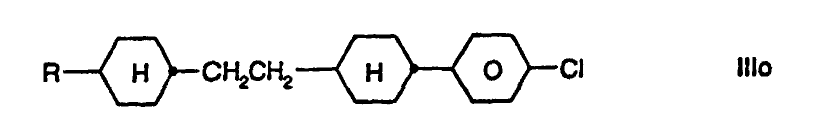

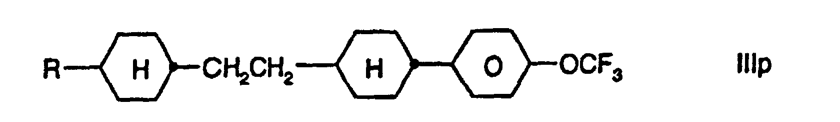

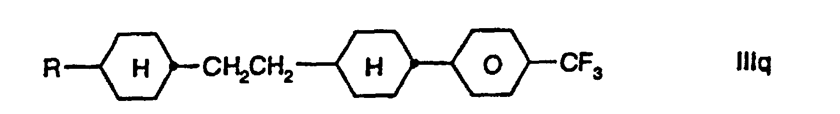

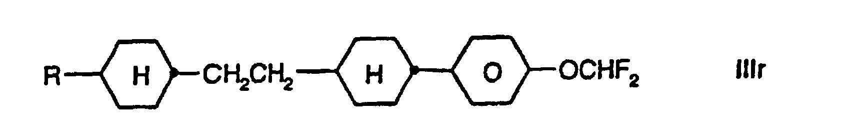

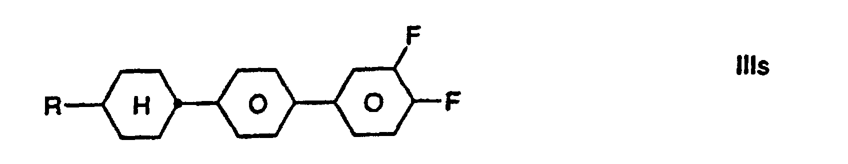

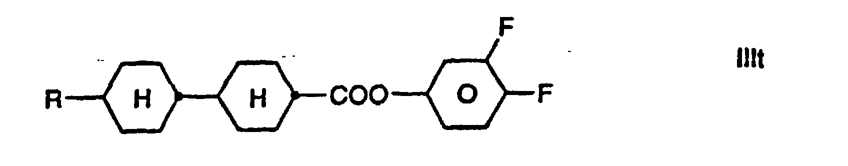

Bevorzugte Mischungen enthalten neben ein oder mehreren Verbindungen der Formel I ein, zwei, drei oder mehrere Verbindungen der Formeln IIa, IIb, IIc, IId, IIId, IIIh, IIIi, IIIl, IIIm oder IIIs, vorzugsweise ein oder mehrere Verbindungen der Formel IIId oder IIIh und ein bis vier Verbindungen der Formel I und ein bis drei Verbindungen der Formeln IIb und IIc.

Die einzelnen Verbindungen z.B. der Formeln II und III bzw. deren Unterformeln oder auch andere Verbindungen, die in den erfindungsgemäßen SFA verwendet werden können, sind entweder bekannt, oder sie können analog zu den bekannten Verbindungen hergestellt werden.

In einer besonders bevorzugten Ausfuhrungsform enthält Komponente A zusätzlich Verbindungen der Formeln AI bis AV: 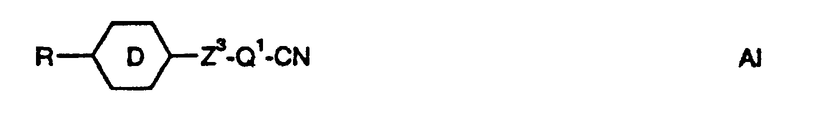 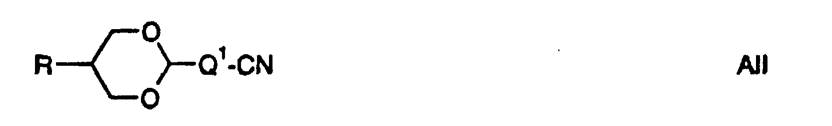 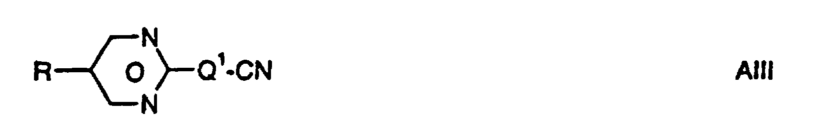 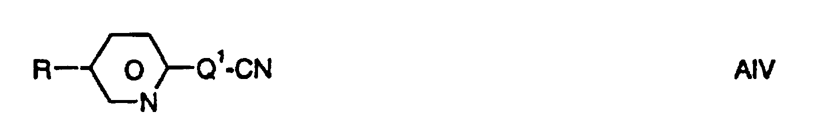 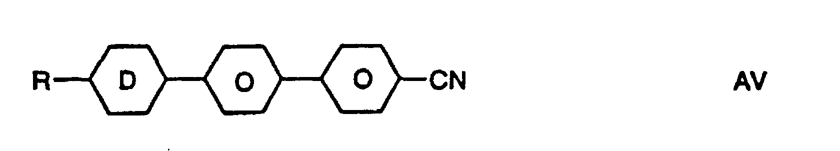 worin
- R: eine Alkylgruppe mit 1 bis 12 C-Atomen ist, wobei auch ein oder zwei nicht benachbarte CH₂-Gruppen durch -O-, -CH=CH-, -CO-, -OCO- oder -COO- ersetzt sein können,
Q¹ 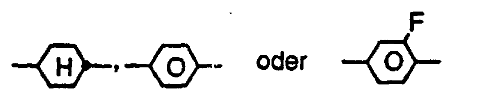
Z³ 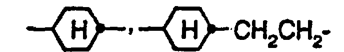 -CH₂CH₂-, -CO-CO-, -O-CO- oder eine Einfachbindung 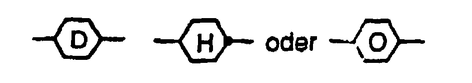
bedeuten.

Vorzugsweise enthalten die Mischungen 5 bis 50 % an Verbindungen der Formel AI. Bevorzugt werden diejenigen Verbindungen der Formel AI eingesetzt, worin Z¹-CH₂CH₂-, -COO- oder eine Einfachbindung bedeutet, insbesondere Verbindungen der Formeln IIa1, IIb1, IIc1, IId1, IIa2, IIb2 und IIc2:

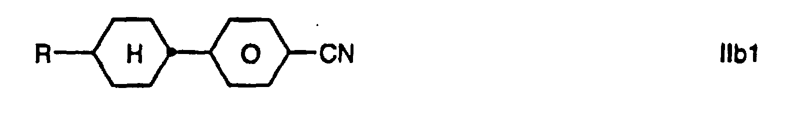

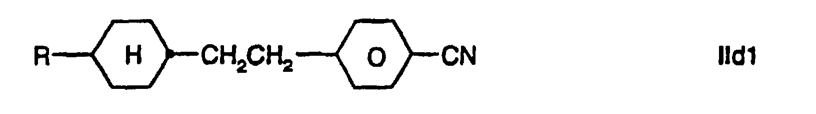

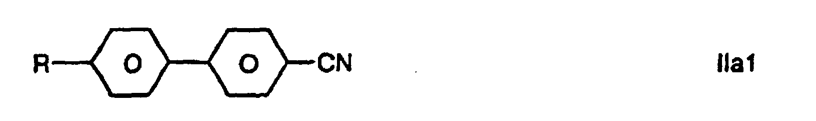

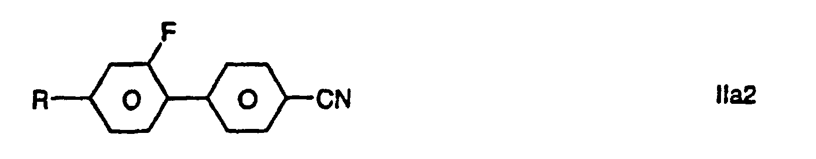

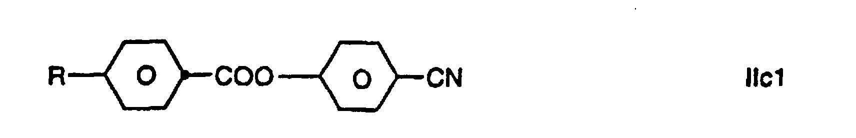

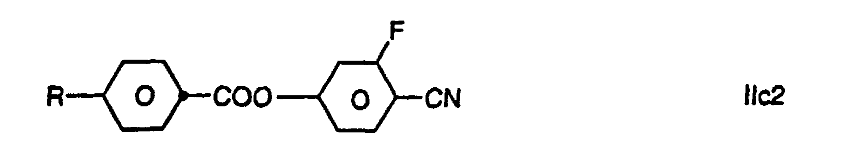

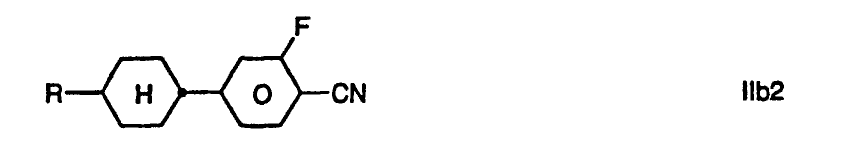

Komponente A enthält vorzugsweise ein oder mehrere Verbindungen der Formeln IIb1 und gegebenenfalls ein oder mehrere Verbindungen der Formeln IIc2.

Vorzugsweise enthalten die erfindungsgemäßen Mischungen ein oder mehrere polare Verbindungen mit einem hohen Klärpunkt ausgewählt aus der Gruppe bestehend aus den Verbindungen IIe bis IIj:

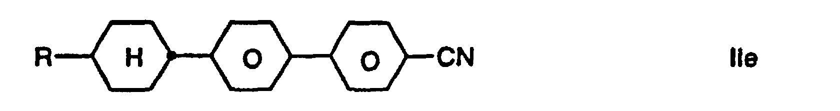

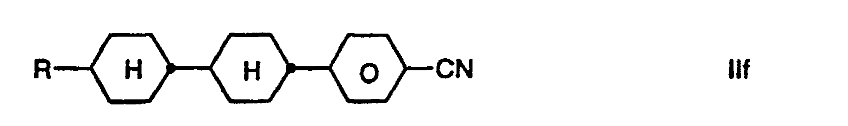

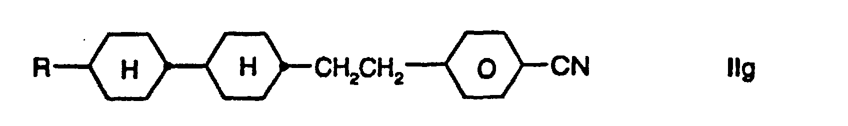

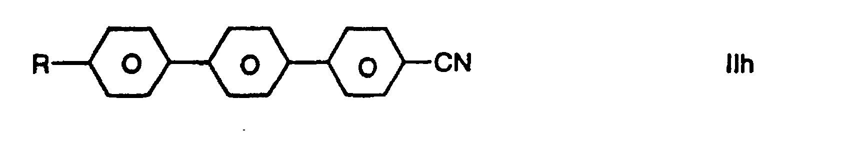

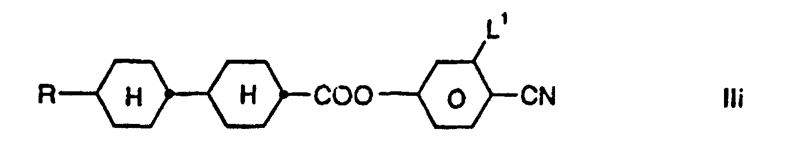

In den Verbindungen IIe bis IIj können die 1,4-Phenylenringe auch lateral durch ein oder zwei Fluoratome substituiert sein. Bevorzugte Verbindungen dieses Typs sind die Verbindungen der Formein IIf1 bis IIj1:

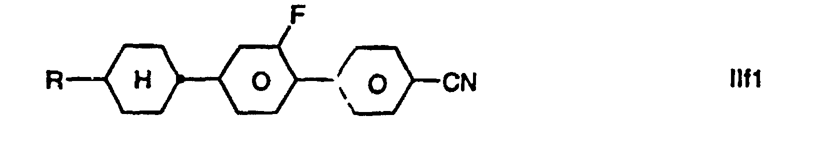

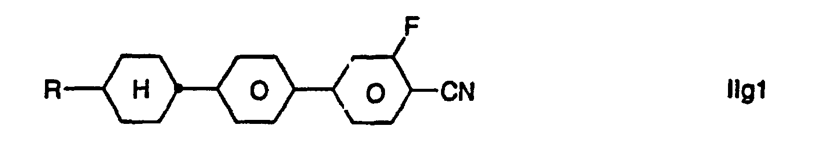

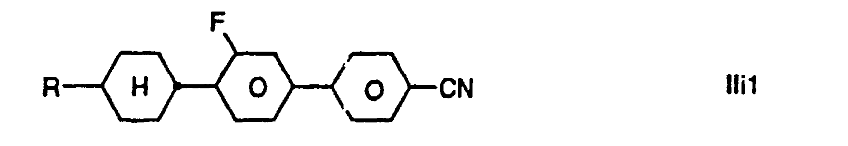

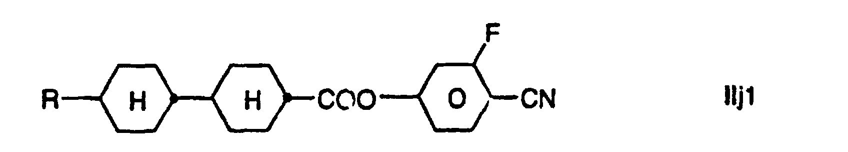

In den erfindungsgemäßen Mischungen liegt der Anteil der Verbindungen IIe bis IIj vorzugsweise bei ca. 2 bis 25 %. Bevorzugte Flüssigkristallmischungen enthalten ein oder mehrere Verbindungen der Gruppe B, vorzugsweise 10 bis 40 %.

Die Verbindungen der Gruppe B zeichnen sich sowohl durch ihre niedrigen Werte für die Rotationsviskosität (γ₁) < 150 mPa.s als auch durch ihren hohen Klärpunkt (> 120 °C) aus.
- Komponente B enthält eine oder mehrere Verbindungen ausgewählt aus der Gruppe bestehend aus den Verbindungen der Formeln IV1 bis IV9: 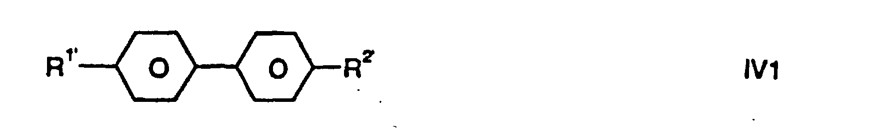 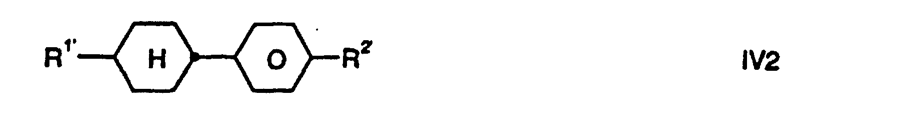 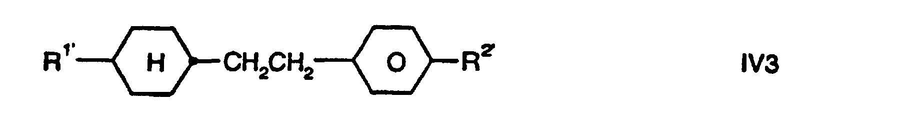 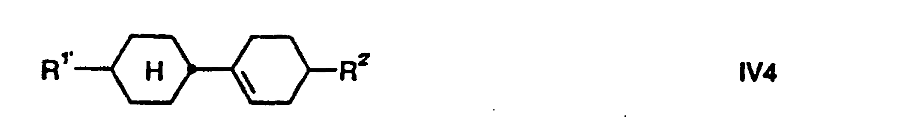 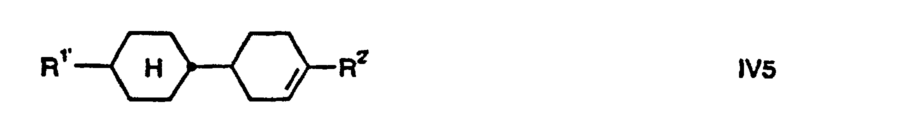 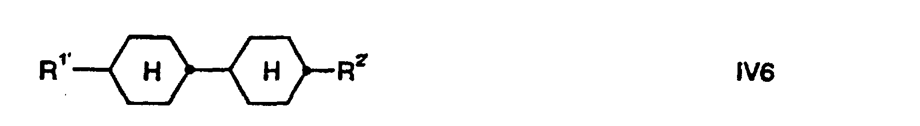 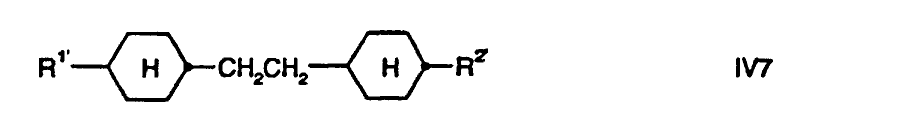 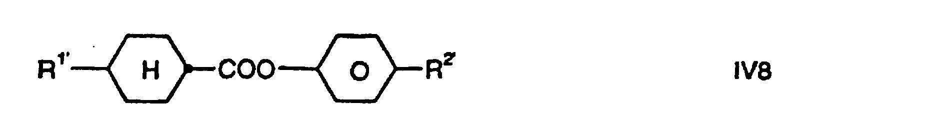 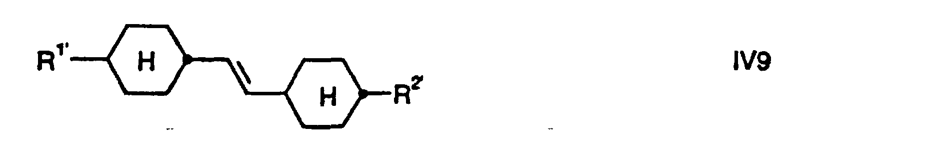 worin R^{1'} und R^{2'} die für R angegebene Bedeutung haben.
- Komponente B enthält zusätzlich eine oder mehrere Verbindungen ausgewählt aus der Gruppe bestehend aus den Verbindungen der Formeln IV10 bis IV24, 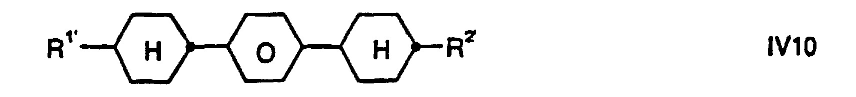 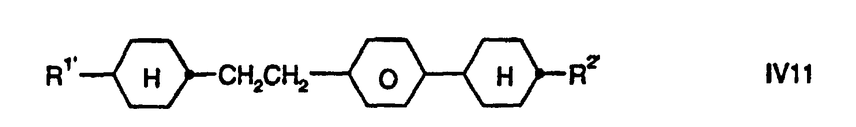 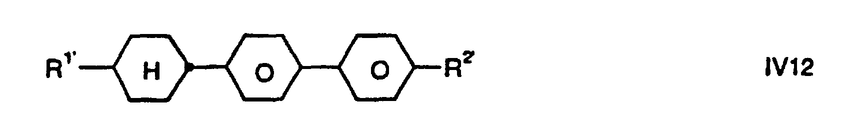 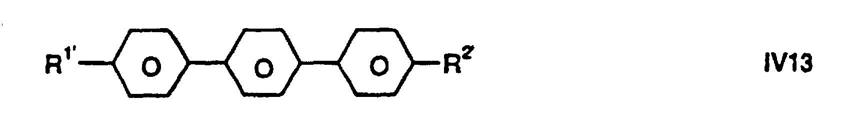 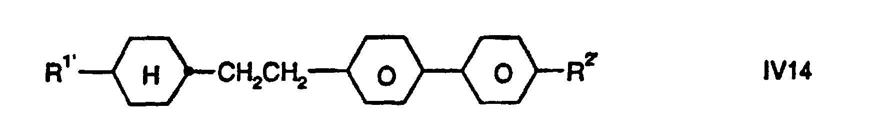 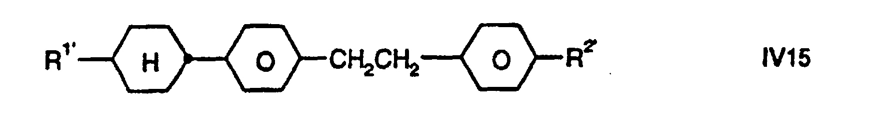 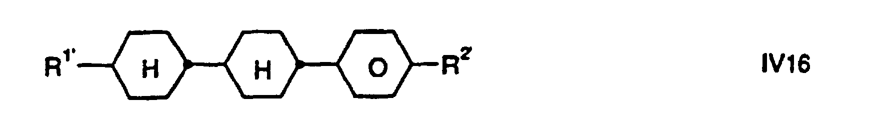 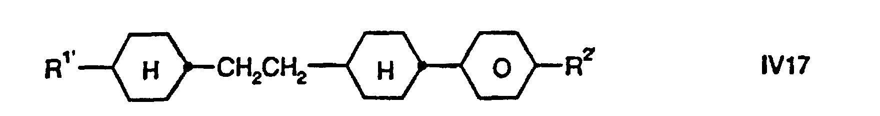 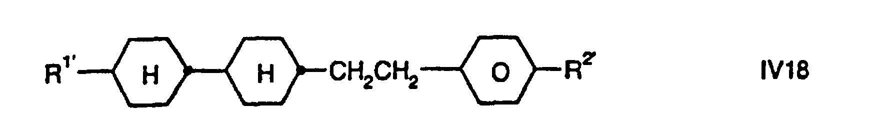 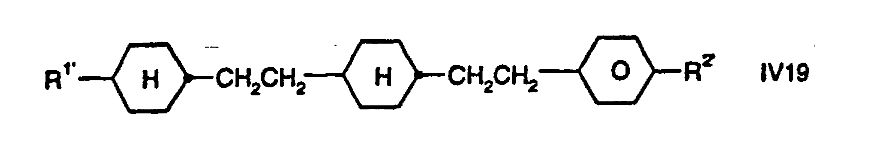 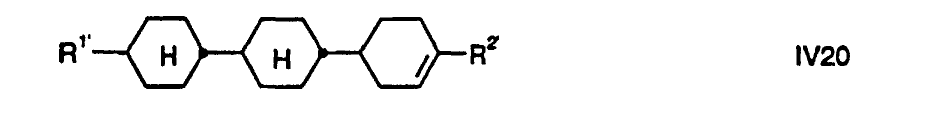 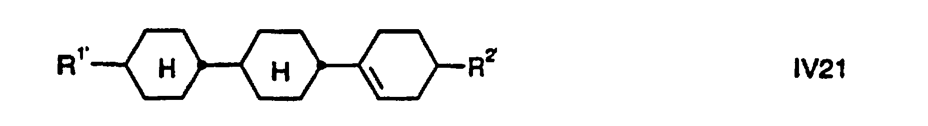 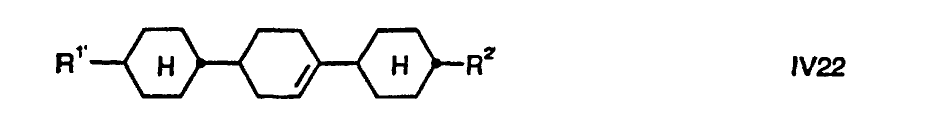 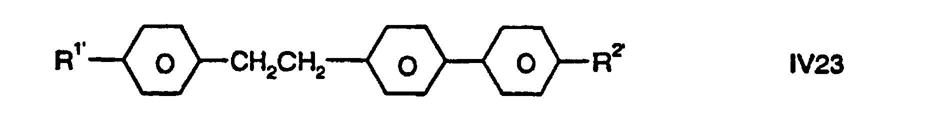 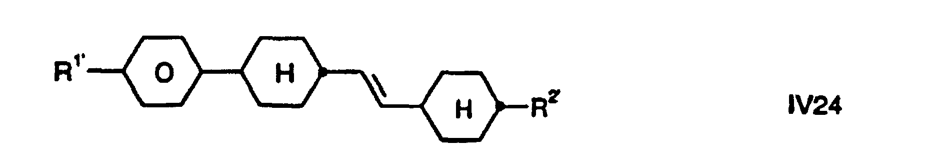 worin R^{1'} und R^{2'} die für R angegebene Bedeutung haben und die 1,4-Phenylengruppen in IV10 bis IV19, IV23 und IV24 jeweils unabhängig voneinander auch durch Fluor ein- oder mehrfach substituiert sein können.
- Komponente B enthält zusätzlich eine oder mehrere Verbindungen ausgewählt aus der Gruppe bestehend aus den Verbindungen der Formeln IV25 bis IV30, 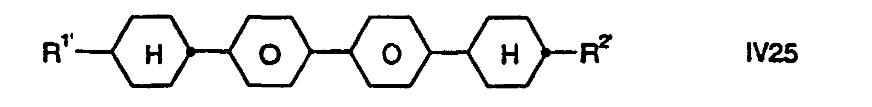 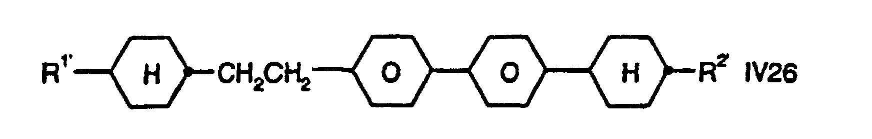 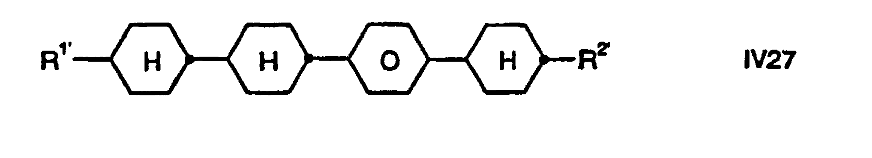 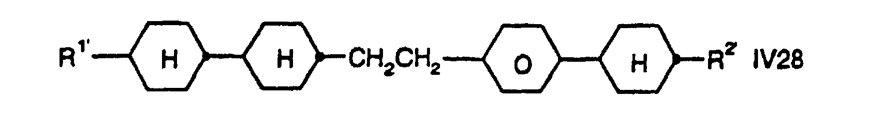 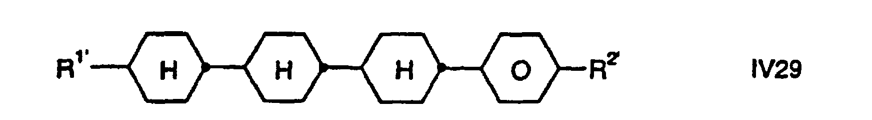 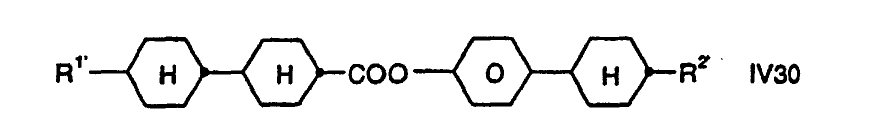 worin R^{1'} und R^{2'} die für R angegebene Bedeutung haben und die 1,4-Phenylengruppen in IV25 bis IV30 jeweils unabhängig voneinander auch durch Fluor ein- oder mehrfach substituiert sein können.
- Komponente B enthält eine oder mehrere Verbindungen ausgewählt aus der Gruppe bestehend aus IV31 und IV32: 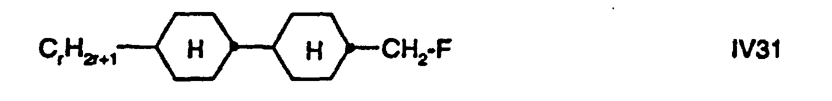 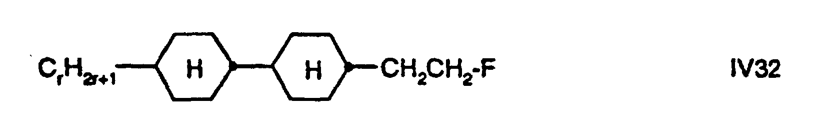 worin CᵣH₂ᵣ₊₁ eine geradkettige Alkylgruppe mit bis zu 9 C-Atomen ist.
- Die Flüssigkristallmischung enthält neben den Komponenten A, B und C zusätzlich ein oder mehrere Verbindungen ausgewählt aus der Gruppe bestehend aus den Verbindungen der Formeln III und IV, worin R^{1'} und R^{2'} die für R angegebene Bedeutung haben:

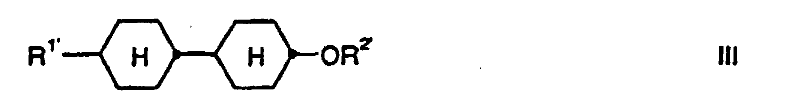

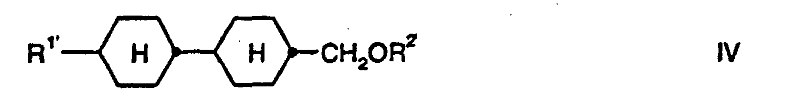

Bevorzugte Flüssigkristallmischungen enthalten mindestens eine Komponente, ausgewählt aus der Gruppe der folgenden Verbindungen; 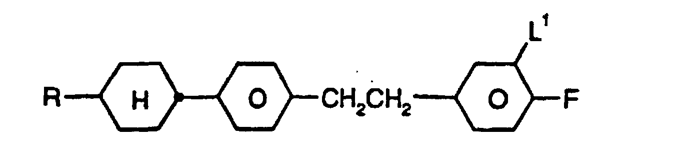 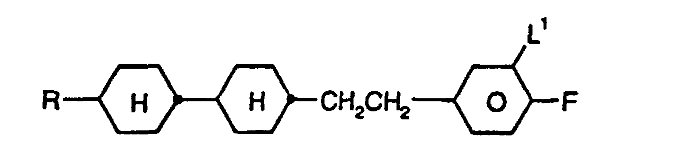 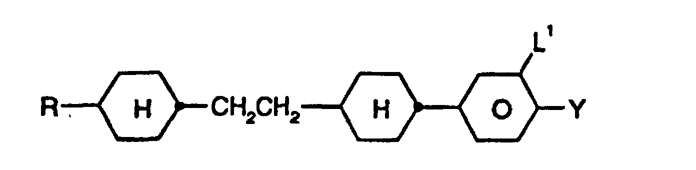 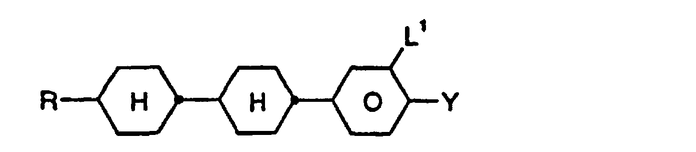 worin Y F oder Cl und L¹ H oder F ist und R die oben angegebene Bedeutung besitzt.

Die flüssigkristallinen Mischungen enthalten ebenfalls eine optisch aktive Komponente C in einer Menge, daß das Verhältnis zwischen Schichtdicke (Abstand der planparallelen Trägerplatten) und natürlicher Ganghöhe der chiralen nematischen Flüssigkristallmischung größer 0,2 ist. Für die Komponente stehen dem Fachmann eine Vielzahl, zum Teil kommerziell erhältlicher chiraler Dotierstoffe zur Verfügung z.B. wie Cholesterylnonanoat, S811 der Fa. E. Merck, Darmstadt, BRD und CB15 (BDH, Poole, UK). Die Wahl der Dotierstoffe ist an sich nicht kritisch.

Die erfindungsgemäße Flüssigkristallmischung enthält vorzugsweise eine oder mehrere Verbindungen ausgewählt aus der Gruppe B1 bestehend aus Verbindungen der Formeln B1I bis B1IV: 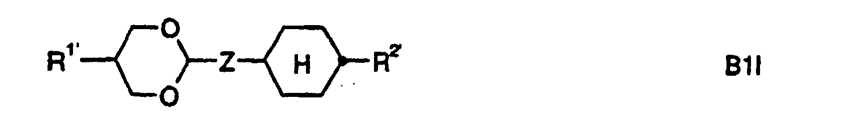 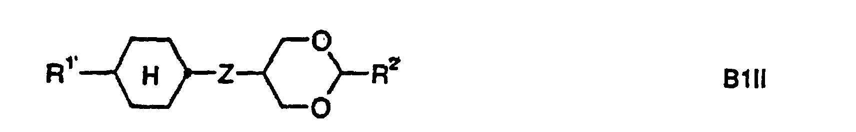 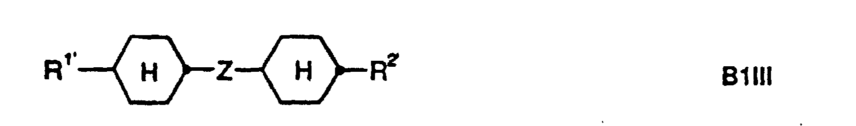 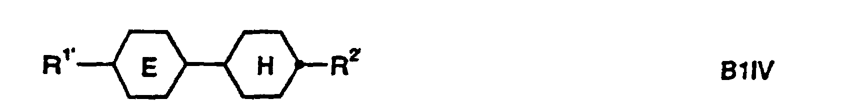 worin
- R^{1'} und R^{2'}: jeweils unabhängig voneinander die Bedeutung von R besitzen,
- Z: -CH₂CH₂-, -CO-O-, -O-CO- oder eine Einfachbindung, und 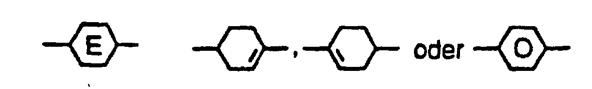
bedeuten,
und/oder wenigstens eine Verbindung ausgewählt aus der Gruppe B2 bestehend aus Verbindungen der Formeln B1V bis B1VII: 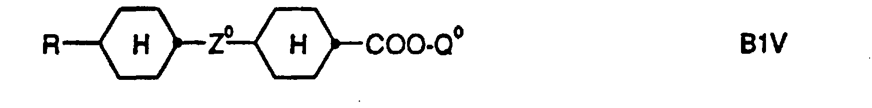 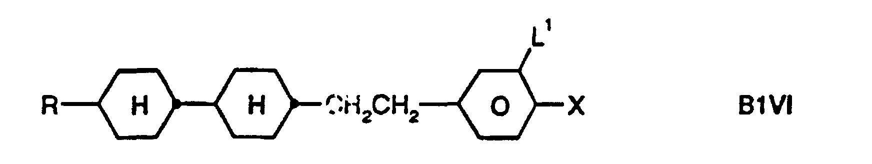 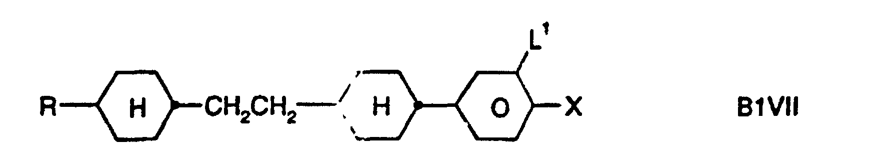 worin
- R: die oben angegebene Bedeutung besitzt,
- Z⁰: -CH₂CH₂- odei eine Einfachbindung,
- Q⁰: 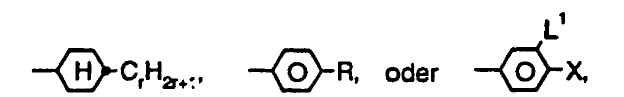 wobei r 1-9,
- X: CN oder F,
- L¹: H oder F
bedeuten,
und/oder wenigstens eine Verbindung ausgewählt aus der Gruppe B3 bestehend aus Verbindungen der Formeln B1VIII, B1IX und B1X: 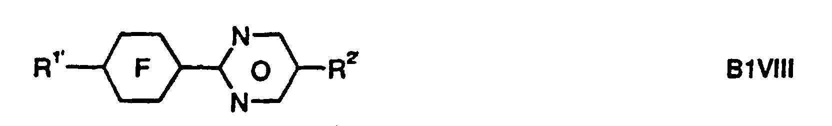 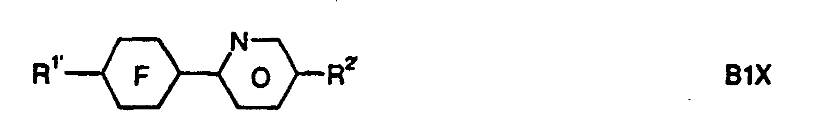 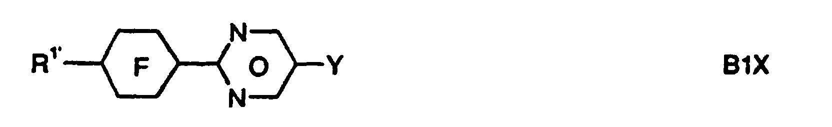 worin
- R^{1'} und R^{2'}: unabhängig voneinander die Bedeutung von R besitzen,
- Y: F oder Cl, und 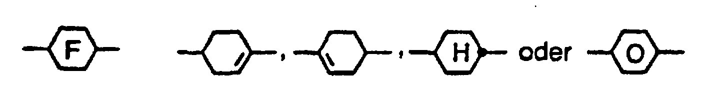
bedeuten.

In einer besonders bevorzugten Ausführungsform enthalten die erfindungsgemäßen Mischungen ca. 5 bis 35 %, insbesondere 10 bis 20 % an flüssigkristallinen Tolan-Verbindungen. Hierdurch kann bei geringeren Schichtdicken (ca. 5-6 µm) gearbeitet werden, wodurch die Schaltzeiten deutlich kürzer werden. Besonders bevorzugt sind Mischungen enthaltend ein oder mehrere Verbindungen ausgewählt aus der Gruppe T bestehend aus den Verbindungen der Formeln T1 bis T3: 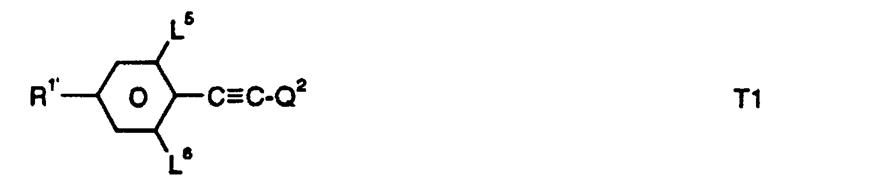 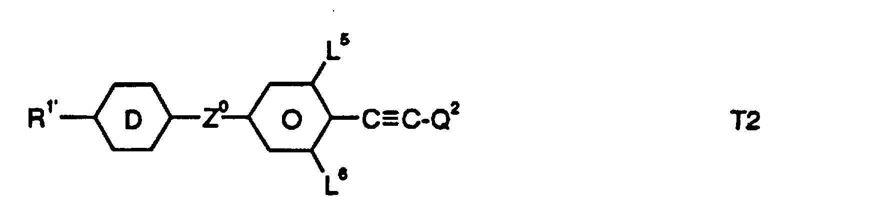 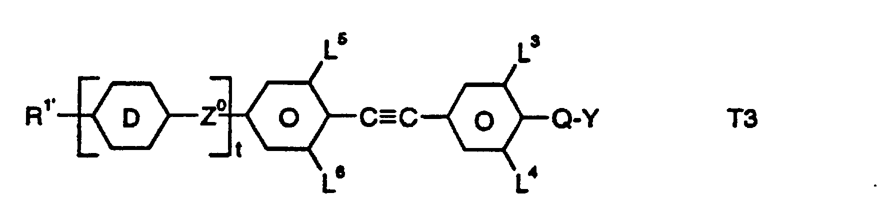 worin 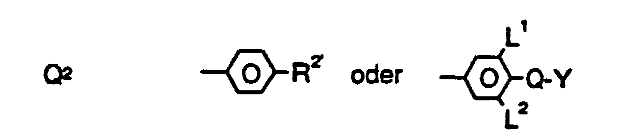 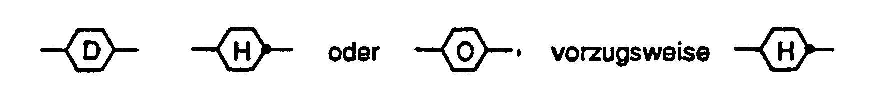
- t: 0 oder 1
- L¹⁻⁶: jeweils unabhängig voneinander H oder F
- Q: -CF₂-, -CHF-, -OCF₂-, -OCHF- oder eine Einfachbindung
- Y: F oder Cl
- R^{1'} und R^{2'}: jeweils unabhängig voneinander die für R angegebene Bedeutung,
besitzen.

Der Anteil der Verbindungen aus der Gruppe T ist vorzugsweise 5 bis 30 %, insbesondere 5 bis 20 %.

Vorzugsweise enthält Komponente B ein oder mehrere Verbindungen der Formeln X bis XII: 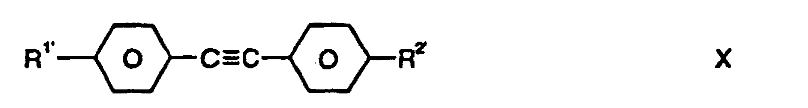 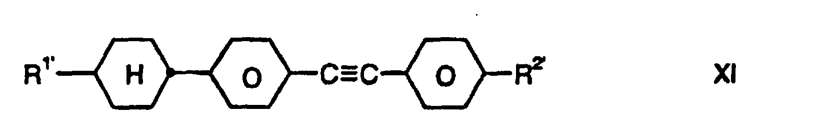 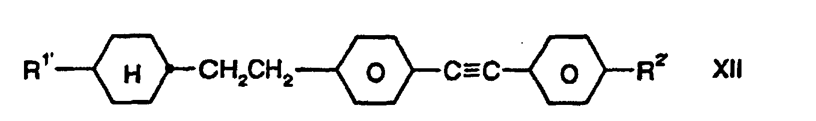 worin
- R^{1'} und R^{2'}: jeweils unabhängig voneinander die für R angegebene Bedeutung besitzen und R^{1'} vorzugsweise Alkyl mit 1 bis 4 C-Atmen, insbesondere 1 bis 2 C-Atomen und R^{2'} vorzugsweise Alkoxy mit 1 bis 4 C-Atomen, insbesondere 1 bis 2 C-Atomen,
bedeuten.

Der Anteil der Verbindungen aus der Gruppe B1 beträgt vorzugsweise 10 bis 50 %, insbesondere 15 bis 40 %. Verbindungen der Formel B1III und B1IV sind bevorzugt.

Besonders bevorzugte Verbindungen der Formel B1III sind diejenigen der folgenden Teilformeln B1IIIa und B1IIIb, 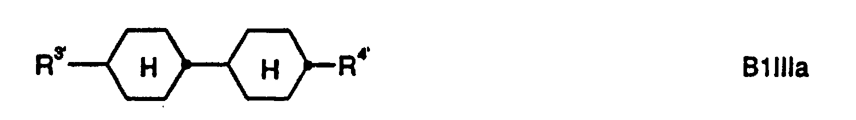 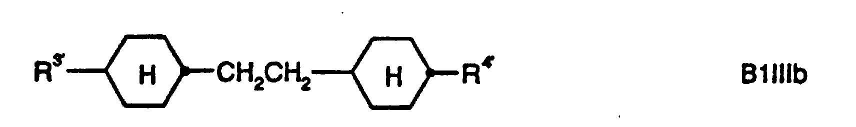 worin
- R^{3'}: CH₃-(CH₂)ₒ-O-, CH₃-(CH₂)ₚ-, trans-H-(CH₂)_{q}-CH=CH-(CH₂CH₂)_{b}-CH₂O- oder trans-H-(CH₂)_{q}-CH=CH-(CH₂CH₂)_{b}-, CH₃-(CH₂)ₒ-O-CH₂-,
- R^{4'}: CH₃-(CH₂)ₚ-,
- o: 1, 2, 3 oder 4,
- q: 0, 1, 2 oder 3,
- b: 0 oder 1, und
- p: 1, 2, 3 oder 4
ist.

Bevorzugt sind insbesondere Verbindungen der Formel B1III, worin einer der Reste R^{3'} oder R^{4'} O-(CH₂)ₒ-CH₃ oder CH₂-O-(CH₂)ₒ-CH₂bedeutet.

Femer bevorzugt sind diejenigen Verbindungen der Teilformel, 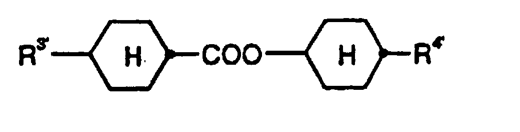 worin R^{3'} und R^{4'} jeweils unabhängig voneinander die oben angegebene Bedeutung haben.

Der Anteil der Verbindungen der Formel B1III der oben angegebenen Teilformeln ist vorzugsweise ca. 5 bis 45 %, insbesondere bevorzugt ca. 10 % bis 35 %. Besonders bevorzugte Verbindungen der Formel B1IV sind diejenigen der folgenden Teilformel, 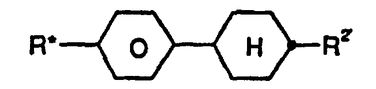 worin
- R*: CH₃-(CH₂)ₒ-O- oder trans-H-(CH₂)_{q}-CH=CH-(CH₂CH₂)_{b}-CH₂O- und
- R^{2'}: CH₃-(CH₂)ₚ- ist, wobei
- o: 1, 2, 3 oder 4,
- q: 0, 1, 2 oder 3,
- b: 0 oder 1, und
- p: 1, 2, 3 oder 4
ist.

Der Anteil dieser Verbindungen, bzw. der Verbindungen der Formel BIV, ist vorzugsweise ca. 5 bis 40 %, insbesondere bevorzugt ca. 10 bis 35 %.

Vorzugsweise enthalten die Mischungen Verbindungen der Formel B1III, insbesondere solche der Teilformel

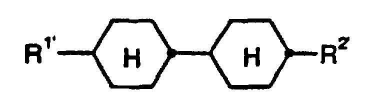

In einer besonders bevorzugten Ausführungsform enthalten die Mischungen gleichzeitig Verbindungen der Formeln B1III und B1IV, wobei der Gesamtanteil für Komponenten der Gruppe B1 gewahrt bleibt.

Falls Verbindungen der Formeln B1I und/oder B1III vorhanden sind, bedeuten R² und R³ vorzugsweise jeweils unabhängig voneinander n-Alkyl mit 1 bis 7 C-Atomen oder (trans)-n-Alkenyl mit 3 bis 7 C-Atomen. Z ist vorzugsweise eine Einfachbindung.

Femer bevorzugt sind erfindungsgemäße Mischungen, die einer oder mehrere Verbindungen der Formel B1IV enthalten, worin 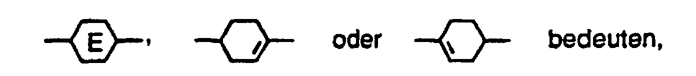
- R^{1'} und R^{2'}: eine der oben angebenen bevorzugten Bedeutungen haben, insbesondere bevorzugt n-Alkyl mit 1 bis 7 C-Atomen bedeuten.

In jedem Fall bleibt der Gesamtanteil für Komponenten der Gruppe B1 gewahrt.

Der Anteil der Verbindungen der Gruppe B2 beträgt vorzugsweise ca. 5 bis 45 %, insbesondere 5 bis 20 %. Der Anteil (bevorzugte Bereiche) für B1V bis B1VII ist wie folgt:
- B1V:: ca. 5 bis 30 %, vorzugsweise ca. 5 bis 15 %

### Summe B1VI

- und B1VII:: ca. 5 bis 25 %, vorzugsweise ca. 10 bis 20 %.

Bevorzugte Verbindungen der Gruppe B2 sind im folgenden angegeben:

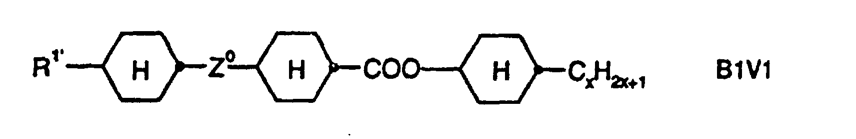

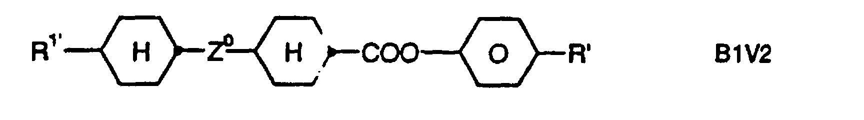

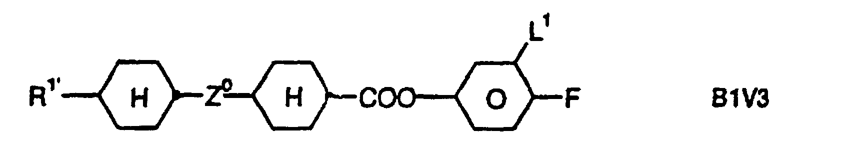

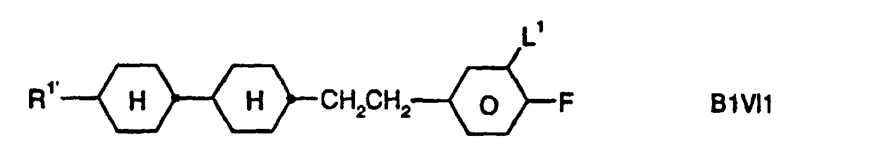

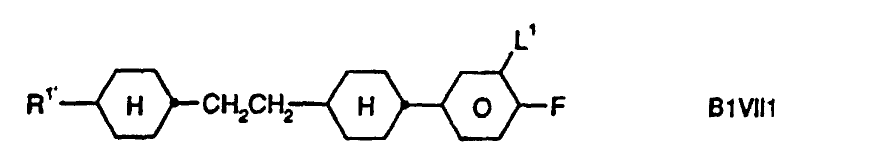

R' ist vorzugsweise n-Alkyl mit 1 bis 7 C-Atomen oder (trans)-n-Alkenyl mit 3 bis 7 C-Atomen. Z⁰ ist vorzugsweise eine Einfachbindung. R^{1'} hat vorzugsweise die oben für R angegebene bevorzugte Bedeutung oder bedeutet Fluor. L¹ ist vorzugsweise Fluor, x ist 1-15.

Vorzugsweise enthalten die erfindungsgemäßen Mischungen eine oder mehrere Verbindungen ausgewählt aus der Gruppe bestehend aus B1V3, B1VI1 und B1VII1 in einem Gesamtanteil von ca. 5 bis 35%.

In einer besonders bevorzugten Ausführungsform enthalten die erfindungsgemäßen Mischungen neben B1V3, B1VI1, B1VII1 (R = F) weitere terminal fluorierte Verbindungen zum Beispiel ausgewählt aus der Gruppe bestehend aus. 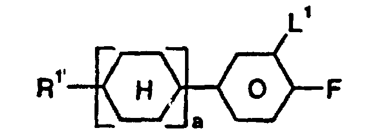 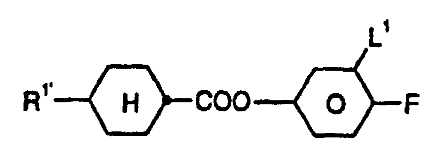 und/oder polare Heterocyclen ausgewählt aus der Gruppe bestehend aus 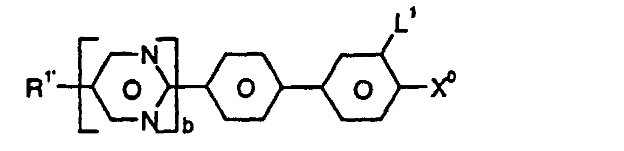 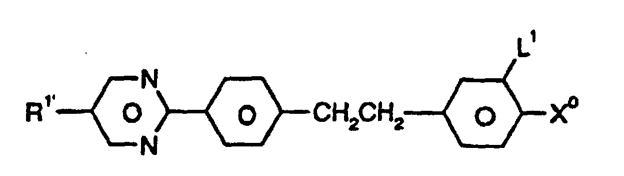 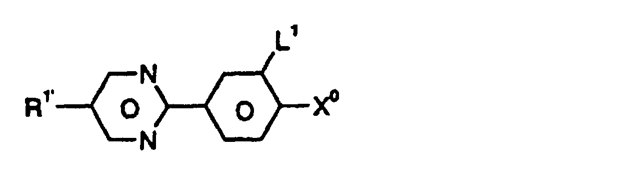 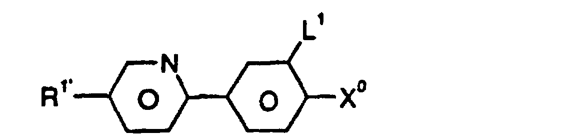 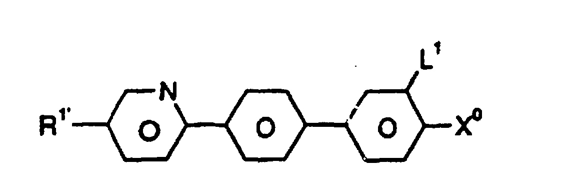 worin R^{1'} vorzugsweise n-Alkyl mit 1 bis 7 C-Atomen oder (trans)-n-Alkenyl mit 3 bis 7 C-Atomen, a 1 oder 2, b 0 oder 1, X⁰ F, Cl, CF₃, -OCF₃ oder -OCHF₃, und L¹ H oder F bedeutet.

Der Gesamtanteil aller terminal fluorierter Verbindungen beträgt vorzugsweise ca. 5 bis 65 %, insbesondere ca. 15 bis 40 %.

Der Anteil der Verbindungen aus Gruppe B3 beträgt vorzugsweise ca. 5 bis 30 %, insbesondere bevorzugt ca. 10 bis 20 %. R^{2^{'}} ist vorzugsweise n-Alkyl oder n-Alkoxy mit jeweils 1 bis 9 C-Atomen.

Es können jedoch auch analoge Verbindungen mit Alkenyl- bzw. Alkenyloxy-Gruppen eingesetzt werden. Verbindungen der Formel B1VIII sind bevorzugt.

Die erfindungsgemäßen Mischungen enthalten vorzugsweise Verbindungen der Formel I und Verbindungen aus mindestens einer der Gruppen B1, B2 und B3. Vorzugsweise enthalten sie eine oder mehrere Verbindungen aus Gruppe B1 und eine oder mehrere Verbindungen aus Gruppe B2 und/oder B3.

Der Anteil der Verbindungen der Komponente C beträgt vorzugsweise 0 bis 20 %, insbesondere 0 bis 10 %.

In einer besonders bevorzugten Ausführungsform enthalten die erfindungsgemäßen Mischungen vorzugsweise ca. 5 bis 20 % einer oder mehrerer Verbindungen mit einer dielektrischen Anisotropie von unter -2 (Komponente C). Derartige Verbindungen sind bekannt, z.B. Derivate der 2,3-Dicyanhydrochinone oder Cyclohexanderivate mit dem Strukturelement 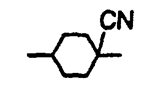 gemäß DE-OS 32 32 707 bzw. DE-OS 34 07 013.

Vorzugsweise werden jedoch Verbindungen mit dem Strukturelement 2,3-Difluor-1,4-phenylen gewählt, z.B. Verbindungen gemäß DE-OS 38 07 801, 38 07 861, 38 07 863, 38 07 864 oder 38 07 908. Besonders bevorzugt sind Tolane mit diesem Strukturelement gemäß der Internationalen Patentanmeldung PCT/DE 88/00133, insbesondere solche der Formeln, 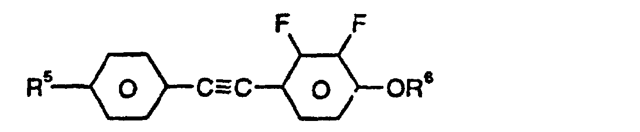 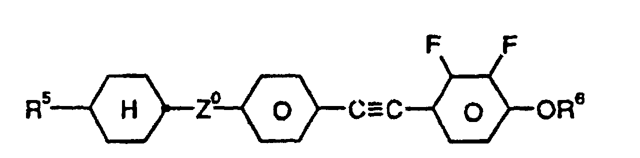 worin R⁵ und R⁶ jeweils unabhängig voneinander vorzugsweise n-Alkyl mit 1 bis 7 C-Atomen oder n-Alkenyl mit 3 bis 7 C-Atomen bedeuten und Z⁰ -CH₂CH₂- oder eine Einfachbindung ist.

Besonders bevorzugt sind Phenylcyclohexylcarboxylate der Formeln 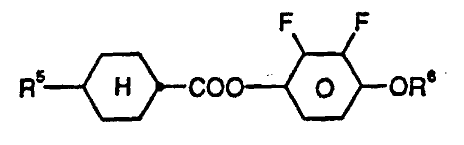 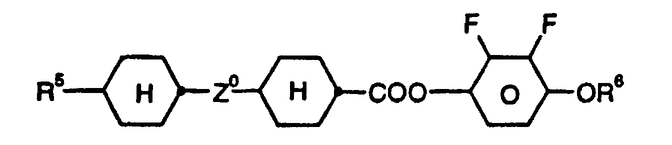
- Die Komponente C enthält eine oder mehrere Verbindungen ausgewählt aus der Gruppe bestehend aus V bis IX: 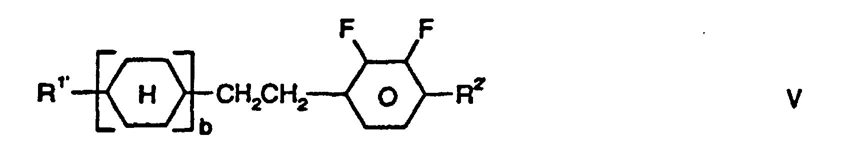 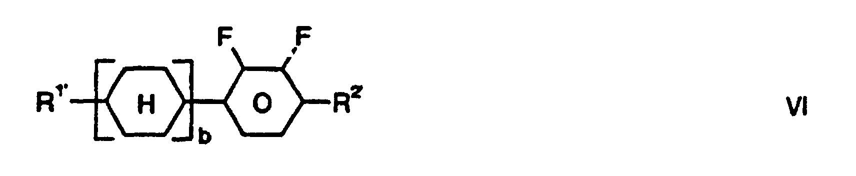 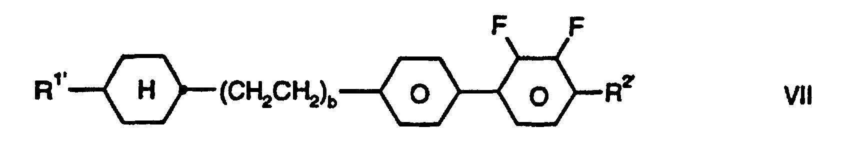 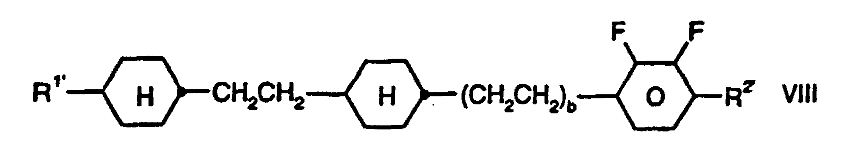 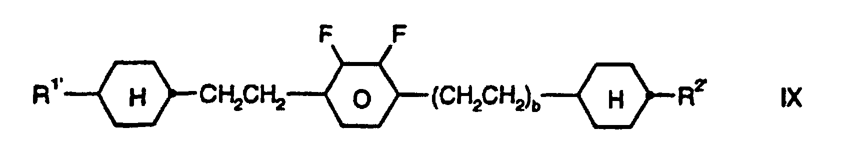 worin R^{1'} und R^{2'} die für R angegebene Bedeutung haben und b 0 oder 1 ist.
- Die Komponente B enthält eine oder mehrere Verbindungen ausgewählt aus der Gruppe bestehend aus Xa bis XIIa, 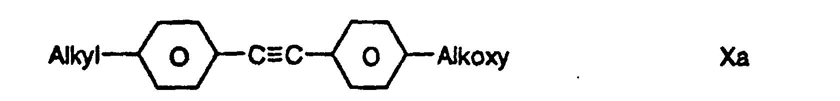 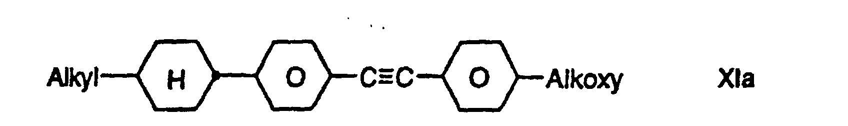 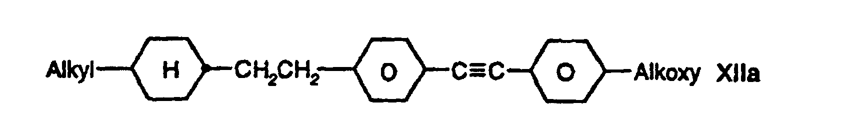 worin Alkyl und Alkoxy einen Alkyl- oder Alkoxyrest mit 1 bis 7 C-Atomen bedeuten.

Insbesondere Komponente C führt zu einer erhöhten Steilheit der Kennlinie.

In einer besonders bevorzugten Ausführungsform enthalten die Mischungen ca. 5 bis 35 %, insbesondere bevorzugt ca. 10 bis 20 % an flüssigkristallinen Tolan-Verbindungen. Hierdurch kann bei geringeren Schichtdicken (ca. 5-6 µm) gearbeitet werden, wodruch die Schaltzeiten deutlich kürzer werden. Besonders bevorzugte Tolane sind im folgenden angegeben: 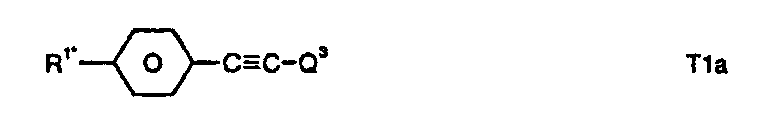 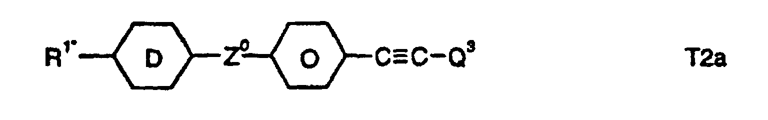
- R^{1'}: ist vorzugsweise n-Alkyl oder n-Alkoxy mit 1 bis 7 C-Atomen,
- Z⁰: ist -CH₂CH₂- oder eine Einfachbindung, 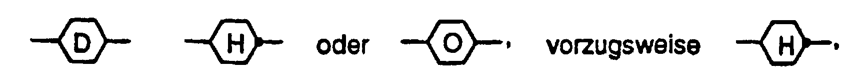
- Q^{s}: ist 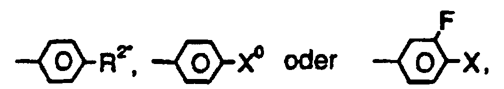 wobei
- X⁰: ist F, Cl oder OCF₃, wobei
- R^{2^{'}}: n-Alkyl oder n-Alkoxy mit jeweils 1 bis 7 C-Atomen oder n-Alkenyl oder n-Alkenyloxy mit jeweils 3 bis 7 C-Atomen bedeutet

Vorzugsweise enthält Komponente A ein oder mehrere Verbindungen der Formel T3a, 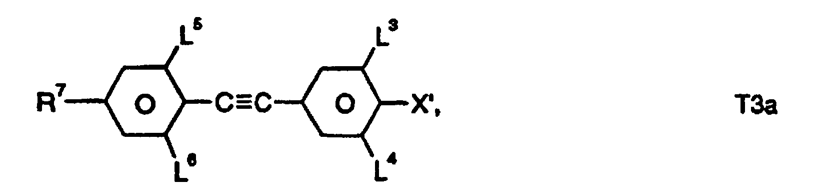 worin
- R⁷: -CₓH₂ₓ₊₁, -OCₓH₂ₓ₊₁, 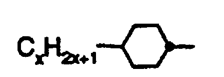 oder 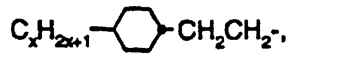
- x: eine ganze Zahl von 1-15,
- L³⁻⁶: jeweils unabhängig voneinander H oder F, und
- X': F, Cl oder OCF₃
bedeuten.

Im folgenden weitere besonders bevorzugte Ausführungsformen:
- Die Komponente C enthält ein oder mehrere Verbindungen mit einer 1-Cyano-trans-1,4-cyclohexylgruppe oder einer 2,3-Difluor-1,4-phenylengruppe
- mindestens zwei Verbindungen der Formeln AIII oder AV
- Verbindungen der Formeln AIII und AV
- mindestens eine Verbindung aus der Gruppe: 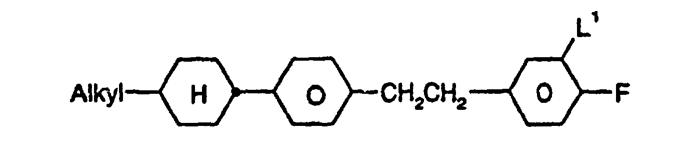 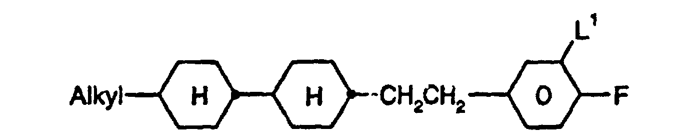 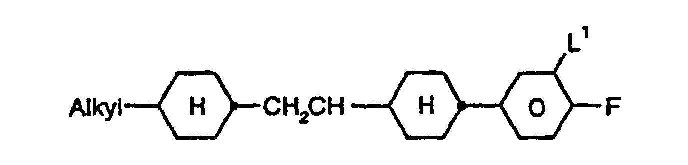 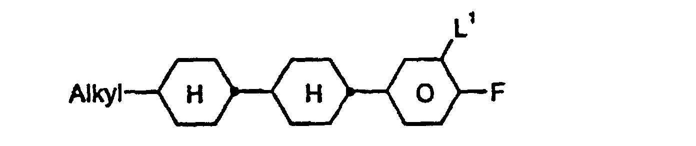 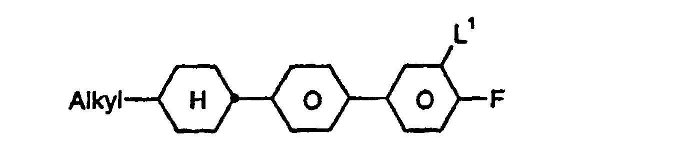 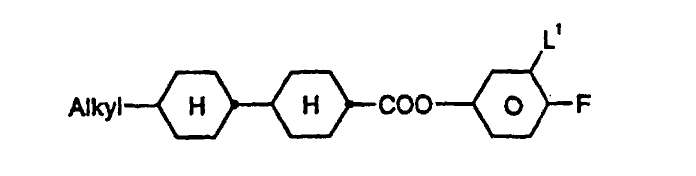 worin Alkyl ein geradkettiger Alkylrest mit 2 bis 7 C-Atomen und L¹ H oder F bedeutet;
- ein oder mehrere Verbindungen, worin R eine trans-Alkenylgruppe oder ein trans-Alkenyloxygruppe ist;
- ein oder mehrere Verbindungen ausgewählt aus der folgenden Gruppe: 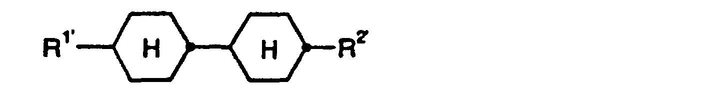 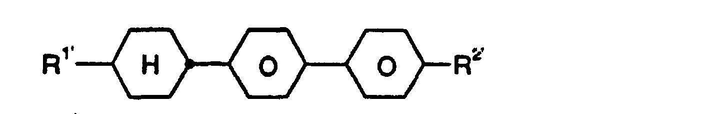 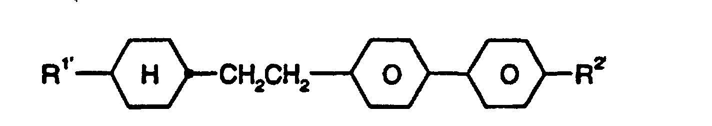 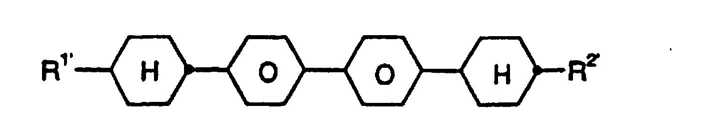 worin R^{1'} und R^{2'} die bevorzugten Bedeutungen, die unter Verbindungen der Komponente B genannt sind, besitzen. Die 1,4-Phenylengruppe in den oben genannten Verbindungen kann auch durch Fluor substituiert sein. Der Anteil dieser Verbindungen in den Flüssigkristallmischungen liegt bei 0 bis 25 %, vorzugsweise 5 bis 15 %.

In einer weiteren bevorzugten Ausführungsform enthalten die Mischungen
- ein oder mehrere, insbesondere 1, 2, 3 oder 4 Verbindungen ausgewählt aus den Verbindungen der Formeln IIId, IIIb, IIIi und IIIp;
- wenigstens zwei Verbindungen ausgewählt aus den Verbindungen der Formeln IIb1, IIc1 oder IIc2;
- ein oder mehrere Verbindungen der Formel B1IV;
- ein oder mehrere Verbindungen der Formel T1 oder T2;
- ein oder mehrere Verbindungen der Formeln 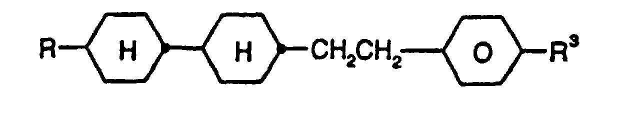 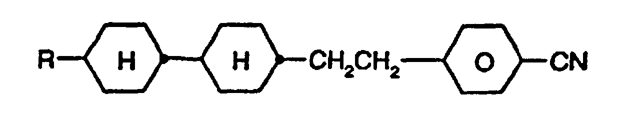 worin R die bei Formel III angegebene Bedeutung hat.

Die erfindungsgemäßen Mischungen zeichnen sich insbesondere beim Einsatz in SFAs mit hohen Schichtdicken durch sehr niedrige Summenschaltzeiten aus (= tₒₙ + t_{off}).

Niedrige Summenschaltzeiten sind insbesondere ein wichtiges Kriterium für SFAs beim Einsatz als Anzeigen von Laptops, um Cursorbewegungen störungsfrei darstellen zu können.

Die in den erfindungsgemäßen SFAs verwendeten Flüssigkristallmischungen sind dielektrisch positiv mit Δε ≥ 1. Besonders bevorzugt sind Flüssigkristallmischungen mit Δε ≥ 3 und ganz besonders solche mit Δε ≥ 5.

Die erfindungsgemäßen Flüssigkristallmischungen weisen günstige Werte für die Schwellenspannung V_{10/0/20} und für die Fließviskosität η auf. Ist der Wert für den optischen Wegunterschied d.Δn vorgegeben, wird der Wert für die Schichtdicke d durch die optische Anisotropie Δn bestimmt. Insbesondere bei relativ hohen Werten für d.Δn ist i.a. die Verwendung erfindungsgemäßer Flüssigkristallmischungen mit einem relativ hohen Wert für die optische Anisotropie bevorzugt, da dann der Wert für d relativ klein gewählt werden kann, was zu günstigeren Werten für die Schaltzeiten führt. Aber auch solche erfindungsgemäßen Flüssigkristallanzeigen, die erfindungsgemäße Flüssigkristallmischungen mit kleineren Werten für Δn enthalten, sind durch vorteilhafte Werte für die Schaltzeiten gekennzeichnet. Die erfindungsgemäßen Flüssigkristallmischungen sind weiter durch vorteilhafte Werte für die Steilheit der elektrooptischen Kennlinie gekennzeichnet und können mit hohen Multiplexraten betrieben werden. Darüber hinaus weisen die erfindungsgemäßen Flüssigkristallmischungen eine hohe Stabilität und günstige Werte für den elektrischen Widerstand und die Frequenzabhängigkeit der Schwellenspannung auf. Die erfindungsgemäßen Flüssigkristallanzeigen weisen einen großen Arbeitstemperaturbereich und eine gute Winkelabhängigkeit des Kontrastes auf.

Der Aufbau der erfindungsgemäßen Flüssigkristall-Anzeigeelemente aus Polarisatoren, Elektrodengrundplatten und Elektroden mit einer solchen Oberflächenbehandlung, daß die Vorzugsorientierung (Direktor) der jeweils daran angrenzenden Flüssigkristall-Moleküle von der einen zur anderen Elektrode gewöhnlich um betragsmäßig 160° bis 720° gegeneinander verdreht ist, entspricht der für derartige Anzeigeelemente üblichen Bauweise. Dabei ist der Begriff der üblichen Bauweise hier weit gefaßt und umfaßt auch alle Abwandlungen und Modifikationen der Supertwistzelle, insbesondere auch Matrix-Anzeigeelemente sowie die zusätzliche Magnete enthaltenden Anzeigeelemente. Der Oberflächentiltwinkel an den beiden Trägerplatten kann gleich oder verschieden sein. Gleiche Tiltwinkel sind bevorzugt.

Ein wesentlicher Unterschied der erfindungsgemäßen Anzeigeelemente zu den bisher üblichen auf der Basis der verdrillten nematischen Zelle besteht jedoch in der Wahl der Flüssigkristallkomponenten der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe zugesetzt werden.

Die folgenden Beispiele sollen die Erfindung erläutern, ohne sie zu begrenzen.

Es bedeutet:
- S-N: Phasenübergangs-Temperatur smektisch-nematisch
- N-I: Phasenübergangs-Temperatur nematisch-isotrop
- Klp.: Klärpunkt
- Visk.: Viskosität (m Pa.s)
- tₒₙ: Zeit vom Einschalten bis zur Erreichung von 90 % des maximalen Kontrastes
- t_{off}: Zeit vom Ausschalten bis zur Erreichung von 10 % des maximalen Kontrastes
- V₉₀/V₁₀: Steilheit
- tₐᵥₑ: $\frac{{\text{t}}_{\text{on}} {\text{+ t}}_{\text{off}}}{\text{2}}$ (mittlere Schaltzeit)

Die SFA wird im Multiplexbetrieb angesteuert (Multiplexverhältnis) 1:240, Bias 1:16, Betriebsspannung 10 Volt, so daß tₒₙ = t_{off} ist.

Vor- und nachstehend sind alle Temperaturen in °C angegeben. Die Prozentzahlen sind Gewichtsprozente. Die Werte für die Schaltzeiten und Viskositäten beziehen sich auf 20 °C.

In der vorliegenen Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R^{1'}, R^{2'}, L² und L²:

| Code für R^{1'}, R^{2'}, L¹, L² | R^{1'} | R^{2'} | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-C₂H₂ₛ- | CN | H | H |
| nNF | CₙH₂ₙ₊₁ | CN | F | H |
| nAm | CₙH₂ₙ₊₁ | COOCₘH₂ₘ₊₁ | H | H |
| nF.Cl | CₙH₂ₙ₊₁ | Cl | H | F |

Bevorzugte Mischungen enthalten neben ein oder mehreren Verbindungen der Formel I ein oder mehrere Verbindungen der in den Tabellen A und B genannten Verbindungen.

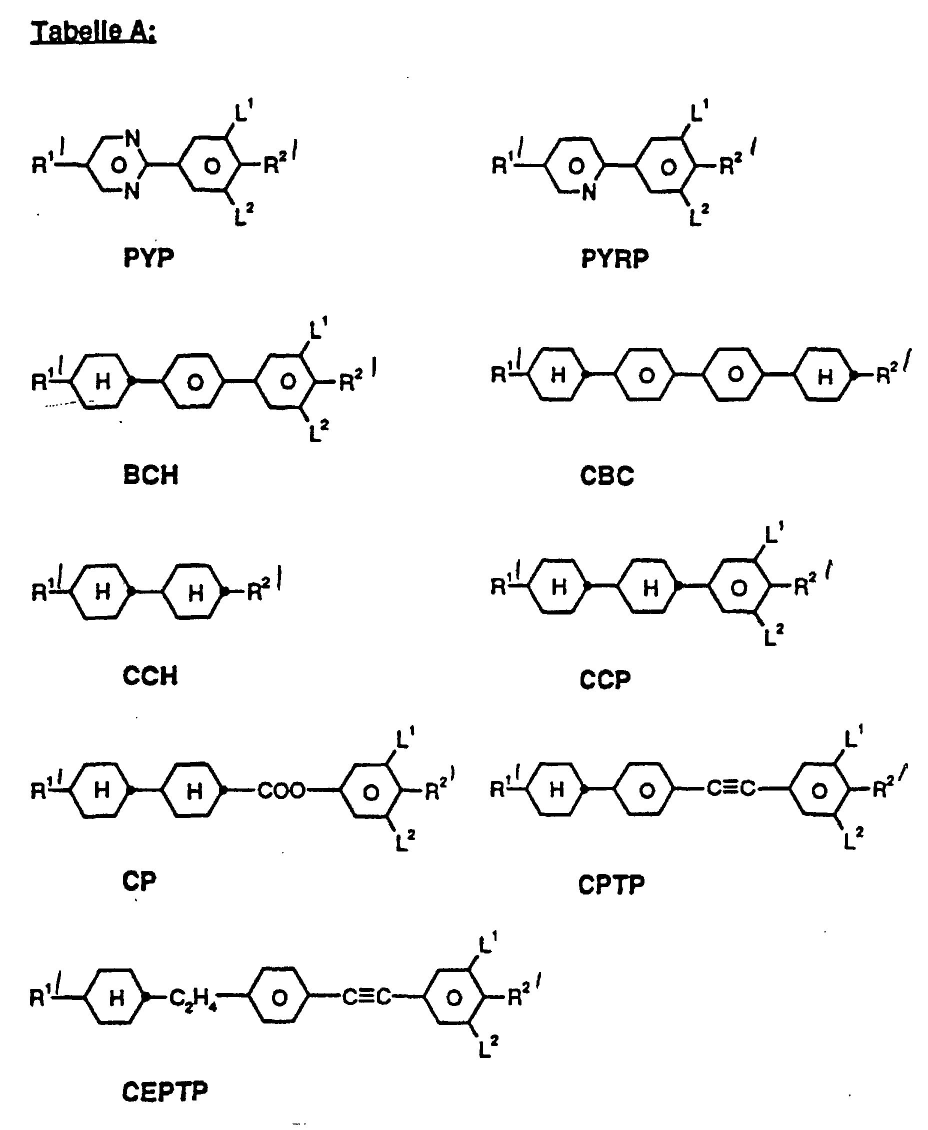

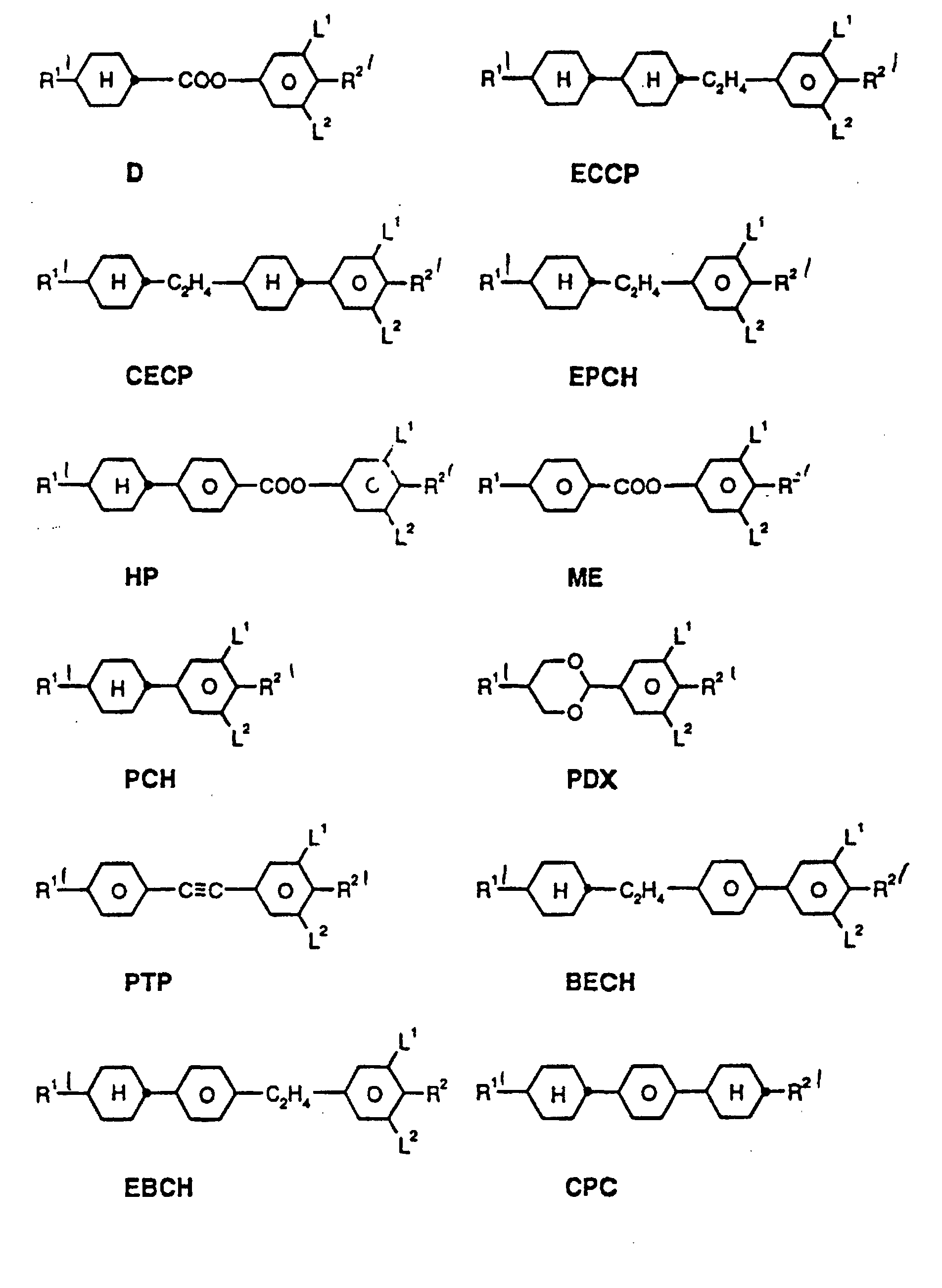

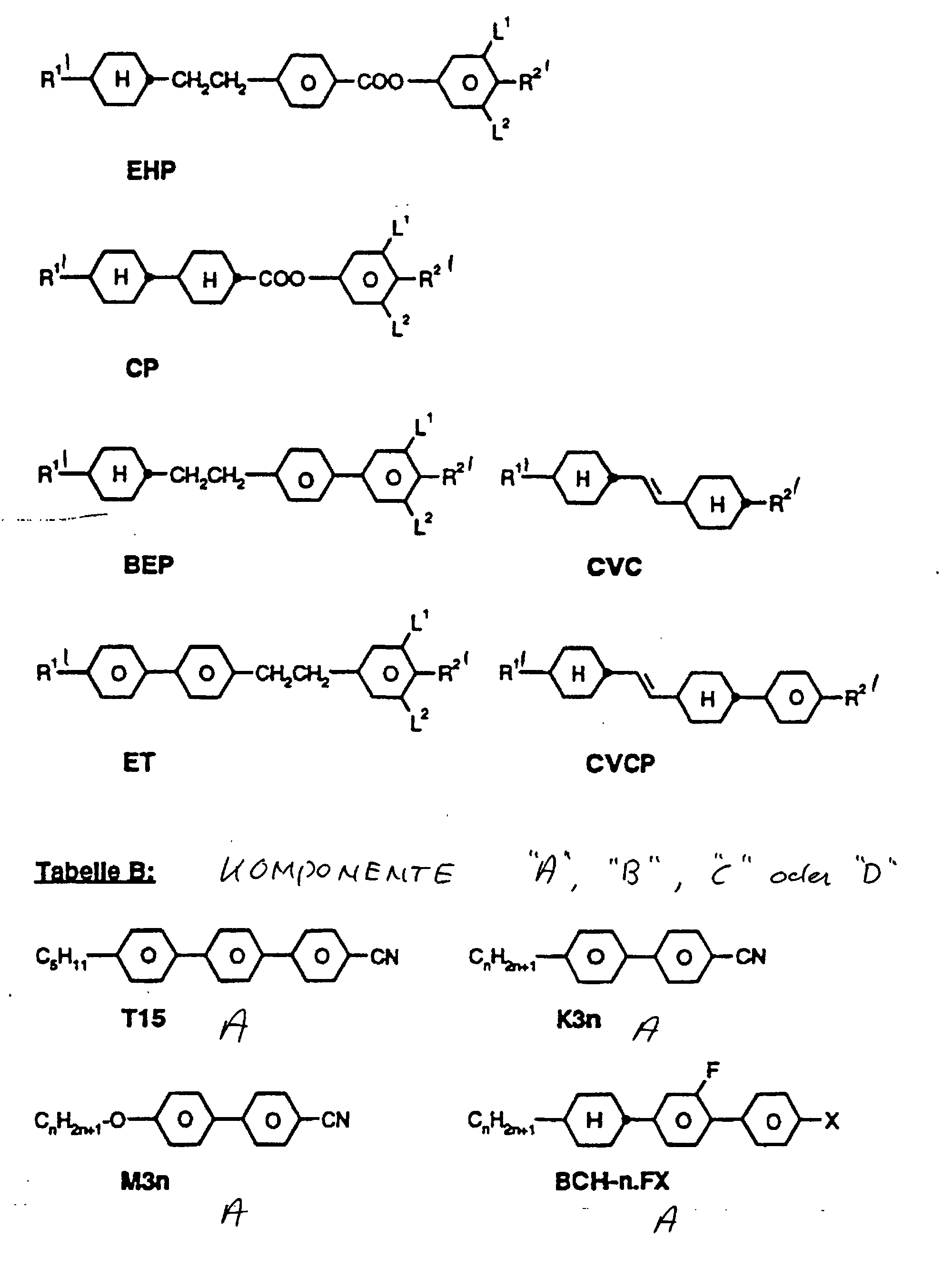

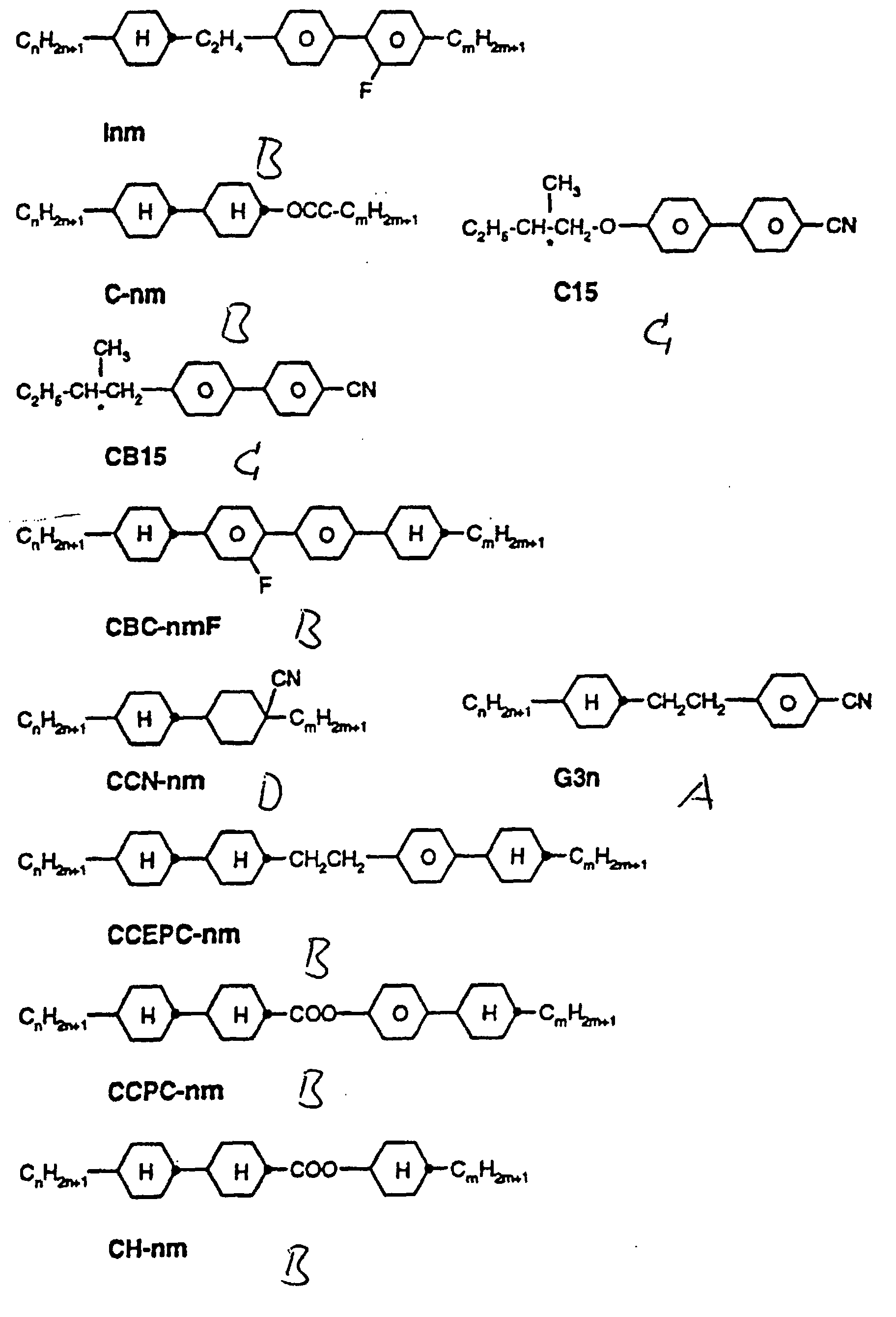

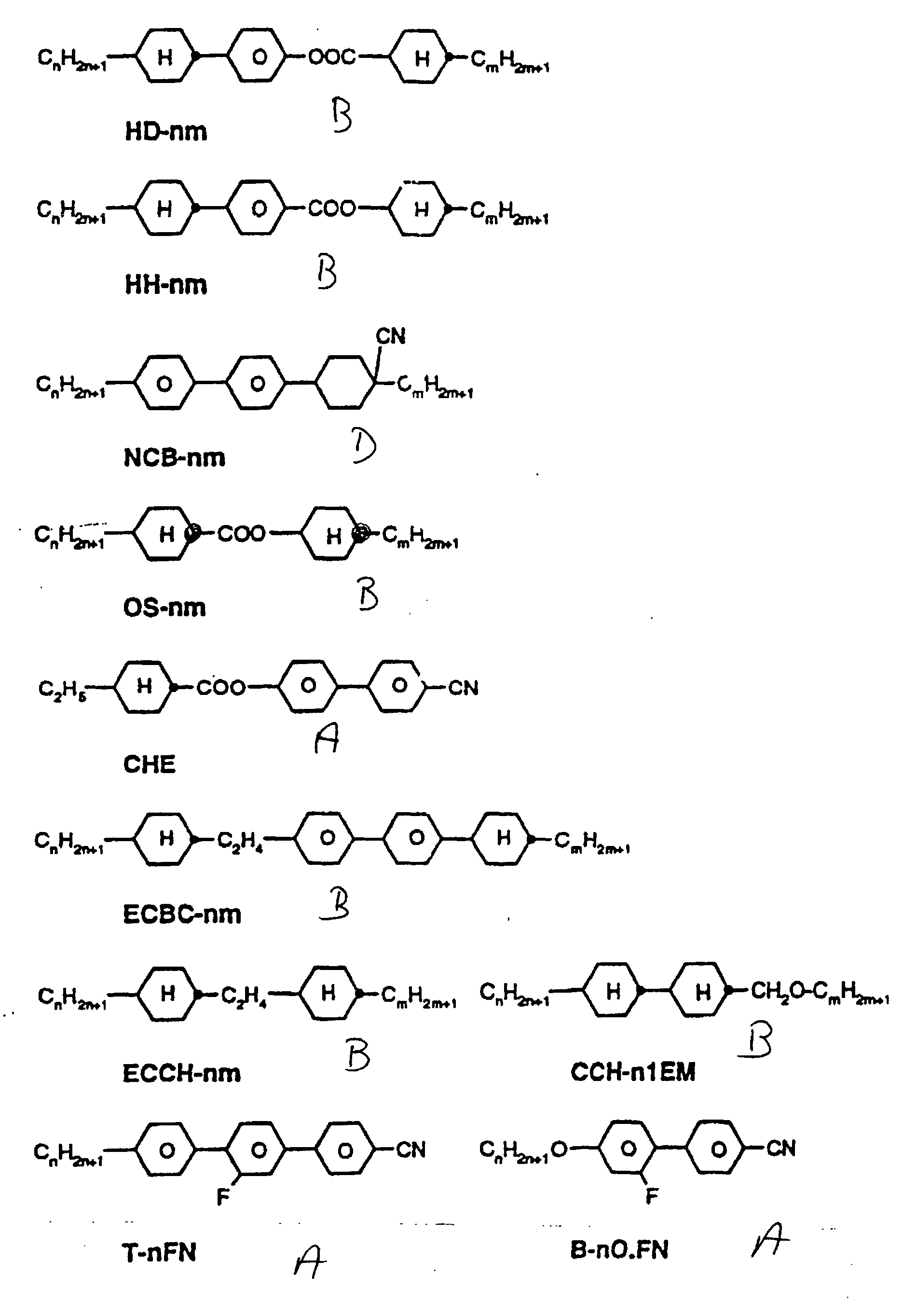

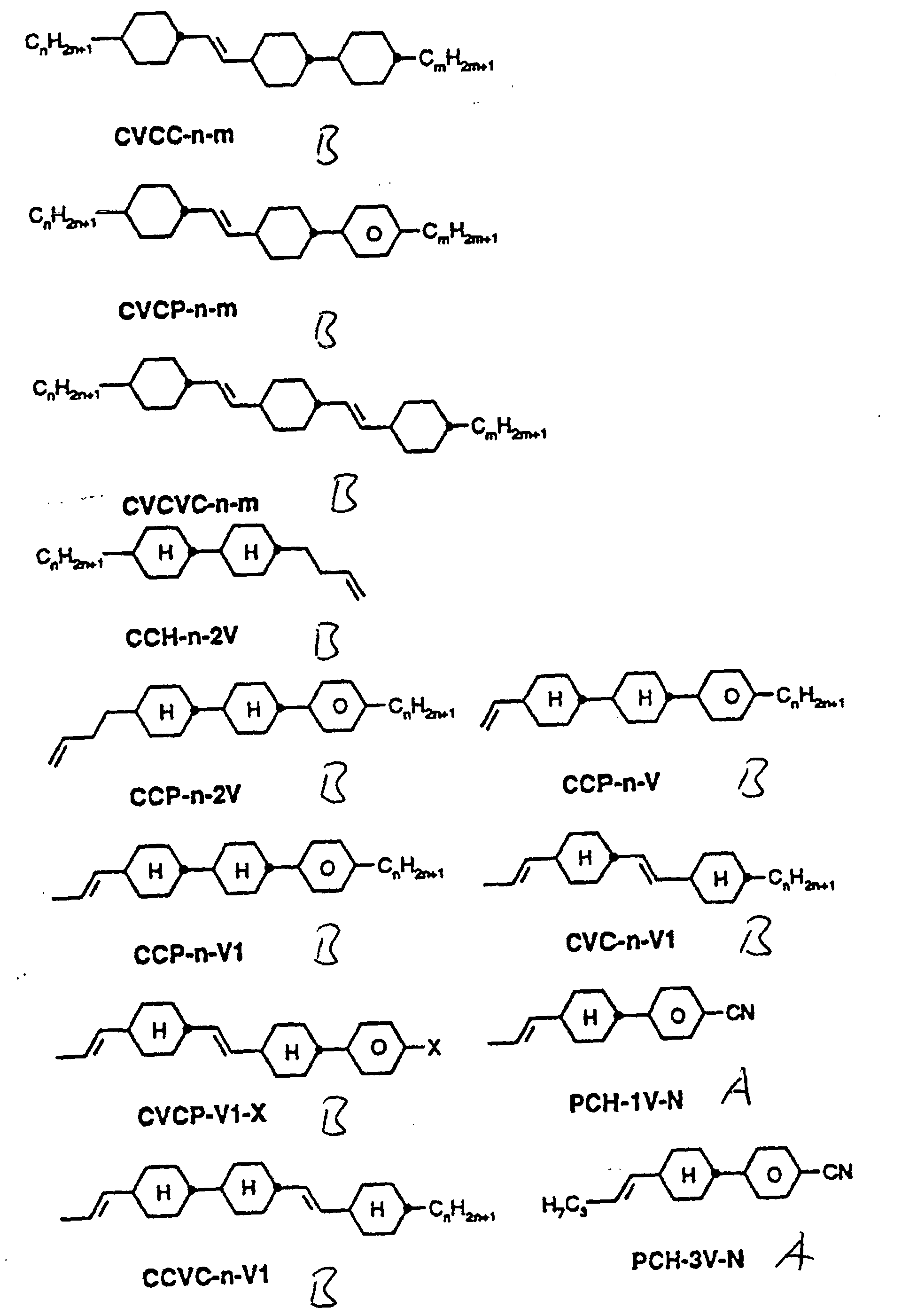

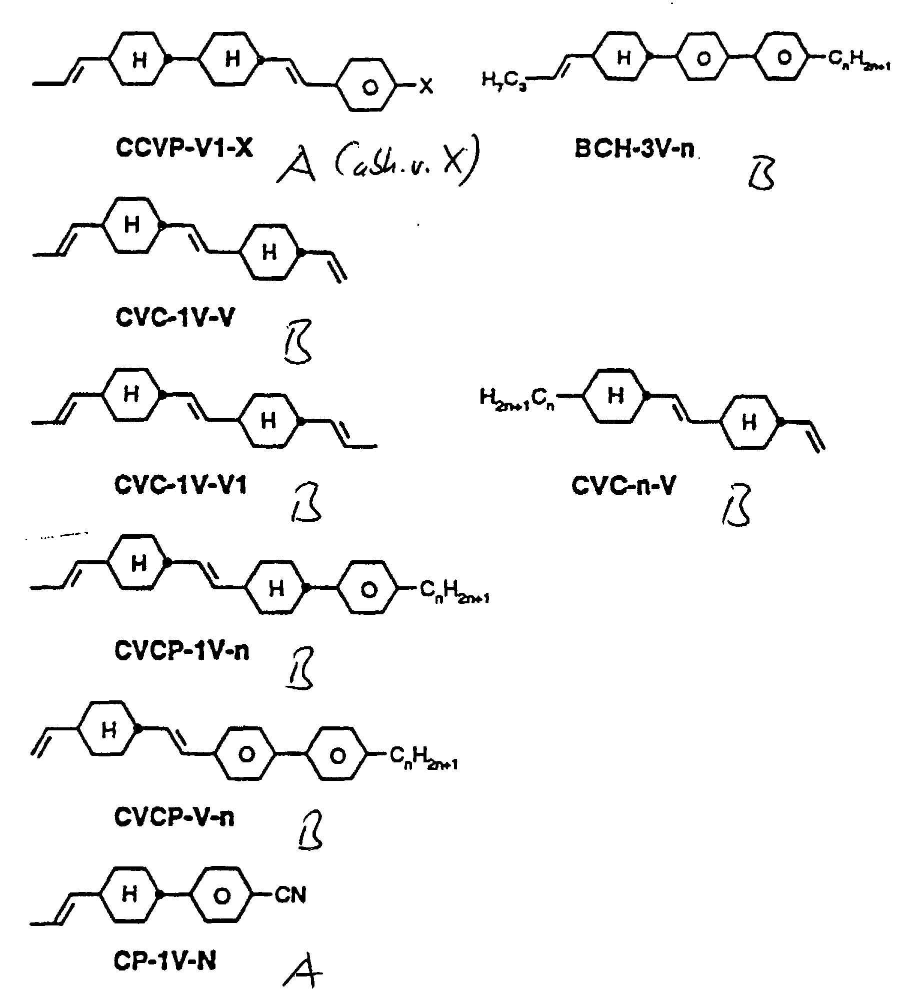

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DMEU: 1,3-Dimethyl-2-imidazolidinon
- KOT: Kalium-tertiär-butanolat
- THF: Tetrahydrofuran
- pTsOH: p-Toluolsulfonsäure

### Beispiel 1

### Schritt 1.1

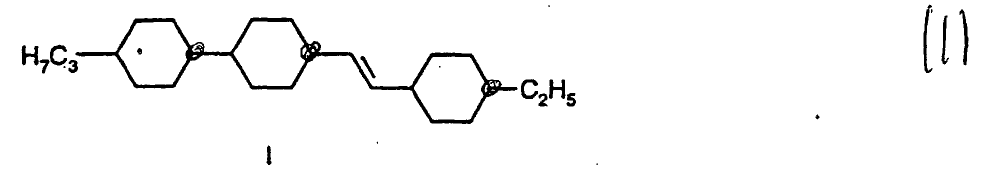

In einer Stickstoffatmosphäre werden 0,4 mol trans-4-n-Ethylcyclohexylmethyttriphenylphosphoniumiodid und 0,4 mol 4-[trans-4-(trans-4-propylcyclohexyl)cyclohexancarbaldehyd in 1140 ml THF gelöst und bei Raumtemperatur unter Rühren mit 0,4 mol Kaliumtert.butylat versetzt. Man rührt über Nacht bei Raumtemperatur, versetzt mit Wasser und verd. Salzsäure und arbeitet wie üblich auf.

### Schritt 1.2

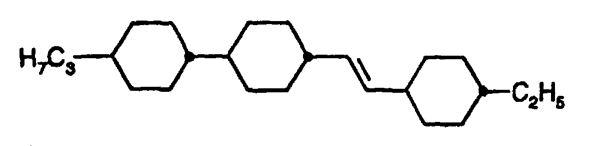

0,275 mol I werden in einer Stickstoffatmosphäre in 400 ml Toluol gelöst und mit 0,072 mol Benzolsulfinsäure-Natriumsalz und 110 ml 1 N Salzsäure am Rückfluß gekocht. Anschließend wird wie üblich aufgearbeitet.

Analog werden die folgenden Verbindungen der Formel 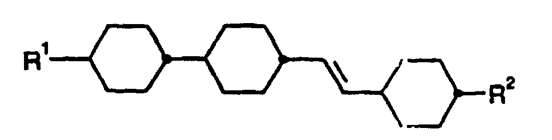 hergestellt:

| R¹ | R² |
|---|---|
| CH₃ | CH₃ |
| CH₃ | C₂H₅ |
| CH₃ | n-C₃H₇ |
| CH₃ | n-C₅H₁₁ |
| CH₃ | n-C₆H₁₃ |
| | |
| C₂H₅ | CH₃ K? - 23 S_{M} 51 S_{B} 168 N 170, 1l; Δε = 0,6, Δn = +0,068 |
| C₂H₅ | C₂H₅ K-11S_{B} 183 N 188,8 I; Δε = 0.41; Δn = +0,078 |
| C₂H₅ | n-C₃H₇ K? -15 S_{B} 184 N 207,3 I; Δε = 0,85; Δn = +0,080 |
| C₂H₅ | n-C₅H₁₁ |
| C₂H₅ | n-C₆H₁₃ |
| n-C₃H₇ | CH₃ K? - 19 S_{M} 16 S_{B} 188 N 197,4 I; Δε = -1,06; Δn = 0,085 |
| n-C₃H₇ | C₂H₅ K - 28 S_{B} 197 N 207,9 I; Δn =+0,078; Δε = 0,88 |
| n-C₃H₇ | n-C₃H₇ K 39 S_{B} 207 N 231,8 I; Δn = +0,087; Δε = 0,99 |
| n-C₃H₇ | n-C₅H₁₁ |
| n-C₃H₇ | n-C₆H₁₃ |
| | |
| n-C₅H₁₁ | CH₃ |
| n-C₅H₁₁ | C₂H₅ |
| n-C₅H₁₁ | n-C₃H₇ |
| n-C₅H₁₁ | n-C₅H₁₁ |
| n-C₅H₁₁ | n-C₆H₁₃ |

### Beispiel 2

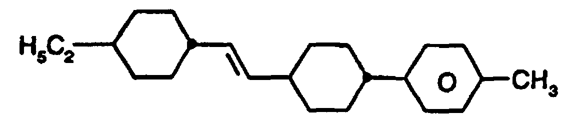

### Schritt 2.1

In einer Stickstoffatmosphäre werden 162 mmol trans-4-n-Ethylcyclohexylethyltriphenylphosphoniumbromid und 162 mmol trans-4-(4-Methylphenyl)-cyclohexancarbaldehyd in 500 ml THF gelöst und bei Raumtemperatur unter Rühren mit 162 mmol Kaliumtert.butylat versetzt. Man rührt 2 h, versetzt mit Wasser und arbeitet wie üblich auf.

### Schritt 2.2

Die Isomerisierung findet analog Schritt 1.2 statt.
K 43 S_{B} 89 N 156, 6 I; Δn = +0,103; Δε = 0,58

Analog werden die folgenden Verbindungen der
Formel 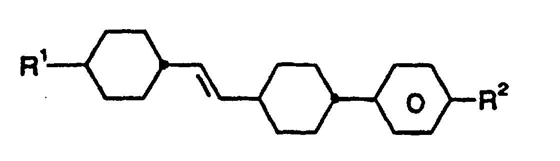 hergestellt:

| R¹ | R² |
|---|---|
| CH₃ | CH₃ K 77 N 141,9 I; Δn = +0,114; Δε = 0,76 |
| CH₃ | C₂H₅ |
| CH₃ | n-C₃H₇ |
| CH₃ | n-C₅H₁₁ |
| CH₃ | n-C₆H₁₃ |
| | |
| C₂H₅ | CH₃ K 43 S_{B} 89 N 157 I |
| C₂H₅ | C₂H₅ |
| C₂H₅ | n-C₃H₇ |
| C₂H₅ | n-C₅H₁₁ |
| C₂H₅ | n-C₆H₁₃ |
| | |
| n-C₃H₇ | CH₃ K 39 S_{B} 90 N 184,2 I; Δn = +0,119; Δε = 1,58 |
| n-C₃H₇ | C₂H₅ |
| n-C₃H₇ | n-C₃H₇ |
| n-C₃H₇ | n-C₅H₁₁ |
| n-C₃H₇ | OCH₃ K 71 S_{B} 104 N 209,7 I; Δn = +0,129; Δε = 1,02 |
| n-C₅H₁₁ | CH₃ |
| n-C₅H₁₁ | C₂H₅ |
| n-C₅H₁₁ | n-C₃H₇ |
| n-C₅H₁₁ | n-C₅H₁₁ |
| n-C₅H₁₁ | n-C₆H₁₃ |
| | |
| n-C₆H₁₃ | CH₃ |
| n-C₆H₁₃ | C₂H₅ |
| n-C₆H₁₃ | n-C₃H₇ |
| n-C₆H₁₃ | n-C₅H₁₁ |
| n-C₆H₁₃ | n-C₆H₁₃ |

### Beispiel 3

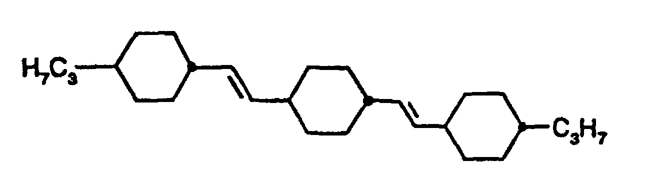

### Schritt 3.1

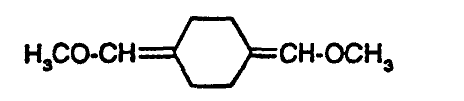

In einer Stickstoffatmosphäre werden 0,21 mol 1,4-Cyclohexandion und 0,53 mol Methoxytriphenylphosphoniumchlorid in 200 ml THF gelöst und bei Raumtemperatur unter Rühren mit 0,56 mol Kalium-tert.butylat versetzt. Man rührt 2 h, versetzt mit Wasser und verd. Salzsäure und arbeitet wie üblich auf.

### Schritt 3.2

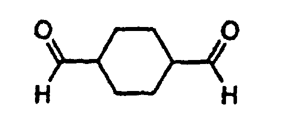

Das Produkt aus Schritt 3.1 wird in 915 ml THF gelöst, mit 230 ml 2 N HCl versetzt und 2 h in der Siedehitze gerührt. Anschließend wird wie üblich aufgearbeitet.

### Schritt 3.3

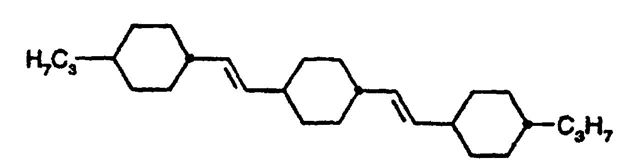

In einer Stickstoffatmosphäre werden 0,31 mol trans-4-n-Propylcyclohexylmethyltriphenylphosphoniumiodid in 520 ml THF suspendiert und portionsweise mit 0,31 mol Kalium-tert.butylat versetzt. Man rührt 1 h bei 0 °C, versetzt mit 0,13 mol Cyclohexan-1,4-dicarbaldehyd und rührt 1 h bei Raumtemperatur. Man versetzt mit Wasser und verd. Salzsäure und arbeitet wie üblich auf.

Die Isomerisierung erfolgt analog Schritt 1.2. K 98 S_{B} 190 N 235,7 I

Analog werden die folgenden Verbindungen der Formel 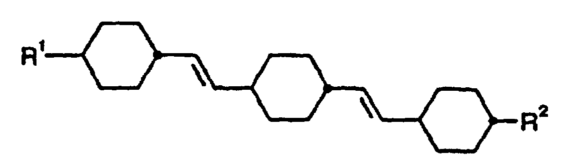 hergestellt:

| R¹ | R² |
|---|---|
| CH₃ | CH₃ |
| CH₃ | C₂H₅ |
| CH₃ | n-C₃H₇ |
| CH₃ | n-C₅H₁₁ |
| CH₃ | n-C₆H₁₃ |
| | |
| C₂H₅ | CH₃ |
| C₂H₅ | C₂H₅ |
| C₂H₅ | n-C₃H₇ |
| C₂H₅ | n-C₅H₁₁ |
| C₂H₅ | n-C₆H₁₃ |
| | |
| n-C₃H₇ | CH₃ |
| n-C₃H₇ | C₂H₅ |
| n-C₃H₇ | n-C₃H₇ |
| n-C₃H₇ | n-C₅H₁₁ |
| n-C₃H₇ | n-C₆H₁₃ |
| n-C₅H₁₁ | CH₃ |
| n-C₅H₁₁ | C₂H₅ |
| n-C₅H₁₁ | n-C₃H₇ |
| n-C₅H₁₁ | n-C₅H₁₁ |
| n-C₅H₁₁ | n-C₆H₁₃ |
| | |
| n-C₆H₁₃ | CH₃ |
| n-C₆H₁₃ | C₂H₅ |
| n-C₆H₁₃ | n-C₃H₇ |
| n-C₆H₁₃ | n-C₅H₁₁ |
| n-C₆H₁₃ | n-C₆H₁₃ |

### Beispiel 4

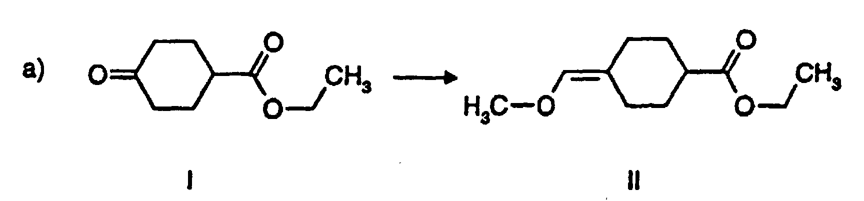

In einer Stickstoffatmosphäre werden 2,93 ml Methoxymethyltrimethyltriphenylphosphoniumchlorid und 2,93 mol 4-Cyclohexanoncarbonsäureester in 4 I THF gelöst und bei Raumtemperatur unter Rühren mit 2,93 mol Kaliumtert.butylat versetzt. Man rührt 2 h, versetzt mit Wasser und verd. Salzsäure und arbeitet wie üblich auf.

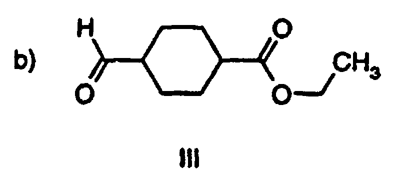

50,4 mmol II, 1,0 g 10%ige HCl, 20 ml THF und 50,4 mmol Acetaldehyd werden 0,75 h bei Raumtemperatur gerührt. Man versetzt mit Wasser und Methyl-tert.butylether und arbeitet wie üblich auf.

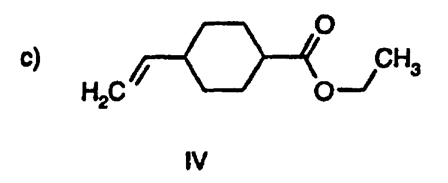

In einer Stickstoffatmosphäre werden 46,1 mmol Kalium-tert.butylat in 20 ml THF zu 46,1 mmol Methyltriphenylphosphoniumbromid gelöst in 50 ml THF bei 0 °C zugetropft. Nach 10 min Rühren werden 46,1 mmol III gelöst in 20 ml THF zu der Lösung zugetropft. Man läßt auf Raumtemperatur erwärmen, versetzt mit Wasser und verd. HCl und Methyl-tert.butylether und arbeitet wie üblich auf.

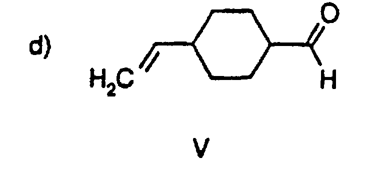

65,8 mmol Vitride werden bei 0°C in 35 ml Toluol vorgelegt und unter Rühren mit 72,3 mmol Morpholin gelöst in 20 ml Toluol versetzt. Die Lösung wird bei -40 °C zu 32,9 mmol IV gelöst in 30 ml Toluol zugetropft und bei -40 bis -50° 70 min gerührt. Man versetzt bei -40 °C mit Wasser und bei -10 °C mit 10%iger HCI. Anschließend wird wie üblich aufgearbeitet.

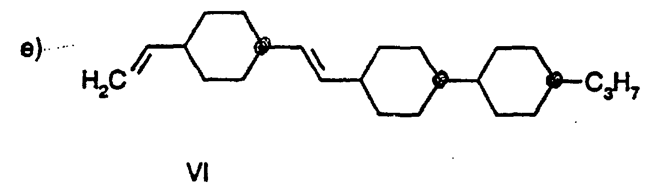

0,04 mol V, 0,04 mol 4-[trans-4-(trans-4-Propylcyclohexyl)cyclohexylmethyltriphenylphosphoniumiodid und 1140 ml THF werden vorgelegt und portionsweise mit 0,40 mol Kalium-tert.butylat versetzt, wobei die Reaktionstemperatur 35 °C nicht übersteigan sollte. Man rührt über Nacht bei Raumtemperatur, hydrolysiert, versetzt mit verd. HCI und arbeitet wie üblich auf. K -2 S_{B} 188 N 222,2 I; Δn = +0,888; Δε = 1,03
Analog werden die folgenden Verbindungen der Formel 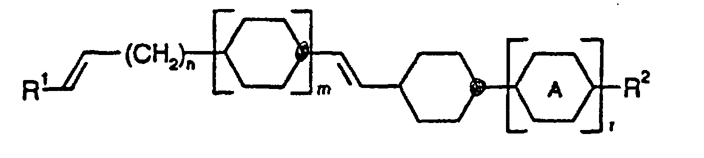 hergestellt:

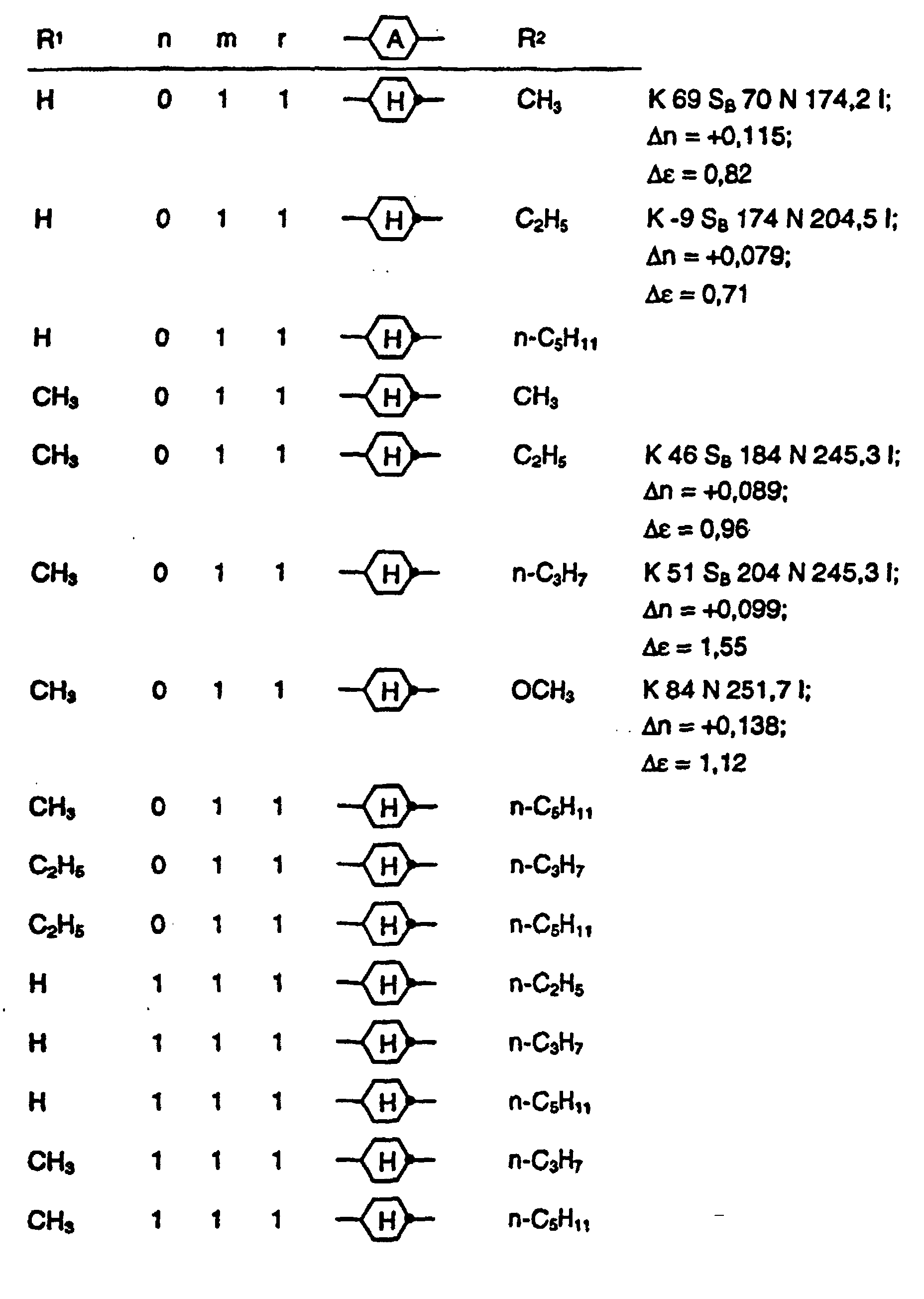

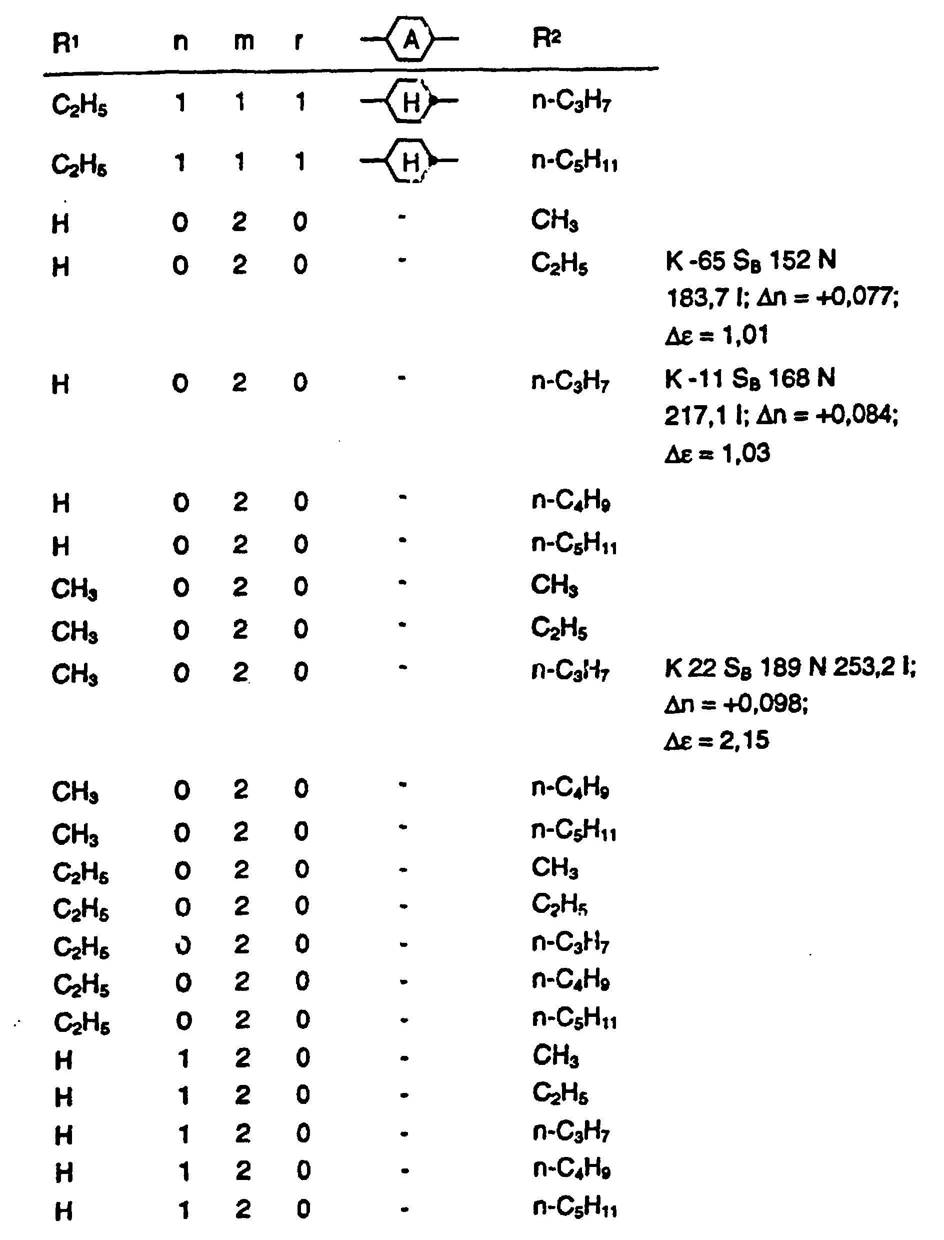

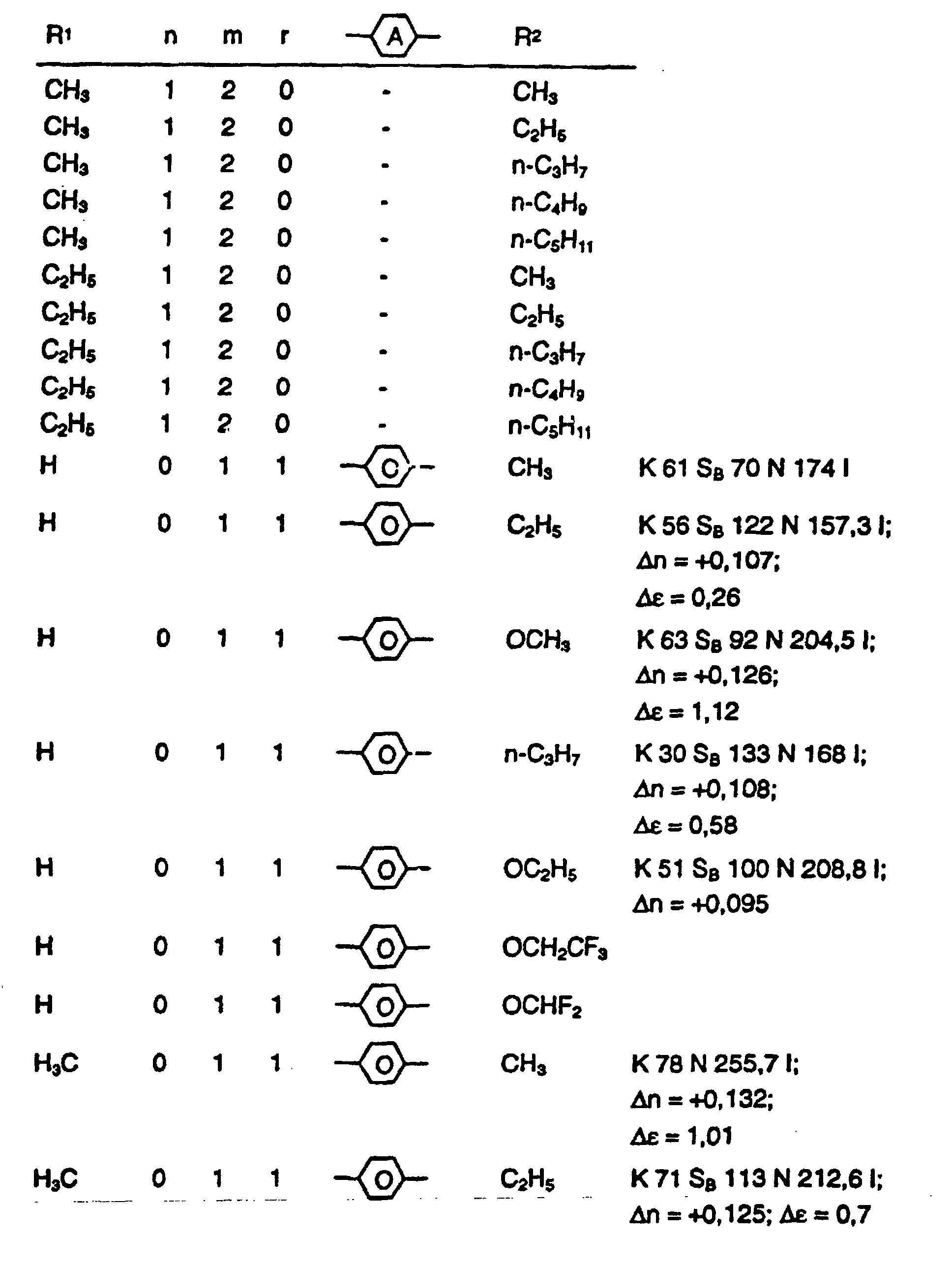

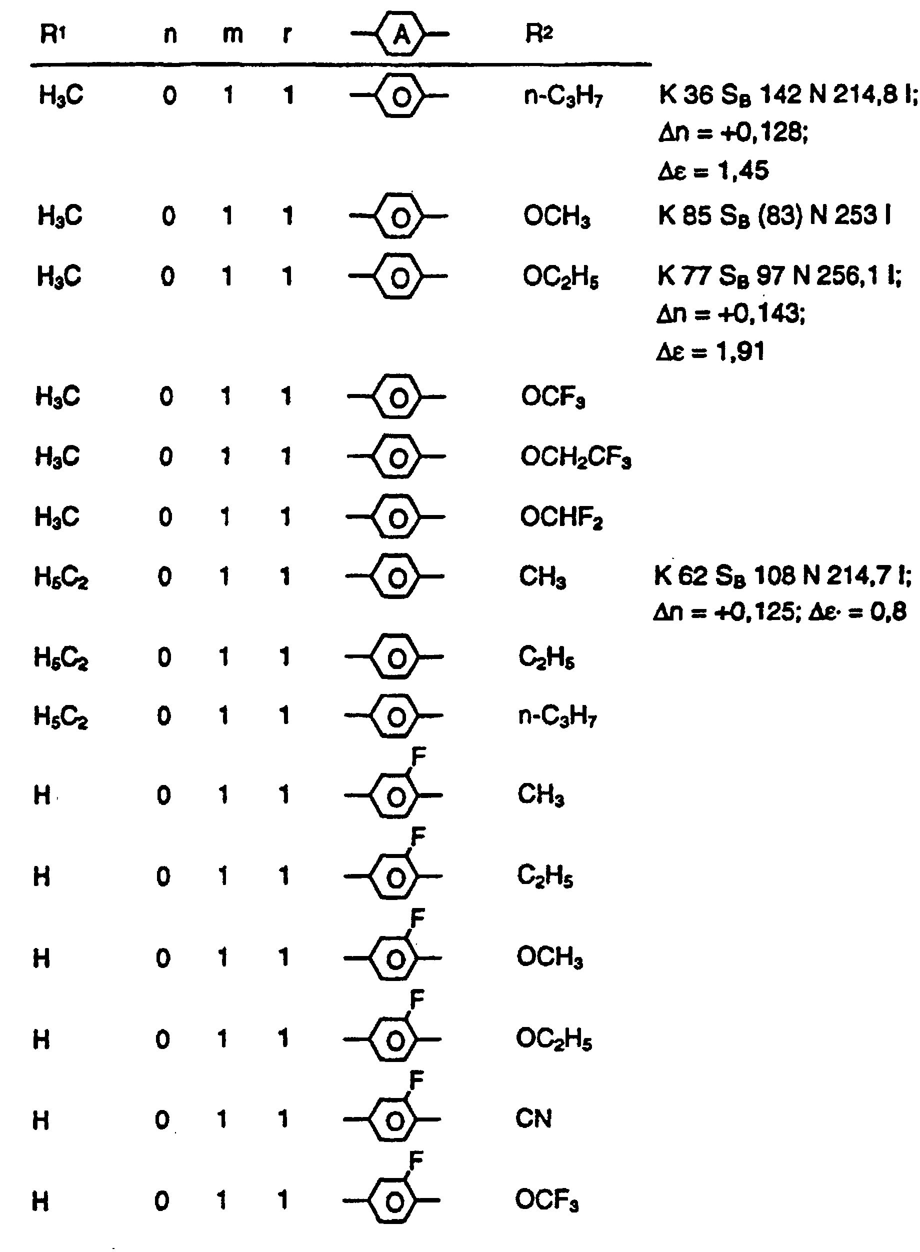

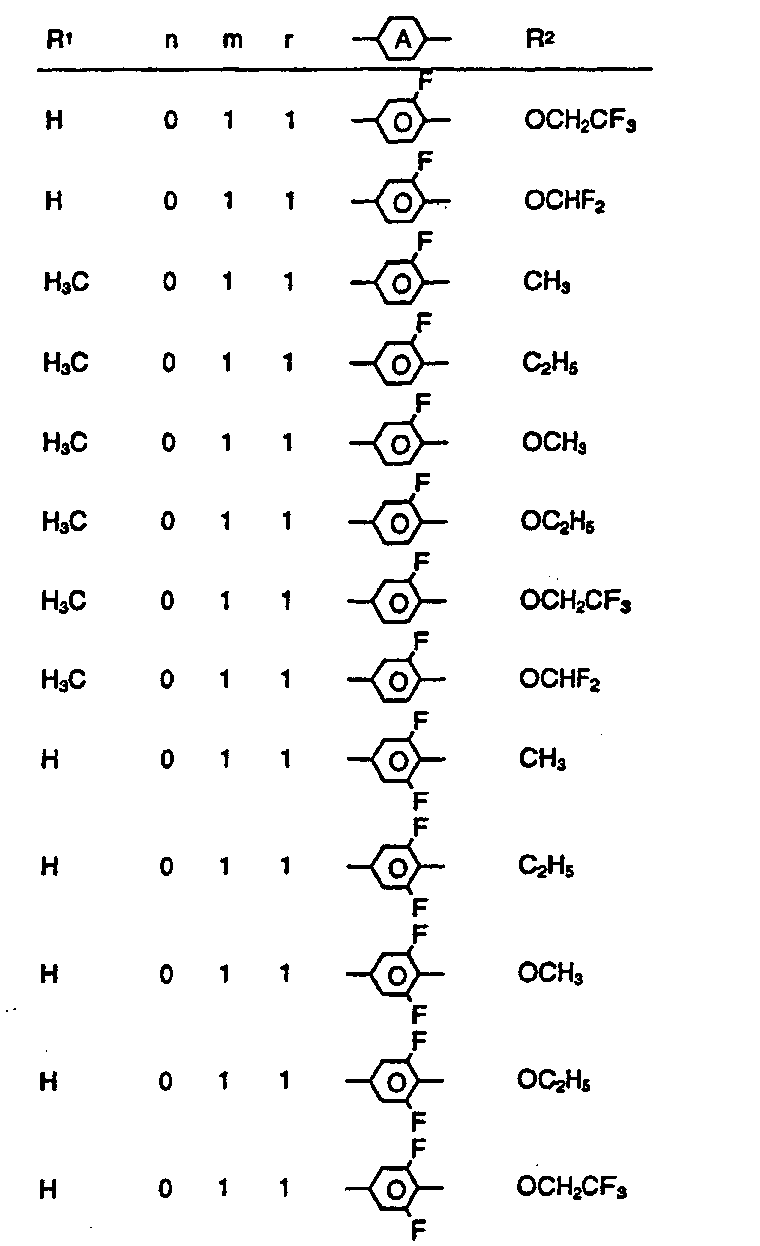

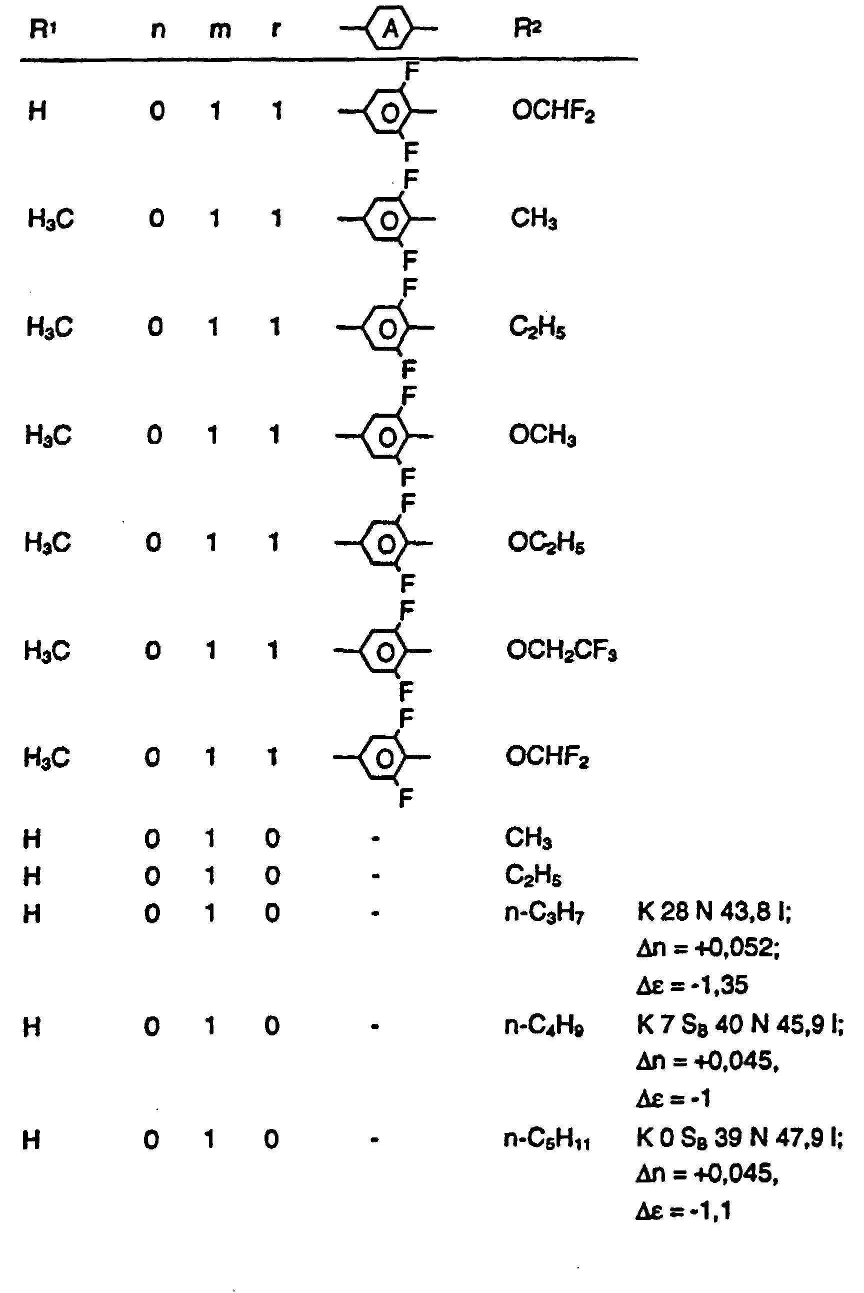

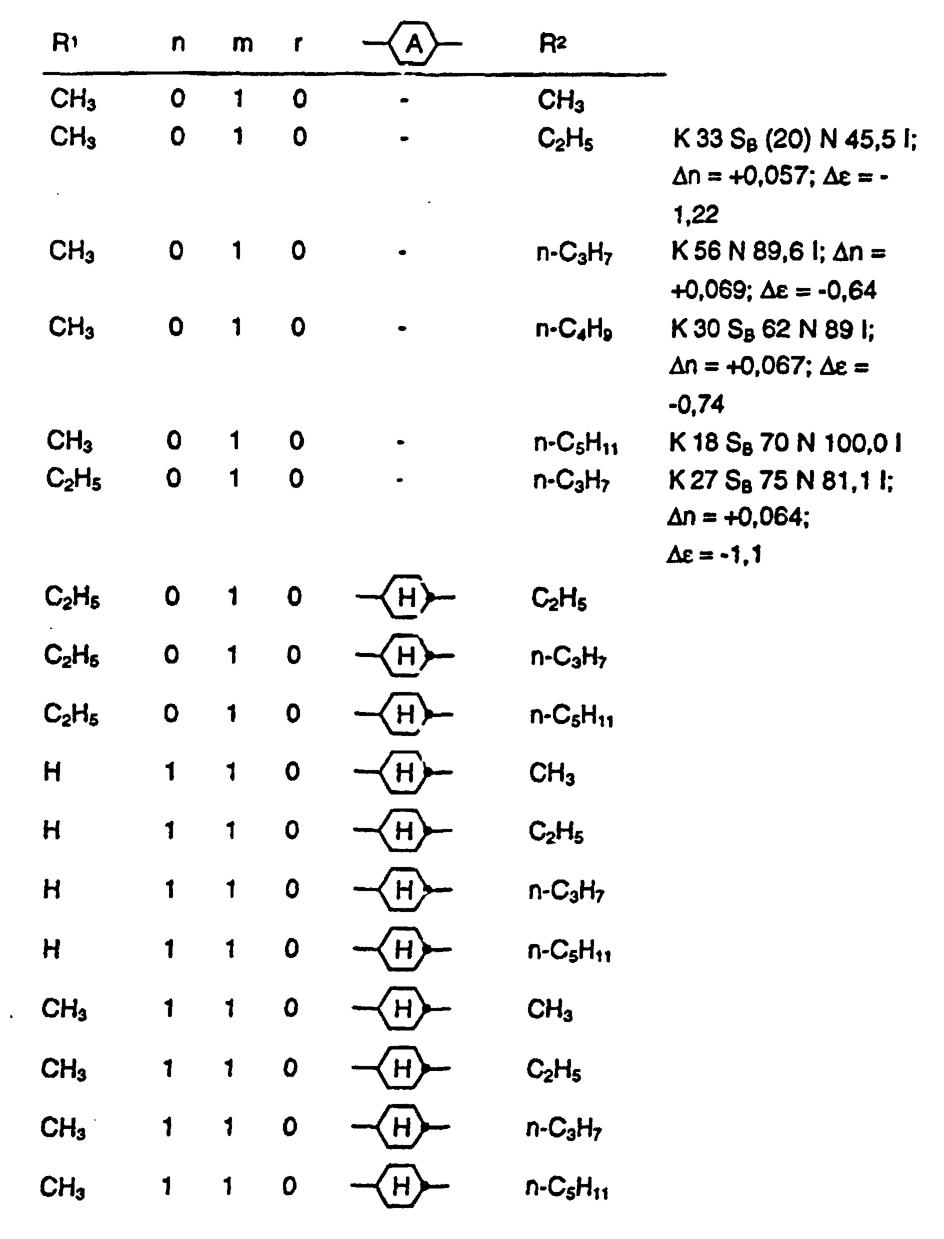

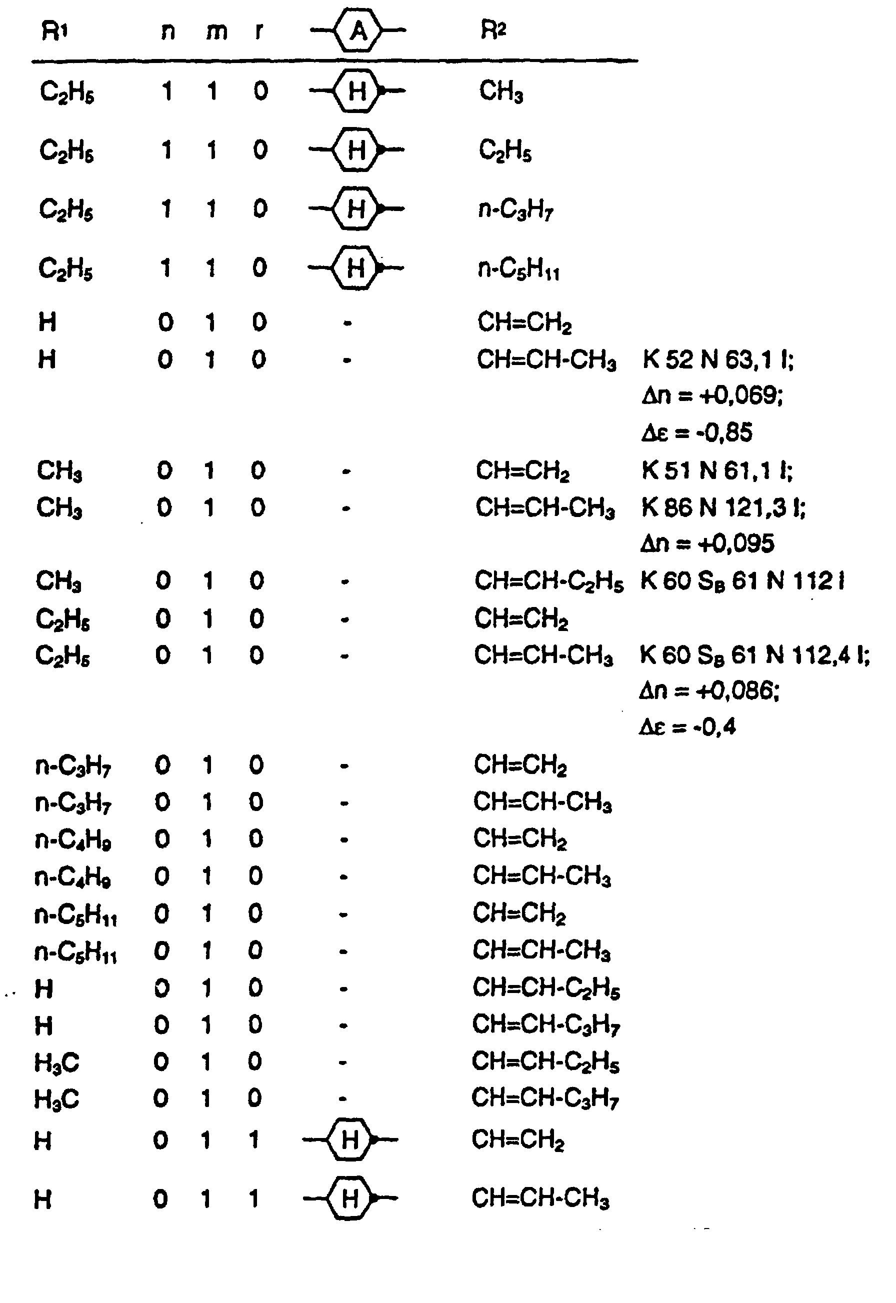

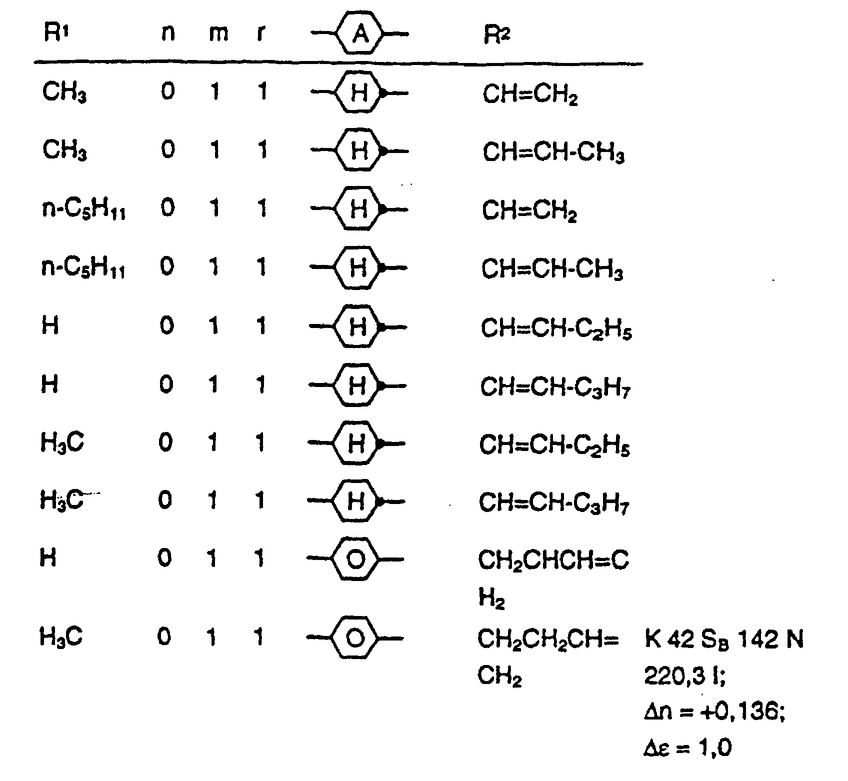

### Mischungsbeispiele

### Beispiel M1

| Komponente | | | | |
|---|---|---|---|---|
| A | PCH-3 | 21,0 % | Klärpunkt [°C]: | +86 |
| A | PCH-5 | 15,0 % | Δn [589 nm; 20 °C]: | +0,1428 |
| B | PCH-302 | 23,0 % | STN 240° | |
| A | K6 | 5,0 % | d.Δn [µm]: | 0,85 |
| B | BCH-32 | 7,0 % | V_{(10.0.20)} [V]: | 2,38 |
| B | ECCP-31 | 5,0 % | V₉₀/V₁₀: | 1.0305 |
| B | ECCP-32 | 5,0 % | tₐᵥₑ [ms]: | 263 |
| B | CPTP-301 | 5,0 % | | |
| B | CPTP-302 | 4,0 % | | |
| B | CPTP-303 | 4,0 % | | |
| B | CVCP-3-1 | 6,0 % | | |

### Beispiel M2

| Komponente | | | | |
|---|---|---|---|---|
| A | PCH-3 | 21,0 % | Klärpunkt [°C]: | +86 |
| A | PCH-5 | 15,0% | Δn [589 nm, 20 °C]: | +0,1416 |
| B | PCH-302 | 24,0 % | STN 240° | |
| A | K6 | 6,0% | d.Δn[µm]: | 0,85 |
| B | BCH-32 | 8,0 % | V_{(10.0.20)} [V]: | 2,34 |
| B | ECCP-31 | 4,0 % | V₉₀/V₁₀: | 1,036 |
| B | ECCP-32 | 4,0 % | tₐᵥₑ [ms]: | 248 |
| B | CPTP-301 | 4,0 % | | |
| B | CPTP-302 | 4,0 % | | |
| B | CPTP-303 | 4,0 % | | |
| B | CVCC-3-3 | 6,0 % | | |

### Beispiel M3

Eine STN-Anzeige mit folgenden Parametem:

| | |
|---|---|
| Twist | 240° |
| MuX | 1:240 |
| d Δn [µm] | 0,85 |

enthält eine nematische Mischung mit folgenden Eigenschaften:

| | |
|---|---|
| Klärpunkt: | 90,1 °C |
| Δn [589 nm; 20 °C]: | 0,1421 |
| d/p | 0,53 |

bestehend aus:

| Komponente | | |
|---|---|---|
| A | ME2N.F | 2,0 % |
| A | ME3N.F | 3,0 % |
| A | PCH-3 | 20,0 % |
| A | PCH-5 | 6,0 % |
| B | PTP-201 | 5,0 % |
| B | BCH-32 | 8,0 % |
| B | BCH-52 | 8,0 % |
| B | CPTP-301 | 5,0 % |
| B | CPTP-302 | 5,0 % |
| B | CPTP-303 | 4,0 % |
| B | PCH-302 | 6,0 % |
| B | CCH-34 | 5,0 % |
| B | CCH-35 | 5,0 % |
| B | CVC-V1-2 | 6,0 % |
| B | CVC-V1-3 | 6,0% |
| B | CVC-V1-4 | 6,0 % |

weist folgendes Schattverhalten auf:

| | |
|---|---|
| V₁₀ | 2,39 V |
| V₉₀/V₁₀ | 1,051 |
| tₐᵥₑ | 217ms |

### Beispiel M4

Eine STN-Anzeige, wie in Beispiel M3 beschrieben, enthält eine nematische Mischung mit folgenden Eigenschaften:

| | |
|---|---|
| Klärpunkt: | 93,1 °C |
| Δn [589 nm; 20 °C]: | 0,1408 |
| d/p | 0,53 |

bestehend aus:

| Komponente | | |
|---|---|---|
| A | ME-2N.F | 2,0% |
| A | K6 | 6,0% |
| A | K9 | 6,0% |
| A | PCH-3 | 18,0% |
| B | BCH-32 | 7,0% |
| B | BCH-52 | 7,0% |
| B | CPTP-301 | 5,0% |
| B | CPTP-302 | 4,0% |
| B | CPTP-303 | 4,0 % |
| B | CCH-34 | 5,0% |
| B | CCH-35 | 4,0% |
| B | CVC-V1-2 | 10,0% |
| B | CVC-V1-3 | 10,0% |
| B | CVC-V1-4 | 10,0 % |
| B | CBC-33 | 2,0 % |

weist folgendes Schaltverhalten auf:

| | |
|---|---|
| V₁₀ | 2,496 V |
| V₉₀/V₁₀ | 1,048 |
| tₐᵥₑ | 213 ms |

### Beispiel M5

Eine STN-Anzeige, wie in Beispiel M3 beschrieben, enthält eine nematische Mischung mit folgenden Eigenschaften:
- Klärpunkt:: 87,6 °C
- Δn [589 nm, 20 °C]:: 0,1397
- d/p:: 0,52
bestehend aus:

| Komponente | | |
|---|---|---|
| A | ME-2N.F | 2,0% |
| A | K6 | 4,0% |
| A | K9 | 5,0% |
| A | PCH-3 | 22,0 % |
| B | PTP-201 | 2,0% |
| B | BCH-32 | 5,0 % |
| B | BCH-52 | 5,0 % |
| B | CPTP-301 | 5,0% |
| B | CPTP-302 | 5,0% |
| B | CPTP-303 | 5,0% |
| B | CCH-34 | 5,0 % |
| B | CCH-35 | 5,0% |
| B | CVC-V1-2 | 10,0% |
| B | CVC-V1-3 | 10,0% |
| B | CVC-V1-4 | 10,0% |

weist folgendes Schaltverhalten auf:
- V₁₀: 2,44 V
- V₉₀/V₁₀: 1,039
- tₐᵥₑ: 223 ms

### Beispiel M6

Eine STN-Anzeige, wie in Beispiel M3 beschrieben, enthält eine nematische Mischung mit folgenden Eigenschaften:
- Klärpunkt:: 86,4 °C
- Δn [589 nm, 20 °C]:: 0,1395
- d/p:: 0,52
bestehend aus:

| Komponente | | |
|---|---|---|
| A | ME-2N.F | 2,0 % |
| A | K6 | 4,0 % |
| A | K9 | 5,0 % |
| A | PCH-3 | 22,0 % |
| B | PTP-201 | 2,0 % |
| B | BCH-32 | 5,0% |
| B | BCH-52 | 5,0% |
| B | CPTP-301 | 5,0 % |
| B | CPTP-302 | 5,0 % |
| B | CPTP-303 | 5,0% |
| B | CCH-34 | 5,0 % |
| B | CCH-35 | 5,0% |
| B | CVC-V1-2 | 10,0% |
| B | CVC-V1-3 | 10,0 % |
| B | CVC-V-3 | 10,0 % |

weist folgendes Schaltverhalten auf:
- V₁₀: 2,432 V
- V₉₀/V₁₀: 1,042
- tₐᵥₑ: 215 ms

### Beispiel M7

Eine STN-Anzeige, wie in Beispiel M3 beschrieben, enthält eine nematische Mischung mit folgenden Eigenschaften:
- Klärpunkt:: 85,9 °C
- Δn [589 nm, 20 °C]:: 0,1395
- d/p:: 0,52
bestehend aus:

| Komponente | | |
|---|---|---|
| A | ME-2N.F | 2,0% |
| A | K6 | 4,0% |
| A | K9 | 5,0% |
| A | PCH-3 | 22,0 % |
| B | PTP-201 | 2,0% |
| B | BCH-32 | 5,0 % |
| B | BCH-52 | 5,0% |
| B | CPTP-301 | 5,0 % |
| B | CPTP-302 | 5,0 % |
| B | CPTP-303 | 5,0% |
| B | CCH-34 | 5,0% |
| B | CCH-35 | 5,0% |
| B | CVC-V1-2 | 10,0% |
| B | CVC-V1-3 | 10,0% |
| B | CVC-1V-V | 10,0% |

weist folgendes Schattverhalten auf:
- V₁₀: 2,4 V
- V₉₀/V₁₀: 1,039
- tₐᵥₑ: 209 ms

### Beispiele M8-M13

Die Zusammensetzungen der flüssigkristallinen Medien und die Eigenschaften der mit deren Hilfe hergestellten STN-Anzeigen (Twist = 240°) können den folgenden Tabellen entnommen werden:

**Tabelle I**

| Beispiel/ Komponente/Verb. | | M8 (%) | M9 (%) | M10 (%) | M11 (%) | M12 (%) | M13 (%) |
|---|---|---|---|---|---|---|---|
| A | K6 | 10 | 10 | 10 | 9 | 10,00 | 10 |
| A | K9 | 10 | 10 | 10 | 9 | 10,00 | 10 |
| A | K12 | 4 | 4 | 5 | - | 4,63 | - |
| A | ME2N.F | 4 | 2 | 2 | 2 | 2,00 | - |
| A | ME3N.F | - | - | - | 3 | - | 4 |
| A | PCH-3 | 6 | 8 | 6 | 9 | 6,74 | 8 |
| B | PTP-201 | 7 | 6 | 4 | 7 | 4,74 | 4 |
| B | BCH-32 | - | 4 | 11 | 6 | 8,41 | 5 |
| B | CPTP-302 | - | - | - | 2 | - | - |
| B | CPTP-303 | - | - | - | - | - | 4 |
| B | CCH-34 | 9 | 11 | 14 | 14 | 12,89 | 14 |
| B | CCH-35 | - | - | 4 | 4 | 2,52 | - |
| B | CVC-V1-2 | - | - | - | - | - | 6 |
| B | CVC-V1-3 | 20 | 15 | 15 | 15 | 15,00 | 7 |
| B | CVC-V1-4 | - | - | - | - | - | 6 |
| B | CVCP-1V-1 | 13 | 13 | 8 | - | 9,85 | 7 |
| B | CVCP-1V-2 | - | - | - | - | - | 7 |
| B | CVCP-1V-3 | - | - | - | - | - | 8 |
| B | CVCP-V-1 | 17 | 17 | 11 | 8 | 13,22 | - |
| B | CVCP-V-1 | - | - | - | 12 | - | - |

**Tabelle II**

| Beispiel / Eigenschaften | M8 | M9 | M10 | M11 | M12 | M13 |
|---|---|---|---|---|---|---|
| Δn (589 nm) | 0,1321 | 0,1436 | 0,1387 | 0,1386 | 0,1405 | 0,1400 |
| Klärpunkt (°C) | 82,2 | 86,4 | 81.7 | 82,4 | 83,5 | 87,4 |
| Pitch [µm] | -11,2 | -11,3 | -11,3 | -11,4 | -11,6 | -11,8 |
| d [µm] | 5,89 | 5,92 | 6,13 | 6,14 | 6,07 | 6,07 |
| V₁₀ [V] | 2,20 | 2,26 | 2,21 | 2,18 | 2,28 | 2,29 |
| V₀/V₁₀ | 1,020 | 1,024 | 1.050 | 1,046 | 1,041 | 1,038 |
| tₐᵥₑ [ms] | 218 | 215 | 183 | 210 | 192 | 215 |

### Beispiele M14-M17

Die Zusammensetzungen der flüssigkristallinen Medien und die Eigenschaften der mit deren Hilfe hergestellten STN-Anzeigen (Twist = 240°) können den nachfolgenden Tabellen entnommen werden.

**Tabelle III**

| Beispiel / Komponente/verb. | | M14 (%) | M15 (%) | M16 (%) | M17 (%) |
|---|---|---|---|---|---|
| A | PCH-2 | 18,0 | 8,0 | 8,0 | 8,0 |
| A | PCH-3 | 25,0 | 25,0 | 24,0 | 24,0 |
| A | ME-2N.F | 3,0 | 3,0 | 2,0 | 2,0 |
| A | ME-3N.F | - | 3,0 | 3,0 | 3,0 |
| A | ME-5N.F | - | 3,0 | 7,0 | 7,0 |
| B | CCH-34 | - | 6,0 | - | - |
| B | CVC-V1-2 | 6,0 | 6,0 | - | - |
| B | CVC-V1-3 | 7,0 | 10,0 | 12,0 | 12,0 |
| B | CVC-V1-4 | 6,0 | 9,0 | 12,0 | 12,0 |
| B | CVCP-1V-1 | - | 6,0 | 5,0 | 5,0 |
| B | CVCP-1V-2 | - | 5,0 | 6,0 | 6,0 |
| B | PTP-102 | 5,0 | 5.0 | 4,0 | 4,0 |
| B | PTP-201 | 3,0 | 6,0 | 4,0 | 4,0 |
| B | CPTP-301 | 3,0 | - | 4,0 | 3,0 |
| B | CPTP-302 | 4,0 | 5,0 | 3,0 | 3,0 |
| B | CPTP-303 | 3,0 | - | - | - |
| B | BCH-32 | 6,0 | 6,0 | 6,0 | 7,0 |
| D | CP-33F | 6,0 | - | - | - |
| D | CP-35F | 5,0 | - | - | - |

**Tabelle IV**

| Beispiel / Eigenschaften | M14 | M15 | M16 | M17 |
|---|---|---|---|---|
| Δn (589 nm) | 0,1394 | 0,1390 | 0,1413 | 0,1397 |
| Klärpunkt (°C) | 75 | 74 | 77 | 77 |
| Pitch [µm] | -11,3 | -11,4 | -11,4 | -11,3 |
| d [µm] | 6,11 | 6,12 | 6,02 | 6,08 |
| V₁₀ [V] | 1,86 | 1,87 | 1,78 | 1,78 |
| V₉₀/V₁₀ | 1,031 | 1,036 | 1,030 | 1,037 |
| tₐᵥₑ [ms] | 280 | 228 | 249 | 240 |

### Beispiele M18-M25

Die Zusammensetzungen der flüssigkristallinen Medien und die Eigenschaften der mit deren Hilfe hergestellten STN-Anzeigen (Twist = 240°) können den nachfolgenden Tabellen entnommen werden.

**Tabelle V**

| Beispiel/ Komponente/verb | | M18 (%) | M19 (%) | M20 (%) | M21 (%) | M22 (%) | M23 (%) | M24 (%) | M25 (%) |
|---|---|---|---|---|---|---|---|---|---|
| A | ME2N.F | 2 | 2 | 2 | 2 | 2 | 2 | 2,5 | 2,5 |
| A | ME3N.F | 2 | 2 | 2 | - | | 3 | 3,5 | 3,5 |
| A | ME4N.F | - | - | - | - | - | - | 6,0 | 6,0 |
| A | K6 | 6 | 6 | 6 | 6 | 6 | 6 | - | - |
| A | K9 | 4 | 4 | 4 | 5 | 6 | 5 | 10,0 | 10,0 |
| A | PCH-3 | 20 | 20 | 20 | 18 | 16 | 17 | 10,0 | 10,0 |
| A | ECCP-3F | - | - | - | - | - | - | 13.0 | - |
| A | CCP-2OCF₃ | - | - | - | - | - | - | 6,0 | 6,0 |
| A | CCP-3OCF₃ | - | - | - | - | 6 | 6 | 6,0 | 6,0 |
| A | OCP-4OCF₃ | - | - | - | - | - | - | 6,0 | 6,0 |
| A | CCP-5OCF₃ | - | - | - | - | - | - | 4,0 | 6,0 |
| A | PTP-20F | - | - | - | - | - | - | - | |
| B | PTP-102 | 4 | 3 | 4 | 4 | 3 | 3 | 4,0 | - |
| B | PTP-201 | 3 | 3 | 4 | 4 | 4 | 4 | 4,0 | - |
| B | BCH-32 | 9 | 8 | 10 | 7 | 11 | 9 | - | - |
| B | BCH-52 | - | - | - | 7 | - | - | - | - |
| B | CPTP-301 | 4 | 4 | 4 | 3 | 2 | 4 | - | 5,0 |
| B | CPTP-302 | - | - | - | - | - | - | - | 5,0 |
| B | PCH-301 | - | 8 | - | - | - | 6 | - | - |
| B | PCH-302 | - | - | - | - | 9 | - | - | 15,0 |
| B | CCH-34 | 7 | 6 | 7 | 8 | 7 | 8 | - | - |
| B | CCH-35 | 6 | - | 7 | 7 | - | - | - | - |
| B | CCH-301 | - | - | - | - | - | - | - | 2,0 |
| B | CBC-33 | 3 | 4 | - | - | - | - | - | - |
| B | CCH-3-2V | - | - | 15 | - | - | 15 | - | - |
| B | CVCP-1V-1 | 6 | 6 | 6 | 4 | 5 | 6 | 12,0 | 8,0 |
| B | CVCP-V-1 | 9 | 9 | 9 | 7 | 7 | 8 | 12,0 | 8,0 |
| B | CVCP-1V-1 | - | - | - | - | - | 4 | - | - |
| B | CVCP-1V-2 | - | - | - | - | - | - | - | 5,0 |
| B | CVCP-V-1 | - | - | - | 5 | 6 | - | - | - |
| B | CVC-V1-3 | 5 | 5 | - | - | - | - | 11,0 | 6,0 |
| B | CVC-V-3 | 10 | 10 | - | 8 | 6 | - | - | - |
| B | CVC-V-4 | - | - | - | 5 | 4 | - | - | - |

**Tabelle VI**

| Beispiel/ Eigenschaften | M18 | M19 | M20 | M21 | M22 | M23 | M24 | M25 |
|---|---|---|---|---|---|---|---|---|
| Δn | 0,1409 | 0,1403 | 0,1390 | 0,1390 | 0,1398 | 0,1408 | 0,1262 | 0,1271 |
| Klärpunkt [°C] | 87,8 | 83,3 | 83,2 | 84,8 | 83,3 | 82,6 | 111 | 108 |
| S→N | <-15 | <-15 | <-15 | <-15 | <-15 | <-15 | <0 | <-30 |
| Pitch [µm] | -10,91 | -10,99 | -11,55 | -11,55 | -11,20 | -11,40 | - | - |
| HTP [1/µm] | -10,32 | -10,72 | -9,70 | -10,05 | -10,44 | -10,36 | - | - |
| d [µm] | 6,03 | 6,06 | 6,12 | 6,12 | 6,07 | 6,04 | - | - |
| d*dn | 0,85 | 0,85 | 0,85 | 0,85 | 0,85 | 0,85 | 0,85 | 0,85 |
| d/p | 0,55 | 0,55 | 0,53 | 0,53 | 0,54 | 0,53 | - | - |
| V₁₀ | 2,283 | 2,183 | 2,256 | 2,285 | 2,272 | 2,131 | 2,26 | 2,21 |
| V₉₀ | 2,382 | 2,248 | 2,374 | 2,392 | 2,373 | 2,222 | - | - |
| V₉₀/V₁₀ | 1,043 | 1,030 | 1,052 | 1,047 | 1,044 | 1,043 | 1,034 | 1,036 |
| t-ave [ms] | 193 | 215 | 179 | 204 | 203 | 185 | 258 | 275 |

### Beispiel M26

Man stellt ein flüssigkristallines Medium her bestehend aus:

| Komponente | | | | |
|---|---|---|---|---|
| A | ME2N.F | 2,0 % | Δn [589 nm, 20 °C] | 0,1390 |
| A | K6 | 6,0 % | Klärpunkt | 84,6 °C |
| A | K9 | 5,0 % | V₁₀ | 2,265 V |
| A | PCH-3 | 18,0% | V₉₀/V₁₀ | 1,045 |
| B | PTP-102 | 4,0% | tₐᵥₑ [ms] | 200 |
| B | PTP-201 | 4,0 % | | |
| B | CH-32 | 7,0 % | | |
| B | BCH-52 | 7,0% | | |
| B | CPTP-301 | 3,0% | | |
| B | CCH-34 | 8,0% | | |
| B | CCH-35 | 7,0 % | | |
| B | CVCP-1V-1 | 4,0% | | |
| B | CVCP-V-1 | 7,0% | | |
| B | CVCP-V-1 | 5,0% | | |
| B | CVC-V-3 | 8,0% | | |
| B | CVC-V-4 | 5,0% | | |

### Vergleichsbeispiel 1

Eine STN-Anzeige wie in Beispiel M3 beschrieben:

| Komponente | | | | |
|---|---|---|---|---|
| A | PCH-3 | 21,0% | Klärpunkt [°C] | +85 |
| A | PCH-5 | 15,0% | Δn [589 nm] | +0,1413 |
| A | PCH-302 | 23,0 % | STN 240° | |
| A | K6 | 4,0 % | d.Δn [µm] | 0,85 |
| B | BCH-32 | 7,0 % | d/p | 0,54 |
| A | CCP-2OCF₃ | 4,0 % | V_{(10.0.20)} [V] | 2,36 |
| A | CCP-3OCF₃ | 2,0 % | V₉₀/V₁₀ | 1,042 |
| B | ECCP-31 | 5,0% | tₐᵥₑ [ms] | 245 |
| B | ECCP-32 | 5,0 % | | |
| B | CPTP-301 | 5,0 % | | |
| B | CPTP-302 | 5,0 % | | |
| B | CPTP-303 | 4,0 % | | |

### Verglelchsbelspel 2

Eine STN-Anzeige wie in Beispiel M20 beschrieben:

| Komponente | | | | |
|---|---|---|---|---|
| A | ME2N.F | 4,0 % | Klärpunkt [°C] | +87,2 |
| A | ME3N.F | 3,0 % | Δn [589 nm] | 0,1365 |
| A | PTP-20F | 4,0 % | STN 240° | |
| B | BCH-32 | 6,0 % | d.Δn[µm] | 0,85 |
| B | CCH-34 | 3,0 % | V₁₀[V] | 2,26 |
| A | CCH-3-2V | 29,0 % | V₉₀/V₁₀ | 1,038 |
| B | PCH-1V-N | 25,0 % | tₐᵥₑ [ms] | 208 ns |
| B | PCH-3V-N | 5,0 % | | |
| A | BCH-3V-2 | 21,0 % | | |

### Beispiel M27

| Komponente | | | | |
|---|---|---|---|---|
| A | PCH-2 | 10,0 % | Klärpunkt [°C]: | 89 |
| A | PCH-3 | 24,0 % | Δn [589 nm, 20°C]: | +0,1395 |
| A | ME2N.F | 2,0 % | Δε [1 kHz, 20°C]: | 7,5 |
| B | CVC-3V | 5,0 % | d • Δn [µm]: | 0,85 |
| B | CVC-3-V1 | 4,0% | d/p: | 0,53 |
| B | CVCP-V-1 | 6,0% | V_{(10,0,20)} [V]: | 2,20 |
| B | CVCP-1V-1 | 6,0% | V₉₀/V₁₀ [V]: | 3,4 % |
| B | CVCP-V-1 | 7,0 % | | |
| B | PTP-102 | 4,0 % | | |
| B | PTP-201 | 5,0 % | | |
| B | CPTP-301 | 5,0 % | | |
| B | BCH-32 | 8,0 % | | |
| B | BCH-52 | 5,0 % | | |
| B | CCH-34 | 9,0 % | | |

## Patentansprüche

1. Supertwist-Flüssigkristall-Display mit
- zwei planparallelen Trägerpfatten, die mit einer Umrandung eine Zelle bilden,
- einer in der Zelle befindlichen nematischen Flüssigkristallmischung mit positiver dielektrischer Anisotropie,
- Elektrodenschichten mit darüberliegenden Orientierungsschichten auf den Innenseiten der Trägerplatten,
- einem Anstellwinkel zwischen der Längsachse der Moleküle an der Oberfläche der Trägerplatten und den Trägerplatten von 1 Grad bis 30 Grad, und
- einem Verdrillungswinkel der Flüssigkristallmischung in der Zelle von Orientierungsschicht zu Orientierungsschicht dem Betrag nach zwischen 100 und 600°,
- einer nematischen Flüssigkristallmischung mit einem Δε ≥ 1 bestehend aus
a) 20 - 90 Gew.% einer flüssigkristallinen Komponente A, bestehend aus einer oder mehreren Verbindungen mit einer dielektrischen Anisotropie von über +1,5;
b) 10 - 65 Gew.% einer flüssigkristallinen Komponente B, bestehend aus einer oder mehreren Verbindungen mit einer dielektrischen Anisotropie zwischen -1,5 und +1,5;
c) 0 - 20 Gew.% einer flüssigkristallinen Komponente D, bestehend aus einer oder mehreren Verbindungen mit einer dielektrischen Anisotropie von unter -1,5,
d) einer optisch aktiven Komponente C in einer Menge, daß das Verhältnis zwischen Schichtdicke (Abstand der planparallelen Trägerplatten) und natürlicher Ganghöhe der chiralen nematischen Flüssigkristallmischung 0,2 bis 1,3 beträgt,
**dadurch gekennzeichnet, daß** Komponente B mindestens eine Verbindung der Formel I 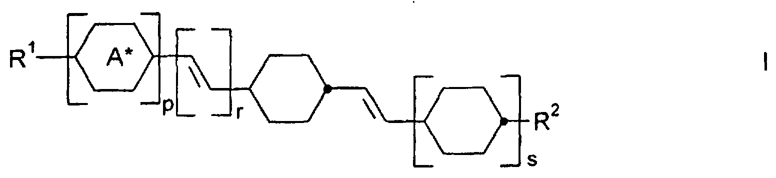 enthält,
worin 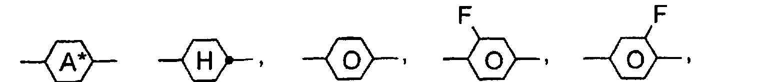 oder 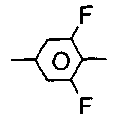
R¹ F, Cl, einen unsubstituierten oder einfach durch CN oder CF₂ oder mindestens einfach durch Halogen substituierten Alkyl-, Alkoxy oder Alkenylrest mit 1 bis 10 C-Atomen, wobei in diesem Rest auch ein oder mehrere CH₂-Gruppen durch -O- ersetzt sein können,
R² (CH₂)ₙ-CH=CH-R^{a} oder (CH₂)ₙ-CH=CH₂ und im Fall r = 1 auch R^{a}
R^{a} geradkettiges Alkyl mit 1-6 C-Atomen,
n 0-6,
p 0 oder 1,
r 0, 1 oder 2, und
s 1 oder 2
bedeuten.

2. Display nach Anspruch 1, **dadurch gekennzeichnet, daß** Komponente A Verbindungen der Formeln II und III 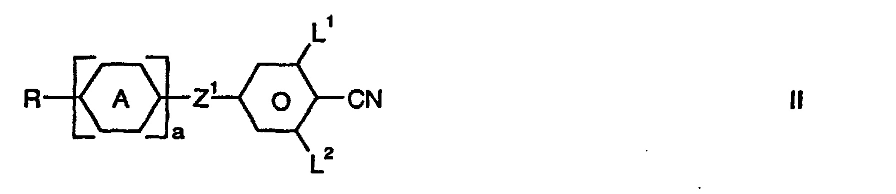 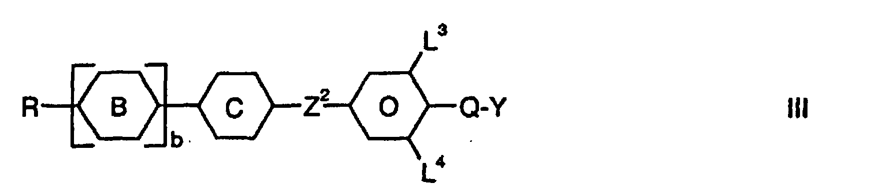 worin
R eine Alkylgruppe mit 1 bis 12 C-Atomen, wobei auch ein oder zwei nicht benachbarte CH₂-Gruppen durch -O-, -CH=CH-, -CO-, -OCO- oder -COO- ersetzt sein können, 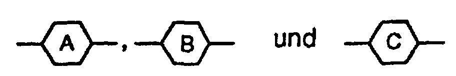 jeweils unabhängig voneinander 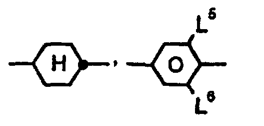 oder 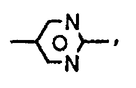
L¹⁻⁶ jeweils unabhängig voneinander H oder F,
Z¹ -COO-, -CH₂CH₂- oder eine Einfachbindung,
Z² -CH₂CH₂-, -COO-, -C≡C- oder eine Einfachbindung,
Q -CF₂-, -CHF-, -OCF₂-, -OCFH- oder eine Einfachbindung,
Y F oder Cl
a 1 oder 2, und
b 0 oder 1
bedeuten,
enthält.

3. Display nach Anspruch 1 oder 2, **dadurch gekennzeichnet, daß** Komponente A wenigstens eine Verbindung der Formeln lla bis IId, 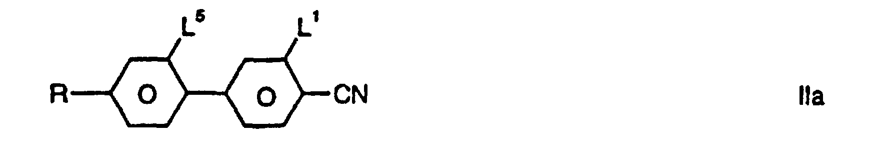 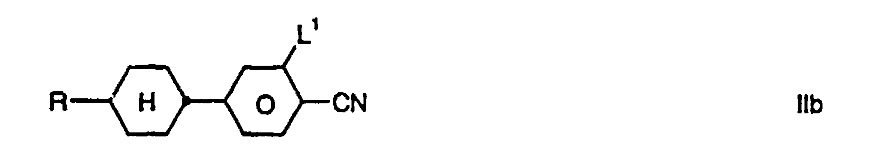 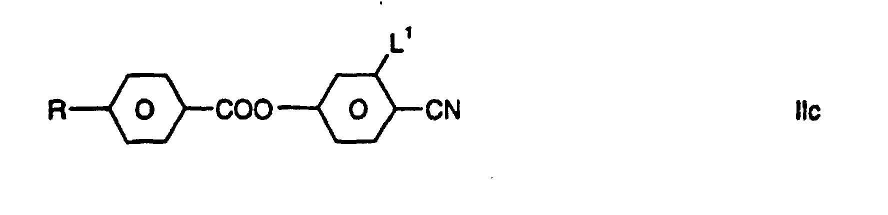 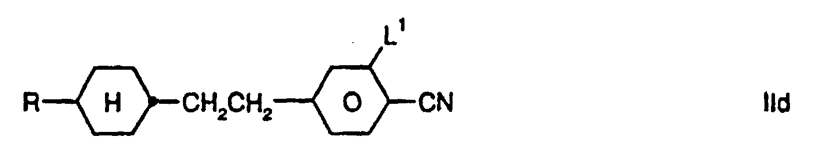 worin
R, L¹ und L⁵ die in Anspruch 2 angegebene Bedeutung besitzen,
enthält.

4. Display nach einem der Ansprüche 1 bis 3, **dadurch gekennzeichnet, daß** Komponente B ein oder mehrere Verbindungen der Formeln 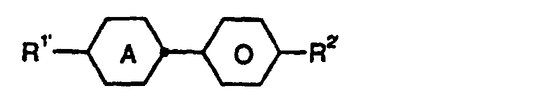 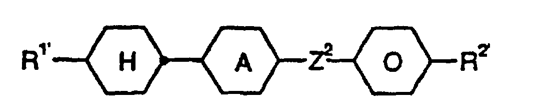 worin
R^{1'} und R^{2'} jeweils unabhängig voneinander Alkyl, Alkoxy, Alkenyl, Alkenyloxy mit 1-12 C-Atomen, und 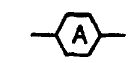 und Z² die in Anspruch 2 gegebene Bedeutung besitzen,
enthält

5. Display nach mindestens einem der Ansprüche 1 bis 4, **dadurch gekennzeichnet, daß** die Flüssigkristallmischung eine oder mehrere Verbindungen der Formel B1IIIa. 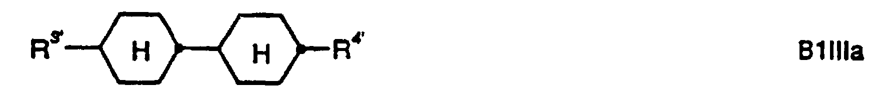 worin
R^{3'} CH₃-(CH₂)ₒ-O-, CH₃-(CH₂)ₚ-, trans- H-(CH₂)_{q}-CH=CH-(CH₂CH₂)_{b}-CH₂O- oder trans-H-(CH₂)_{q}-CH=CH-(CH₂CH₂)_{b}-, CH₃-(CH₂)₀-O-CH₂-,
R^{4'} CH₃-(CH₂)ₚ-,
o 1, 2, 3 oder 4,
q 0, 1, 2 oder 3,
b 0 oder 1, und
p 1, 2, 3 oder 4
ist,
enthält.

6. Display nach mindestens einem der Ansprüche 1 bis 5, **dadurch gekennzeichnet, daß** die Komponente C eine oder mehrere Verbindungen ausgewählt aus der Gruppe bestehend aus V bis IX enthält, 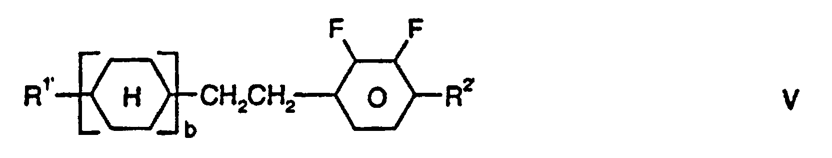 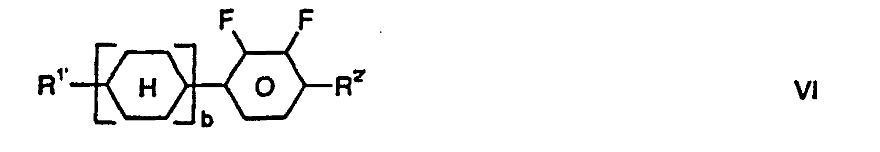 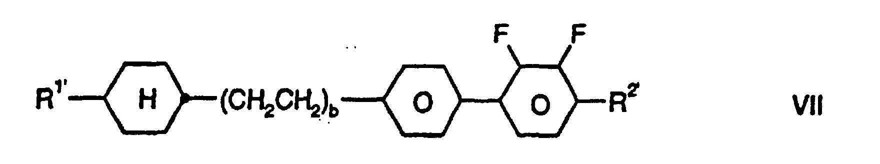 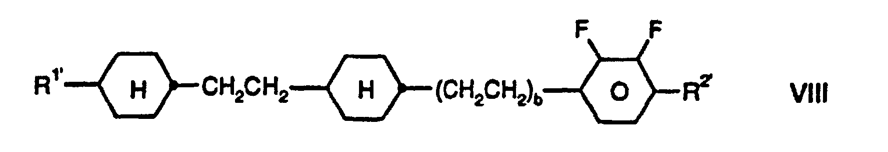 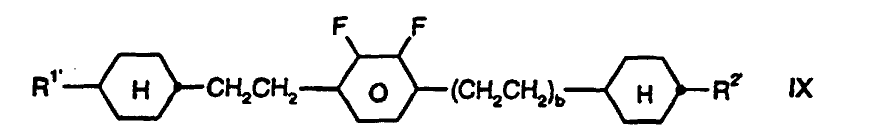 worin R^{1'} und R^{2'} die für R angegebene Bedeutung haben und b 0 oder 1 ist,
enthält.

7. Display nach mindestens einem der Ansprüche 1 bis 6, **dadurch gekennzeichnet, daß** die Komponente B eine oder mehrere Verbindungen ausgewählt aus der Gruppe bestehend aus Xa bis Xlla enthält. 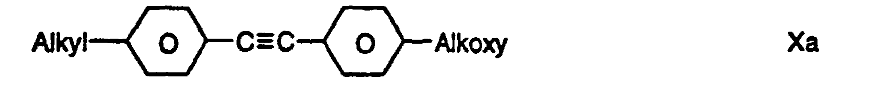 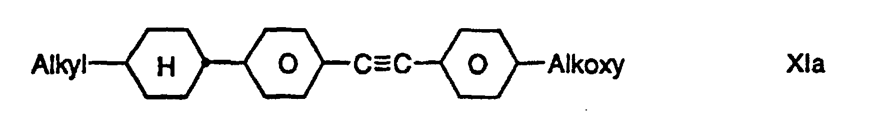 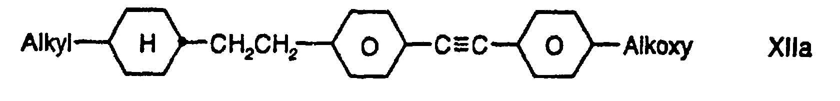 worin der Alkyl- und Alkoxyrest bis zu 7 C-Atomen enthält.

8. Display nach mindestens einem der Ansprüche 1 bis 7, **dadurch gekennzeichnet, daß** Komponente A eine oder mehrere Verbindungen der Formel T3a enthält, 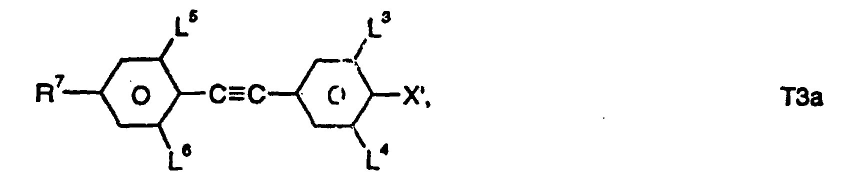 worin
R⁷ -CₓH₂ₓ₊₁, -OCₓH₂ₓ₊₁, 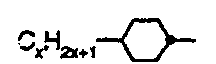 oder 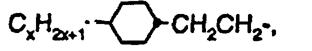
x eine ganze Zahl von 1-15,
L³⁻⁶ jeweils unabhängig voneinander H oder F, und
X' F, Cl oder OCF₃
bedeuten.

9. Display nach Anspruch 1, **dadurch gekennzeichnet, daß** Komponente B mindestens eine Verbindung der Formel lJ und/oder der Formel IK, beides Teilformeln der in Anspruch 1 gegebenen Formel I 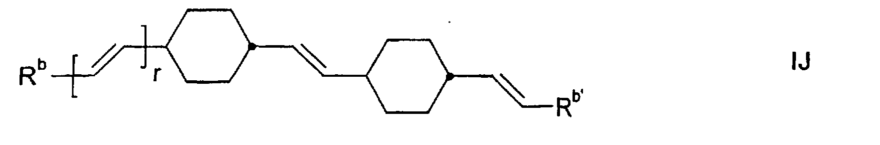 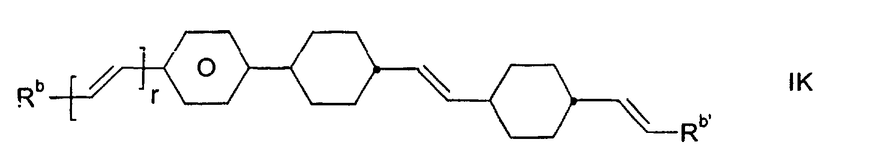 enthält,
worin
R^{b} und R^{b'} jeweils unabhängig voneinander H oder Alkyl mit 1 bis 6 C-Atomen,
R^{b} im Falle der Formel IK und r = 0 auch Alkoxy mit 1 bis 6 C-Atomen,
r 0 oder 1
bedeuten.

10. Verbindungen der Formel, 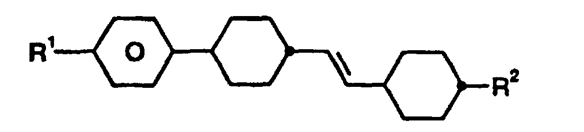 worin
R¹ und R² die in Anspruch 1 angegebene Bedeutung haben.

11. Verbindungen der Formel IB, einer Teilformel der in Anspruch 1 gegebenen Formel I 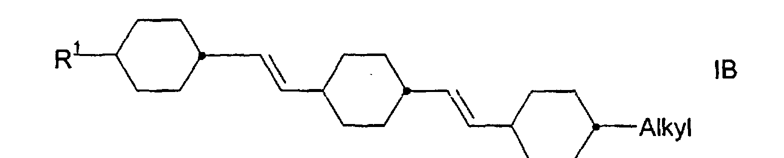 worin
R¹ die in Anspruch 1 angegebene Bedeutung hat und Alkyl ein Alkylrest mit 1-6 C-Atomen bedeutet.

12. Verbindungen der Formel IC, einer Teilformel der in Anspruch 1 gegebenen Formel I 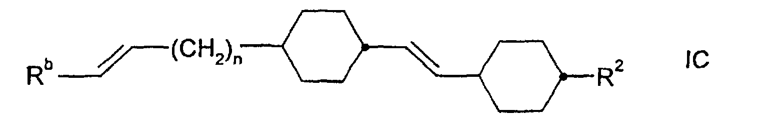 worin R² und n die in Anspruch 1 angegebene Bedeutung besitzen und R^{b} H oder Alkyl mit 1-6 C-Atomen bedeutet.

13. Verbindungen der Formel ID, einer Teilformel der in Anspruch 1 gegebenen Formel I 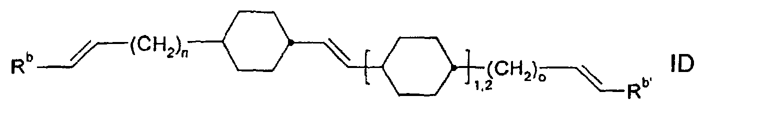
R^{b} und R^{b'} jeweils unabhängig voneinander H oder Alkyl mit 1-6 C-Atomen,
n und o jeweils unabhängig voneinander 0-6
bedeuten.

14. Verbindung der Formel IE, einer Teilformel der in Anspruch 1 gegebenen Formel I 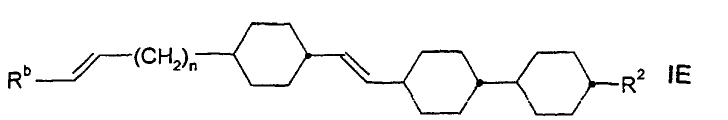 worin R² und n die in Anspruch 1 angegebene Bedeutung besitzen und R^{b} H oder Alkyl mit 1-6 C-Atomen bedeutet.

15. Verbindung der Formel IF, einer Teilformel der in Anspruch 1 gegebenen Formel I 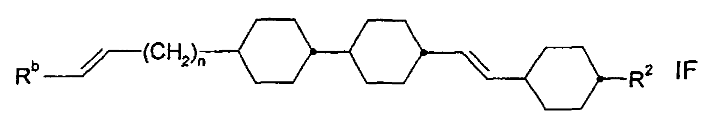 worin R² und n die in Anspruch 1 angegebene Bedeutung besitzen und R^{b} H oder Alkyl mit 1-6 C-Atomen bedeutet.

16. Verbindung der Formel IG, einer Teilformel der in Anspruch 1 gegebenen Formel I 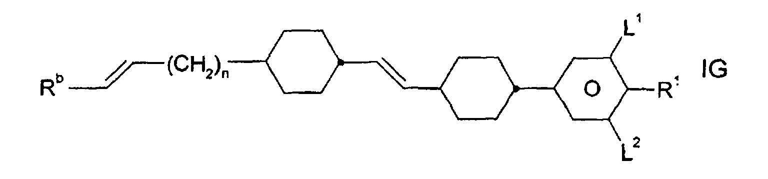 worin R¹ und n die in Anspruch 1 angegebene Bedeutung besitzen, R^{b} H oder Alkyl mit 1-6 C-Atomen, L¹ und L² H oder F bedeuten.

17. Verbindung der Formel IH, einer Teilformel der in Anspruch 1 gegebenen Formel I 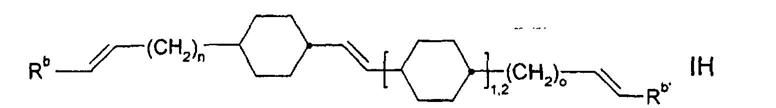 worin
R^{b} und R^{b'} jeweils unabhängig voneinander H oder Alkyl mit 1 bis 6 C-Atomen und n oder o jeweils unabhängig voneinander 0-6 bedeuten.

18. Verbindungen der Formel, 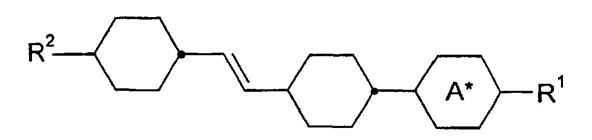 einer Teilformel der in Anspruch 1 gegebenen Formel I
worin
R¹, R² und 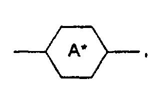 die in Anspruch 1 angegebene Bedeutung haben.

19. Verbindungen der Formel 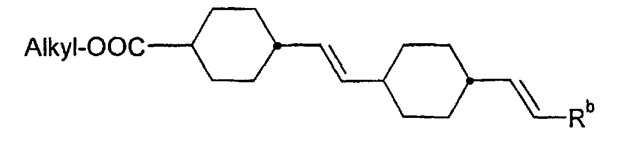 einer Teilformel der in Anspruch 1 gegebenen Formel I
worin
Alkyl C₁₋₆-Alkyl und
R^{b} H oder Alkyl mit 1-6 C-Atomen
bedeuten.

## Claims

1. Supertwist liquid-crystal display having
- two plane-parallel outer plates which, together with a frame, form a cell,
- a nematic liquid-crystal mixture having positive dielectric anisotropy which is located in the cell,
- electrode layers with overlying alignment layers on the insides of the outer plates,
- a tilt angle between the longitudinal axis of the molecules at the surface of the outer plates and the outer plates of from 1 degree to 30 degrees, and
- a twist angle of the liquid-crystal mixture in the cell from alignment layer to alignment layer with a value of between 100 and 600°,
- a nematic liquid-crystal mixture having a Δε ≥ 1 consisting of
a) 20 - 90% by weight of a liquid-crystalline component A consisting of one or more compounds having a dielectric anisotropy of greater than +1.5;
b) 10 - 65% by weight of a liquid-crystalline component B consisting of one or more compounds having a dielectric anisotropy of between -1.5 and +1.5;
c) 0 - 20% by weight of a liquid-crystalline component D consisting of one or more compounds having a dielectric anisotropy of below -1.5,
d) an optically active component C in such an amount that the ratio between layer thickness (separation of the plane-parallel outer plates) and natural pitch of the chiral nematic liquid-crystal mixture is from 0.2 to 1.3,
**characterised in that** component B comprises at least one compound of the formula I 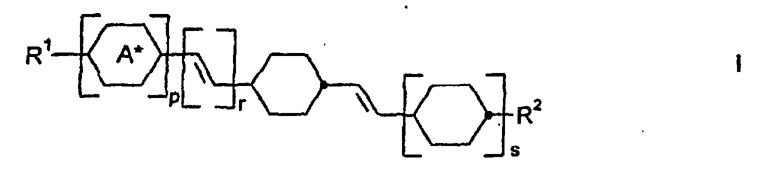 in which 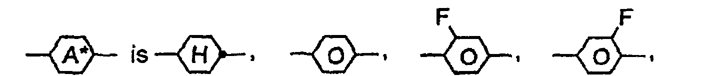 or 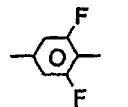
R¹ is F, Cl, or an alkyl, alkoxy or alkenyl radical having from 1 to 10 carbon atoms which is unsubstituted or monosubstituted by CN or CF₂ or at least monosubstituted by halogen, where one or more CH₂ groups in this radical may also be replaced by -O-,
R² is (CH₂)ₙ-CH=CH-R^{a} or (CH₂)ₙ-CH=CH₂ and, in the case where r = 1, also R^{a},
R^{a} is straight-chain alkyl having 1-6 carbon atoms,
n is 0-6,
p is 0 or 1,
r is 0, 1 or 2, and
s is 1 or 2.

2. Display according to Claim 1, **characterised in that** component A comprises compounds of the formulae II and III 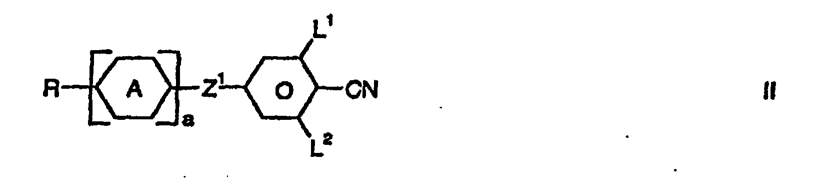 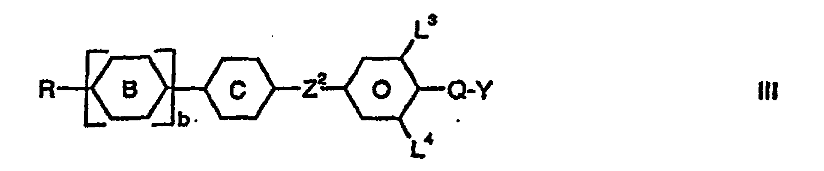 in which
R is an alkyl group having from 1 to 12 carbon atoms, in which, in addition, one or two non-adjacent CH₂ groups may be replaced by -O-, -CH=CH-, -CO-, -OCO- or -COO-, 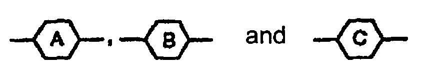 are each, independently of one another, 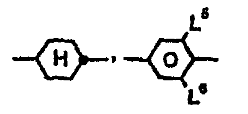 or 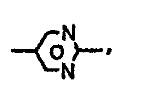
L¹⁻⁶ are each, independently of one another, H or F,
Z¹ is -COO-, -CH₂CH₂- or a single bond,
Z² is -CH₂CH₂-, -COO-, -C≡C- or a single bond,
Q is -CF₂-, -CHF-, -OCF₂-, -OCFH- or a single bond,
Y is F or Cl,
a is 1 or 2, and
b is 0 or 1.

3. Display according to Claim 1 or 2, **characterised in that** component A comprises at least one compound of the formulae lla to IId 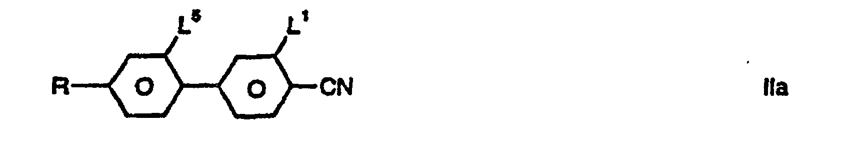 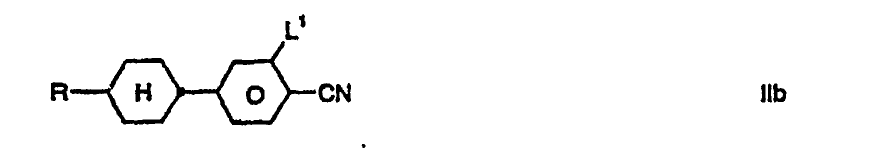 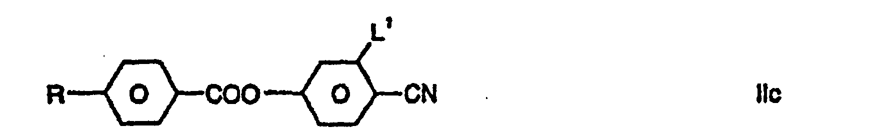 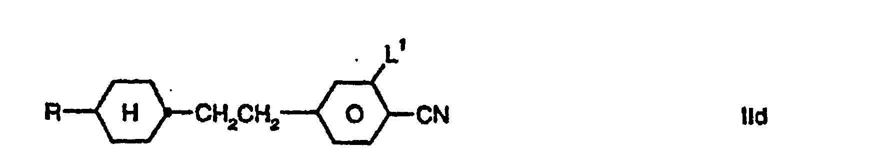 in which
R, L¹ and L⁵ are as defined in Claim 2.

4. Display according to one of Claims 1 to 3, **characterised in that** component B comprises one or more compounds of the formulae 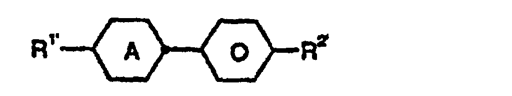 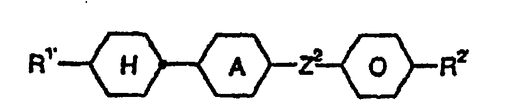 in which
R^{1'} and R^{2'} are each, independently of one another, alkyl, alkoxy, alkenyl or alkenyloxy having 1-12 carbon atoms, and 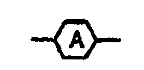 and Z² are as defined in Claim 2.

5. Display according to at least one of Claims 1 to 4, **characterised in that** the liquid-crystal mixture comprises one or more compounds of the formula B1IIIa 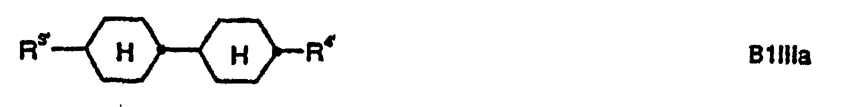 in which
R^{3'} is CH₃-(CH₂)ₒ-O-, CH₃-(CH₂)ₚ-, trans-H-(CH₂)_{q}-CH=CH- (CH₂CH₂)_{b}-CH₂O- or trans-H-(CH₂)_{q}-CH=CH-(CH₂CH₂)_{b}- or CH₃-(CH₂)ₒ-O-CH₂-,
R^{4'} is CH₃-(CH₂)ₚ-,
o is 1, 2, 3 or 4,
q is 0,1, 2 or 3,
b is 0 or 1, and
p is 1, 2, 3 or 4.

6. Display according to at least one of Claims 1 to 5, **characterised in that** component C comprises one or more compounds selected from the group consisting of V to IX 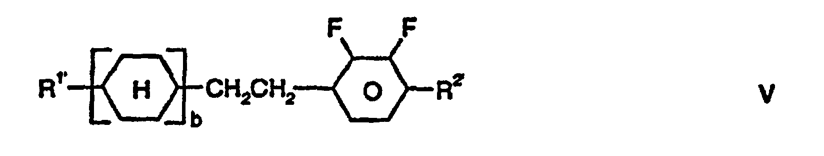 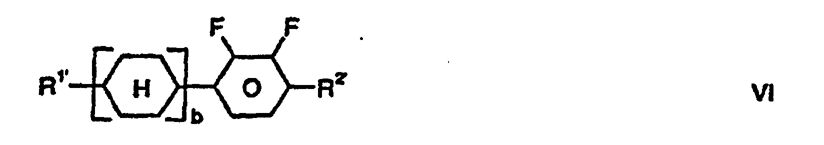 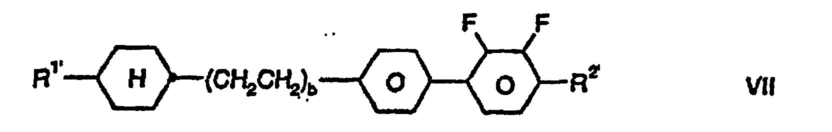 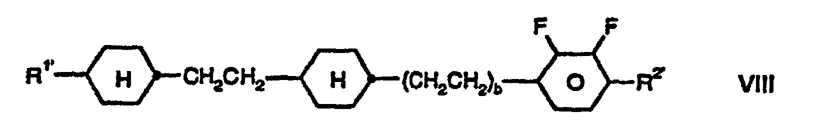 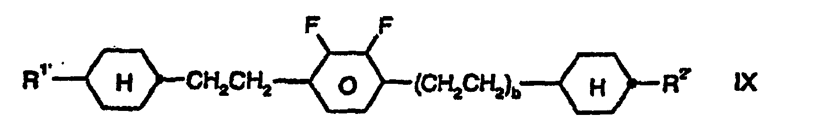 in which R^{1'} and R^{2'} are as defined for R, and b is 0 or 1.

7. Display according to at least one of Claims 1 to 6, **characterised in that** component B comprises one or more compounds selected from the group consisting of Xa to Xlla 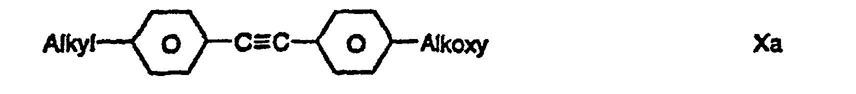 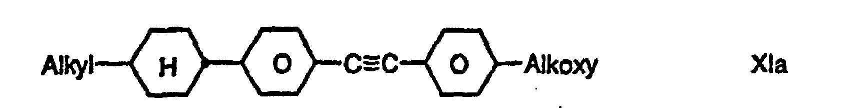 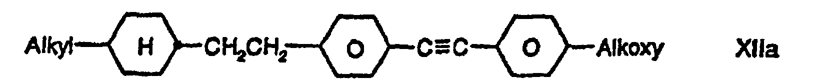 in which the alkyl and alkoxy radicals contain up to 7 carbon atoms.

8. Display according to at least one of Claims 1 to 7, **characterised in that** component A comprises one or more compounds of the formula T3a 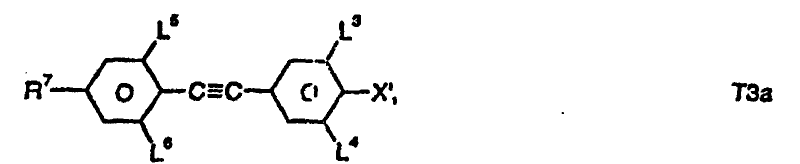 in which
R⁷ is -CₓH₂ₓ₊₁, -OCₓH₂ₓ₊₁, 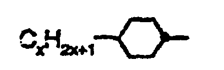 or 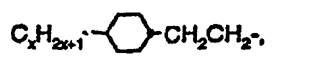
x is an integer from 1 to 15,
L³⁻⁶ are each, independently of one another, H or F, and
X is F, Cl or OCF₃.

9. Display according to Claim 1, **characterised in that** component B comprises at least one compound of the formula IJ and/or of the formula IK, both sub-formulae of the formula I given in Claim 1, 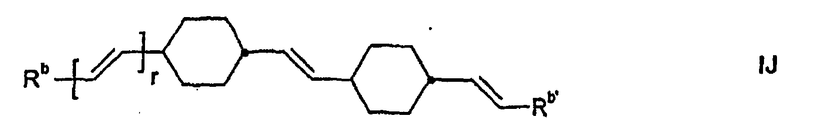 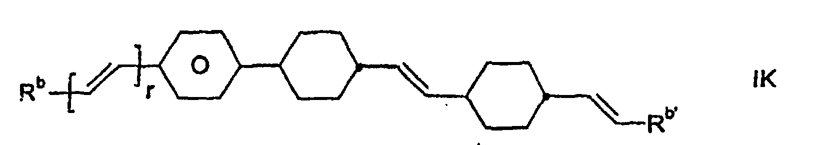 in which
R^{b} and R^{b'} are each, independently of one another, H or alkyl having from 1 to 6 carbon atoms,
R^{b} in the case of the formula IK and r = 0, is alternatively alkoxy having from 1 to 6 carbon atoms,
r is 0 or 1.

10. Compounds of the formula 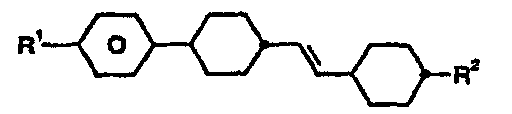 in which
R¹ and R² are as defined in Claim 1.

11. Compounds of the formula IB, a sub-formula of the formula I given in Claim 1, 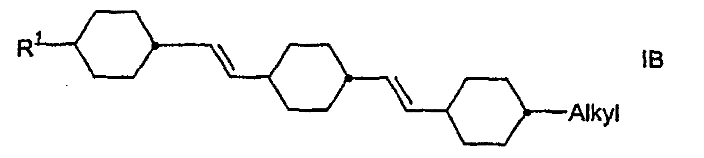 in which
R¹ is as defined in Claim 1, and alkyl is an alkyl radical having 1-6 carbon atoms.

12. Compounds of the formula IC, a sub-formula of the formula I given in Claim 1, 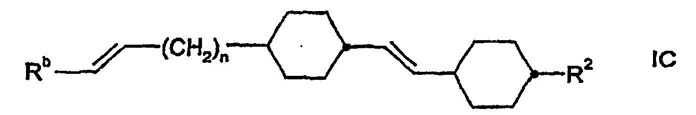 in which R² and n are as defined in Claim 1, and R^{b} is H or alkyl having 1-6 carbon atoms.

13. Compounds of the formula ID, a sub-formula of the formula I given in Claim 1, 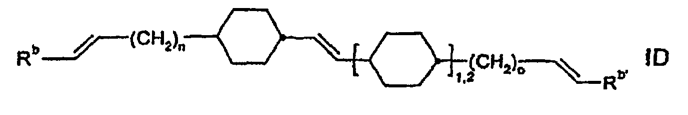 in which
R^{b} and R^{b'} are each, independently of one another, H or alkyl having 1-6 carbon atoms,
n and o are each, independently of one another, 0-6.

14. Compound of the formula IE, a sub-formula of the formula I given in Claim 1, 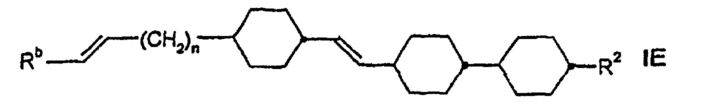 in which R² and n are as defined in Claim 1, and R^{b} is H or alkyl having 1-6 carbon atoms.

15. Compound of the formula IF, a sub-formula of the formula I given in Claim 1, 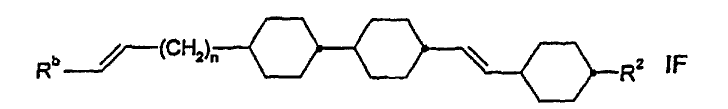 in which R² and n are as defined in Claim 1, and R^{b} is H or alkyl having 1-6 carbon atoms.

16. Compound of the formula IG, a sub-formula of the formula I given in Claim 1, 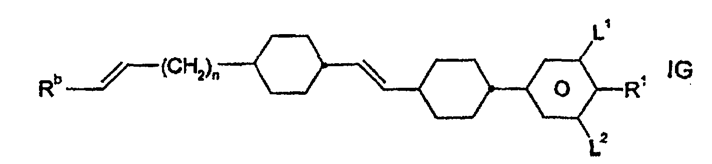 in which R¹ and n are as defined in Claim 1, R^{b} is H or alkyl having 1-6 carbon atoms, and L¹ and L² are H or F.

17. Compound of the formula IH, a sub-formula of the formula I given in Claim 1, 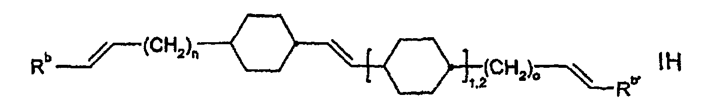 in which
R^{b} and R^{b'} are each, independently of one another, H or alkyl having from 1 to 6 carbon atoms, and n and o are each, independently of one another, 0-6.

18. Compounds of the formula 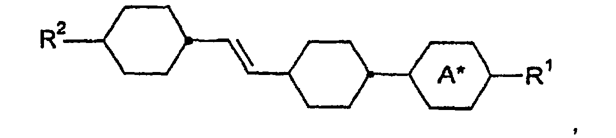 a sub-formula of the formula I given in Claim 1,
in which
R¹, R² and 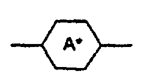 are as defined in Claim 1.

19. Compounds of the formula 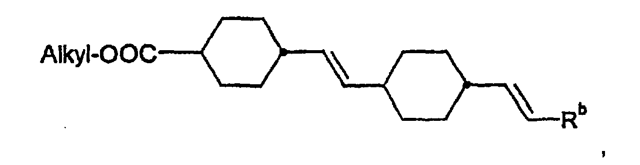 a sub-formula of the formula I given in Claim 1,
in which
alkyl is C₁₋₆-alkyl, and
R^{b} is H or alkyl having 1-6 carbon atoms.

## Revendications

1. Affichage à cristaux liquides STN avec
- deux plaques de support à faces planes et parallèles, qui avec une bordure constituent une cellule,
- un mélange de cristaux liquides nématique présent dans la cellule avec une anisotropie diélectrique positive,
- des couches d'électrodes avec des couches d'orientation disposées à leur surface du côté interne des plaques de support,
- un angle d'attaque entre le grand axe de la molécule à la surface des plaques de support et la plaque de support compris entre 1 grade et 30 grades, et
- un angle de torsion du mélange de cristaux liquides dans la cellule de la couche d'orientation par rapport à la couche d'orientation entre 100 et 600°,
- un mélange de cristaux liquides nématiques avec un Δε ≥ 1 constitué de
a) 20 - 90% en poids d'un composant A cristal liquide constitué d'un ou plusieurs composés avec une anisotropie diélectrique supérieure à +1,5;
b) 10 - 65% en poids d'un composant B cristal liquide constitué d'un ou plusieurs composés avec une anisotropie diélectrique comprise entre -1,5 et +1,5;
c) 0 - 20% en poids d'un composant D cristal liquide constitué d'un ou plusieurs composés avec une anisotropie diélectrique inférieure à -1,5;
d) un composant C optiquement actif à une quantité telle que le rapport entre l'épaisseur de couche (distance des plaques porteuses à faces planes et parallèles) et une hauteur de course naturelle du mélange de cristaux liquides nématiques chiraux comprise entre 0,2 et 1,3,
**caractérisé en ce que** le composant B contient au moins un composé de formule I 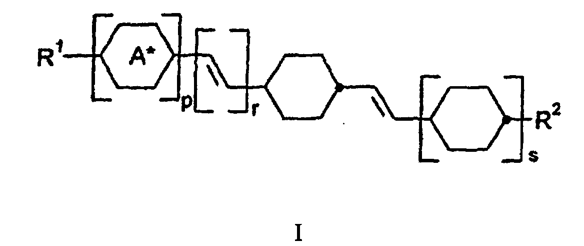 dans laquelle 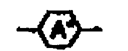 représente 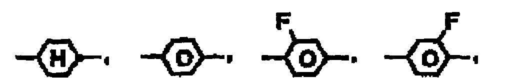 ou 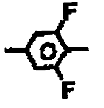
R¹ représente F, Cl, un résidu alkyle, alkoxy ou alkényle à 1 jusqu'à 10 atomes de C non substitué ou monosubstitué par CN ou CF₂ ou au moins monosubstitué par un halogène, tandis que dans ce résidu un ou plusieurs groupes CH₂ peuvent être remplacés par -O-,
R² représente (CH₂)ₙ-CHCH-R^{a} ou (CH₂)ₙ-CH=CH₂ et dans le cas où r = 1 également R^{a}
R^{a} représente un alkyle linéaire avec 1-6 atomes de C,
n représente 0-6,
p représente 0 ou 1,
r représente 0, 1 ou 2, et
s représente 1 ou 2.

2. Affichage selon la revendication 1, **caractérisé en ce que** le composant A contient des composés de formules II et III 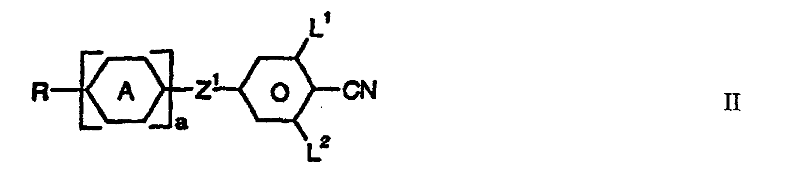 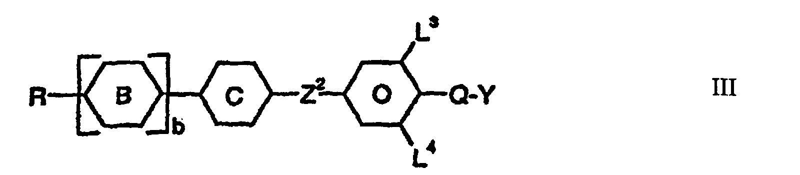 dans lesquelles
R représente un groupe alkyle avec 1 à 12 atomes de C, tandis qu'également un ou deux groupes CH₂ non vicinaux peuvent être substitués par -O-, -CH=CH-, -CO-, -OCO-, ou -COO-, 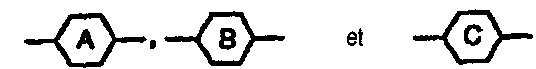 représentent chacun indépendamment l'un de l'autre 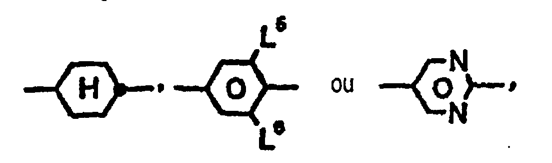
L¹⁻⁶ représentent chacun indépendamment l'un de l'autre H ou F,
Z¹ représente -COO-, -CH₂CH₂- ou une liaison simple,
Z² représente -CH₂CH₂-, -COO-, -C ≡C- ou une liaison simple,
Q représente -CF₂-, -CHF-, -OCF₂-, -OCFH- ou une liaison simple,
Y représente F ou Cl
a représente 1 ou 2, et
b représente 0 ou 1.

3. Affichage selon la revendication 1 ou 2, **caractérisé en ce que** le composant A contient au moins un composé de formule IIa à IId, 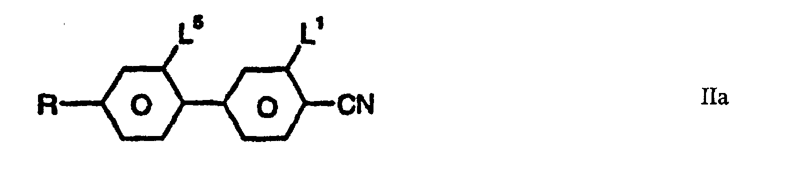 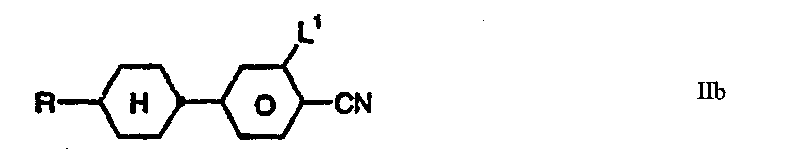 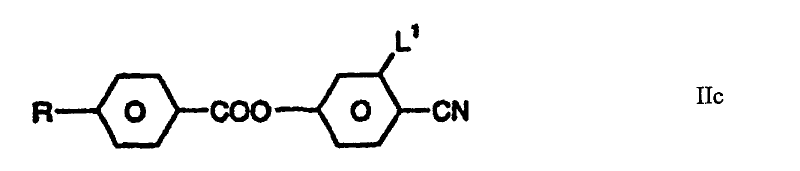 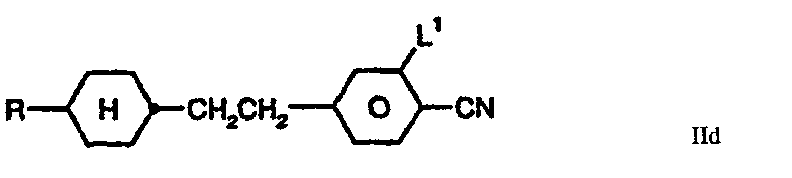 dans laquelle
R, L¹ et L⁵ ont les significations indiquées dans la revendication 2.

4. Affichage selon l'une des revendications 1 à 3, **caractérisé en ce que** le composant B contient un ou plusieurs composés de formules 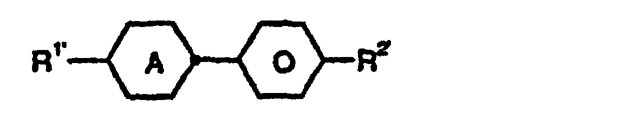 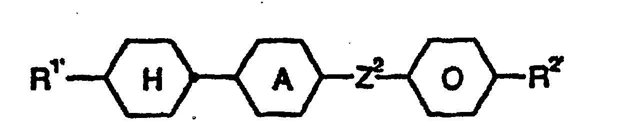 dans lesquelles
R^{1'} et R^{2'} chacun indépendamment l'un de l'autre représentent un alkyle, un alkoxy, un alkényle, un alkényloxy avec 1-12 atomes de C. et 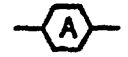 et Z² ont les significations indiquées dans la revendication 2.

5. Affichage selon l'une au moins des revendications 1 à 4, **caractérisé en ce que** le mélange de cristaux liquides contient un ou plusieurs composés de formule B1IIIa 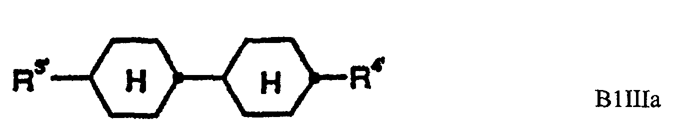 dans laquelle
R^{3'} représente CH₃-(CH₂)ₒ-O-, CH₃-(CH₂)ₚ-, trans-H-(CH₂)_{q}-CH=CH-(CH₂CH₂)_{b}-CH₂O- ou trans-H-(CH₂)_{q}-CH=CH-(CH₂CH₂)_{b}-, CH₃-(CH₂)ₒ-O-CH₂-,
R^{4'} représente CH₃-(CH₂)ₚ-,
O représente 1, 2, 3 ou 4,
q représente 0, 1, 2 ou 3,
b représente 0 ou 1, et
P représente 1,2,3 ou 4.

6. Affichage selon au moins l'une des revendications 1 à 5, **caractérisé en ce que** le composant C contient un ou plusieurs composés choisis parmi le groupe constitué par V jusqu'à IX 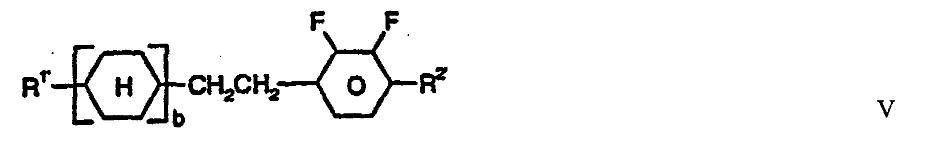 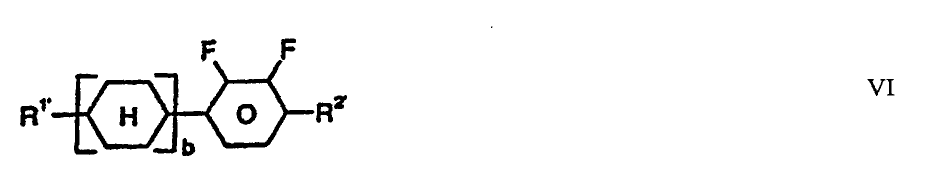 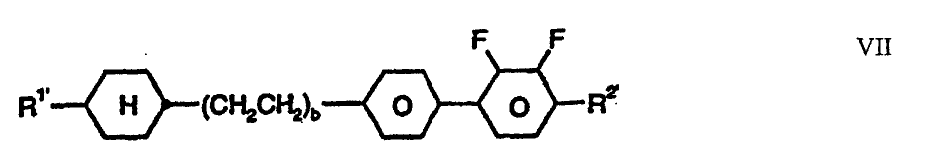 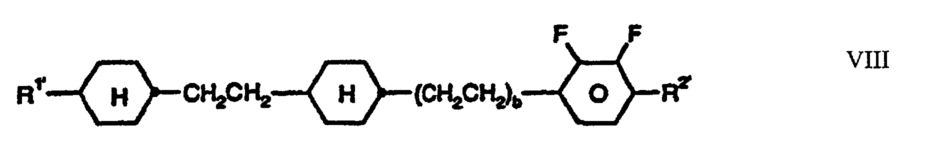 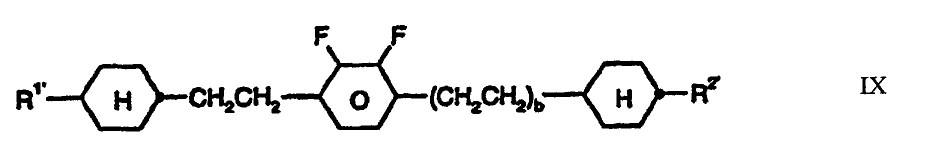 dans lesquelles R^{1'} et R^{2'} ont la signification indiquée pour R et b est 0 ou 1.

7. Affichage selon au moins l'une des revendications 1 à 6, **caractérisé en ce que** le composant B contient un ou plusieurs composés choisis parmi le groupe constitué par Xa à XIIa. 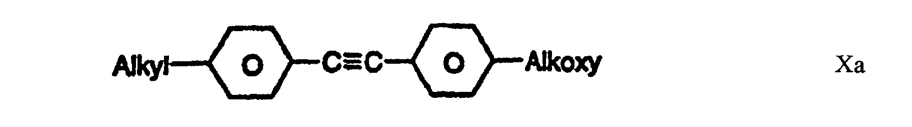 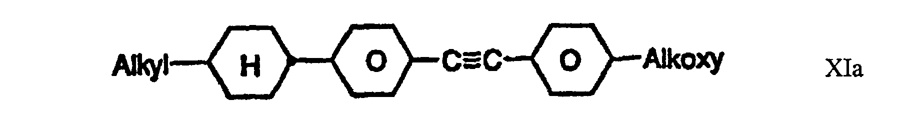 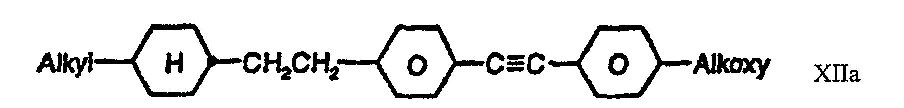 dans lesquelles le résidu alkyle et le résidu alkoxy contient jusqu'à 7 atomes de C.

8. Affichage selon au moins l'une des revendications 1 à 7, **caractérisé en ce que** le composant A contient un ou plusieurs composés de formule T3a, 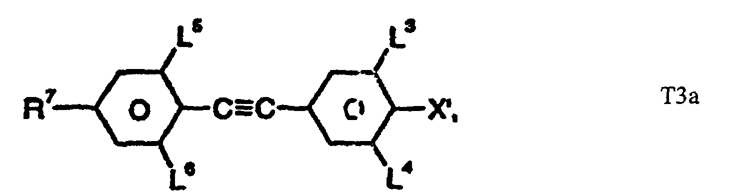 dans laquelle
R⁷ représente -CₓH₂ₓ₊₁, -OCₓH₂ₓ₊₁, CₓH₂ₓ₊₁ 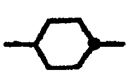 ou 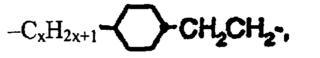
x représente un nombre entier compris entre 1 et 15,
L³⁻⁶ représentent chacun indépendamment l'un de l'autre H ou F, et
X' représente F, Cl ou OCF₃.

9. Affichage selon la revendication 1, **caractérisé en ce que** le composant B contient au moins un composé de formule IJ et/ou de formule IK, les deux formules partielles de la formule I indiquée dans la revendication 1 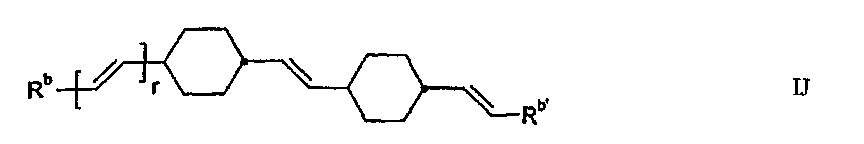 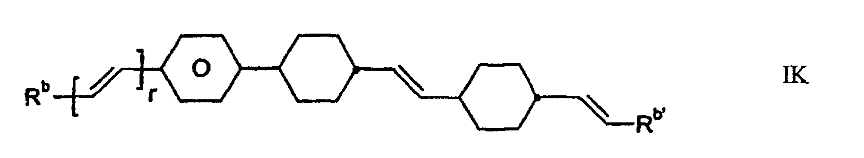 dans laquelle
R^{b} et R^{b'} chacun indépendamment l'un de l'autre représentent H ou un alkyle avec 1 à 6 atomes de C,
R^{b} dans le cas de la formule IK et r = 0 représente également un alkoxy avec 1 à 6 atomes de C,
r représente 0 ou 1.

10. Composés de formule 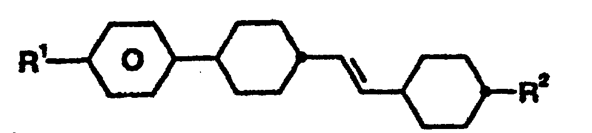 dans laquelle
R¹ et R² ont la signification indiquée dans la revendication 1.

11. Composés de formule IB, d'une formule partielle de la formule I indiquée dans la revendication 1 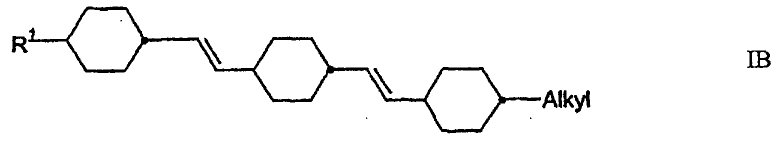 dans laquelle
R¹ a la signification indiquée dans la revendication 1 et alkyl représente un résidu alkyle avec 1-6 atomes de C.

12. Composés de formule IC, d'une formule partielle de la formule indiquée dans la revendication 1 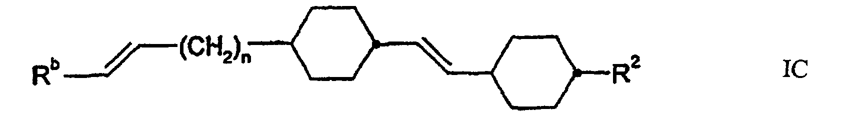 dans laquelle R² et n ont la signification indiquée dans la revendication 1 et R^{b} représente H ou un alkyle à 1-6 atomes de C.

13. Composés de formule ID, d'une formule partielle de la formule I indiquée dans la revendication 1 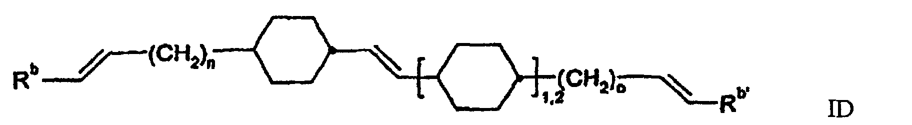 dans laquelle
R^{b} et R^{b'} représentent chacun indépendamment l'un de l'autre H ou un alkyle avec 1-6 atomes de C,
n et o chacun indépendamment l'un de l'autre représentent 0-6.

14. Composé de formule IE, d'une formule partielle de la formule indiquée dans la revendication 1 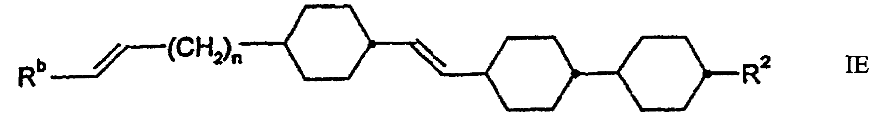 dans laquelle R² et n ont la signification indiquée dans la revendication 1 et R^{b} représente H ou un alkyle à 1-6 atomes de C.

15. Composé de formule IF, d'une formule partielle de la formule indiquée dans la revendication 1 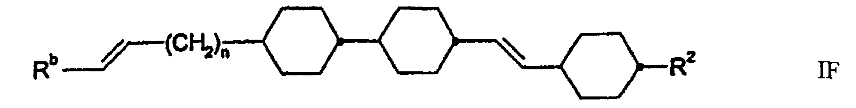 dans laquelle R² et n ont la signification indiquée dans la revendication 1 et R^{b} représente H ou un alkyle à 1-6 atomes de C.

16. Composé de formule IG, d'une formule partielle de la formule I indiquée dans la revendication 1 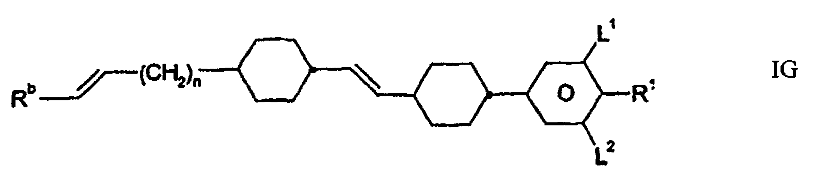 dans laquelle R¹ et n ont la signification indiquée dans la revendication 1, R^{b} représente H ou un alkyle à 1-6 atomes de C, L¹ et L² représentent H ou F.

17. Composé de formule IH, d'une formule partielle de la formule I indiquée dans la revendication 1 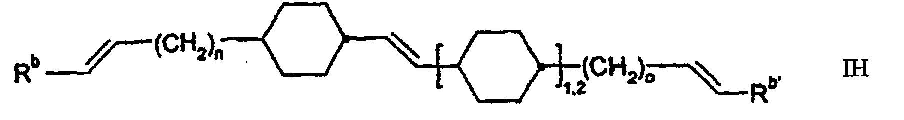 dans laquelle
R^{b} et R^{b'} représentent chacun indépendamment l'un de l'autre H ou un alkyle à 1-6 atomes de C, n et o représentent chacun indépendamment l'un de l'autre 0-6.

18. Composés de formule 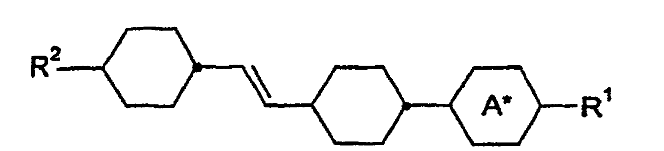 d'une formule partielle de la formule I indiquée dans la revendication 1
dans laquelle
R¹, R² et 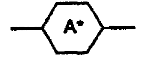 ont la signification indiquée dans la revendication 1.

19. Composés de formule 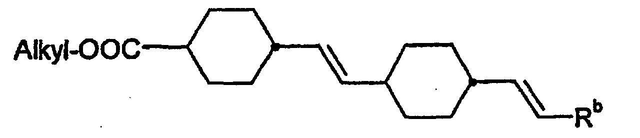 d'une formule partielle de la formule I indiquée dans la revendication 1
dans laquelle
alkyl représente un alkyle C₁₋₆ et
R^{b} représente H ou un alkyle à 1-6 atomes de C.
